# EUROPEAN PATENT SPECIFICATION

(11) **EP 1 658 298 B1**
(45) Date of publication and mention of the grant of the patent: **26.03.2008**
(21) Application number: 04766606.0
(22) Date of filing: 26.08.2004
(51) Int. Cl.: C07F 9/6506, C07C 251/00

(54) **PHOSPHORUS-CONTAINING IMIDAZOLINES AND METAL COMPLEXES THEREOF**
PHOSPHOR-ENTHALTENDE IMIDAZOLINE UND METALLKOMPLEXE DAVON
IMIDAZOLINES CONTENANT DU PHOSPHORE ET SES COMPLEXES METALLIQUES

(30) Priority: 29.08.2003 CH 148403
(43) Date of publication of application: 24.05.2006
(73) Proprietor: Solvias AG, 4057 Basel (CH)
(72) Inventor: MENGES, Frederik, 16548 Glienicke/ Nordbahn (DE); PFALTZ, Andreas, CH-4102 Binningen (CH)
(74) Representative: Maué, Paul Georg
(86) International application number: PCT/EP2004/051915
(87) International publication number: WO 2005/021562

(56) References cited:
- WO-A-01/18012
- DE-A- 4 243 030
- PFALTZ A. ET AL.: "Iridium-Catalyzed Enantioselective Hydrogenation of Olefins" ADV. SYNTH. CATAL., vol. 345, no. 1,2, 1 January 2003 (2003-01-01), pages 33-43, XP002310662 cited in the application

## Description

The present invention relates to chiral phosphorus-containing imidazolines; a process for preparing them; metal complexes comprising metals selected from transition groups I and VIII of the Periodic Table of the Elements (d-10 and d-8 metals, hereinafter referred to as TM8 metals) and phosphorus-containing imidazolines as ligands; a process for asymmetric synthesis by addition of hydrogen, boron hydrides or silanes onto a carbon-carbon or carbon-heteroatom multiple bond in prochiral organic compounds, or addition of C-nucleophiles or amines onto allylic compounds, especially for the asymmetric hydrogenation of carbon-carbon or carbon-heteroatom multiple bonds by means of hydrogen, in the presence of catalytic amounts of the metal complexes; and the use of the metal complexes as catalysts for asymmetric synthesis by addition of hydrogen, boron hydrides or silanes onto a carbon-carbon or carbon-heteroatom multiple bond in prochiral organic compounds, or of C-nucleophiles or amines onto allylic compounds, especially for the asymmetric hydrogenation of carbon-carbon or carbon-heteroatom multiple bonds by means of hydrogen.

Chiral ligands based on oxazolines and imidazolines substituted by complexing groups have recently become of interest. Metal complexes containing such ligands are good catalysts for chiral syntheses by addition reactions with organic compounds having double bonds. The structures (A) to (D) below are described in the literature: 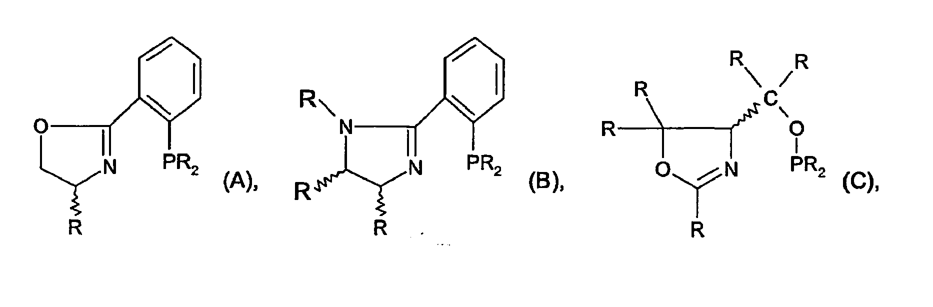 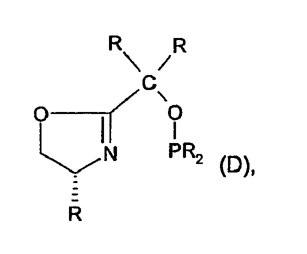
A) G. Helmchen and A. Pfaltz, Accounts of Chemical Research, Volume 33, Number 6, pages 336 to 345 (2000);
B) WO 01/18012, F. Menges et al., Organic Letters (2002), Vol. 4, No. 26, pages 4713-4716; C. A. Busacca et al., Organic Letters (2003), Vol. 5, Number 4, pages 595 to 598, and
C) EP-A2-1 191 030, A. Pfaltz et al., Adv. Synth. Catal. 2003, 345, Numbers 1 + 2, pages 33 to 43. Further oxazolines are described in DE 4243030.

In Synlett 2003, Number 1, pages 102 to 106, M. Casey et al. describe secondary imidazoline alcohols of the formula 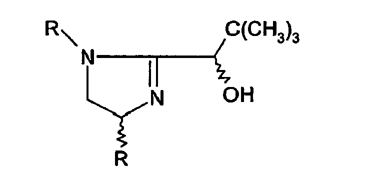 as direct catalysts for the enantioselective reaction of diethylzinc with aldehydes to form secondary alcohols.

Phosphinite-oxazolines, phosphine-oxazolines and phosphine-imidazolines have been found to be valuable ligands for chiral metal complex catalysts by means of which a good catalytic activity, depending on the substrate, and also a distinct to excellent enantioselectivity can be achieved. Studies have shown that the achievable selectivity is strongly dependent on the substrate, so that not every objective can be achieved using the known ligands. There is therefore a need for further ligands to expand the opportunities for effective enantioselective reactions of substrates.

It has surprisingly been found that P,N-ligands which are based on imidazolines and whose phosphorus-O-methyl group is bound to a nonchiral C atom in the imidazoline ring in the α position relative to the two N atoms and which contain at least one chiral C atom in the imidazoline ring can be prepared in a simple manner. With TM8 metals, these substituted imidazolines form chiral complexes which are excellent catalysts for the enantioselective addition of hydrogen, boron hydrides or silanes onto a carbon-carbon or carbon-heteroatom multiple bond in prochiral organic compounds, or of C-nucleophiles or amines onto allylic compounds, or the enantioselective coupling of aryl triflates or alkenyl triflates onto olefins (Heck reaction). The catalytic activity is surprisingly high and is comparable to or better than that of the previously described ligands. The substitution of the N atom enables both the stereoselectivity and the catalytic activity to be strongly influenced and matched to prochiral substrates. The phosphorus-containing imidazolines have been found to be superior in terms of enantioselectivity, particularly in the hydrogenation of prochiral cis isomers of olefins, even of diolefins having two prochiral centres at a high achievable diastereoselectivity.

The ligands can be prepared by a simple, novel process by reacting a central intermediate with primary aromatic amines. The process allows a high modularity with the subsequent introduction of the phosphorus group, so that the steric and electronic properties of the ligands in terms of the catalytic activity and steric selectivity can be matched very well to the substrates to be reacted.

The invention provides compounds of the formulae I and la, 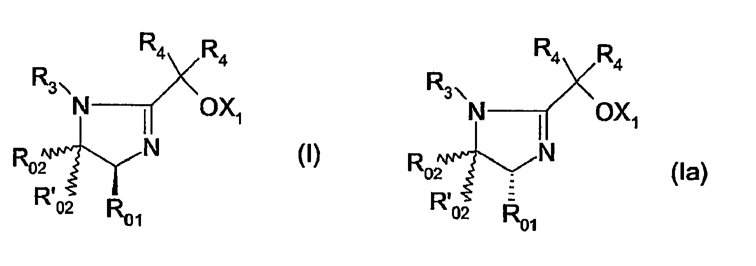 where
X₁ is secondary phosphino;
R₃ is a hydrocarbon radical having from 1 to 20 C atoms, a heterohydrocarbon radical which is bound via a C atom and has from 2 to 20 atoms and at least one heteroatom selected from the group consisting of O, S, NH and NR, or an -SO₂-R radical;
R is C₁-C₁₈-alkyl, phenyl or benzyl;
the radicals R₄ are each, independently of one another, hydrogen or a hydrocarbon radical having from 1 to 20 C atoms, or the two radicals R₄ together with the C atom to which they are bound form a three- to eight-membered hydrocarbon ring;
R₀₁ is a hydrocarbon radical having from 1 to 20 C atoms; and
R₀₂ and R'₀₂ are each a hydrogen atom or independently have the meaning of R₀₁, or
R₀₁ and R₀₂ together with the C atom to which they are bound form a three- to eight-membered hydrocarbon or heterohydrocarbon ring.

For the purposes of the invention, the term secondary phosphino encompasses structures of the formulae 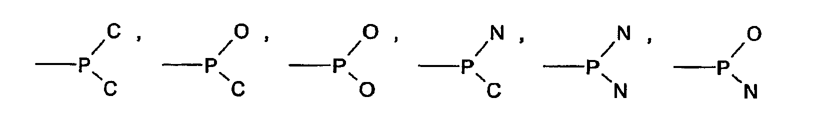 where the C atoms are substituted by hydrogen or by 1-3 hydrocarbon radicals and the O atoms are substituted by one hydrocarbon radical and the N atoms are substituted by two hydrocarbon radicals, or two hydrocarbon radicals together with the atoms to which they are bound form a four- to eight-membered ring and the N atoms bear a further hydrocarbon radical. The N atoms can also be substituted by hydrocarbon-sulphonyl radicals. The hydrocarbon radicals indicated below for the first formula are also applicable to the remaining formulae by inserting O atoms, N-hydrocarbon radicals or N-hydrocarbon-sulphonyl radicals into open-chain or cyclic hydrocarbon radicals between the P-C bond.

X1 as phosphine group P(C)C can contain two identical or two different hydrocarbon radicals, or the two hydrocarbon radicals together with the P atom can form a three- to eight-membered ring. The phosphine group preferably contains two identical hydrocarbon radicals. The hydrocarbon radicals can be unsubstituted or substituted and can contain from 1 to 22 C atoms, preferably from 1 to 12 C atoms. Among the compounds of the formulae I and Ia, particular preference is given to those in which the phosphine group contains two identical or different radicals selected from the group consisting of linear or branched C₁-C₁₂-alkyl; unsubstituted or C₁-C₆-alkyl- or C₁-C₆-alkoxy-substituted C₅-C₁₂-cycloalkyl or C₅-C₁₂-cycloalkyl-CH₂-; phenyl or benzyl; and phenyl or benzyl substituted by halogen (for example F, Cl and Br), C₁-C₆-alkyl, C₁-C₆-haloalkyl (for example trifluoromethyl), C₁-C₆-alkoxy, C₁-C₆-haloalkoxy (for example trifluoromethoxy), (C₆H₅)₃Si, (C₁-C₁₂-alkyl)₃Si, secondary amino or -CO₂-C₁-C₆-alkyl (for example -CO₂CH₃).

The two radicals in the phosphine group can together also form unsubstituted or halogen-, C₁-C₆-alkyl- or C₁-C₆-alkoxy-substituted dimethylene, trimethylene, tetramethylene or pentamethylene. The substituents are preferably bound in the two ortho positions relative to the P atom.

The phosphine groups can be groups of the formulae 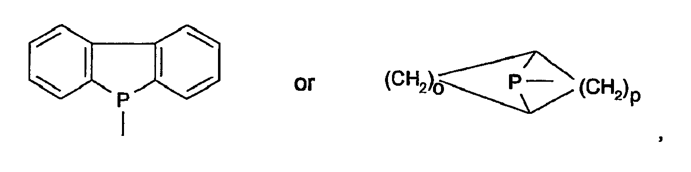 or 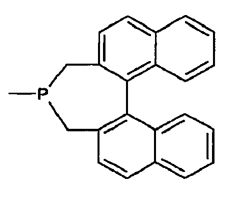 where o and p are each, independently of one another, an integer from 2 to 10 and the sum of o+p is from 4 to 12, preferably from 5 to 8, and the phenyl rings are unsubstituted or substituted by C₁-C₄-alkyl and/or C₁-C₄-alkoxy. Examples are [3.3.1]phobyl and [4.2.1]phobyl of the formulae 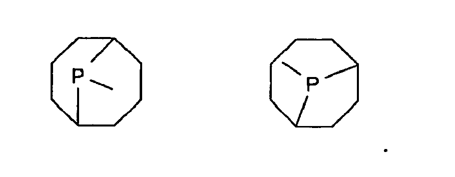 Examples of secondary phosphine groups in which the two hydrocarbon radicals together with the P atom form a 3- to 8-membered ring are, in particular, groups of the formula 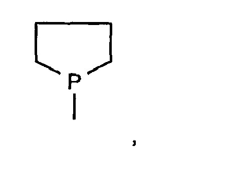 which may be substituted by C₁-C₄-alkyl or C₁-C₄-alkoxy in one or both of the ortho positions and, if desired, the meta positions relative to the P atom.

Examples of alkyl substituents, preferably containing from 1 to 6 C atoms, on P are methyl, ethyl, n-propyl, i-propyl, n-butyl, i-butyl, t-butyl and the isomers of pentyl and hexyl. Examples of unsubstituted or alkyl-substituted cycloalkyl substituents on P are cyclopentyl, cyclohexyl, methylcyclohexyl and ethylcyclohexyl and dimethylcyclohexyl. Examples of alkyl-, alkoxy-, haloalkyl- and/or haloalkoxy-substituted phenyl and benzyl substituents on P are methylphenyl, dimethylphenyl, trimethylphenyl, ethylphenyl, methylbenzyl, methoxyphenyl, dimethoxyphenyl, trifluoromethylphenyl, bistrifluoromethylphenyl, tristrifluoromethylphenyl, trifluoromethoxyphenyl and bistrifluoromethoxyphenyl.

When X₁ is a secondary phosphino group containing O atoms, the substituents on P can be, for example, linear or branched C₁-C₁₂-alkoxy; unsubstituted or C₁-C₆-alkyl- or C₁-C₆-alkoxy-substituted C₅-C₁₂-cycloalkoxy or C₅-C₁₂-cycloalkylmethoxy; phenoxy or benzyloxy, where the cyclic radicals are substituted by halogen (for example F, Cl and Br), C₁-C₆-alkyl, C₁-C₆-haloalkyl (for example trifluoromethyl), C₁-C₆-alkoxy, C₁-C₆-haloalkoxy (for example trifluoromethoxy), (C₆H₅)₃Si, (C₁-C₁₂-alkyl)₃Si, secondary amino or -CO₂-C₁-C₆-alkyl (for example -CO₂CH₃). Some examples are methoxy, ethoxy, n- and i-propoxy, n-, i- and t-butoxy, cyclohexyloxy, phenoxy and benzyloxy.

When X₁ is a secondary phosphino group containing N atoms, the substituents on P can be, for example, open-chain or cyclic secondary amino or disulphonylamino. Some examples are dimethylamino, diethylamino, di-n- and i-propylamino, di-n-butylamino, methyl-propylamino, phenylmethylamino, pyrrolidin-N-yl, piperidin-N-yl, morpholin-N-yl, di(methylsulphonyl)amido, di(ethylsulphonyl)amido, di(propylsulphonyl)amido, di(butylsulphonyl)amido, di(methylsulphonyl)amido, di(p-toluenesulphonyl)amido, di(trifluoromethylsulphonyl)amido.

Examples of bivalent radicals forming a ring are -(C₁-C₄-alkyl)N-C(R')₂-[C(R")₂]₁₋₄-N(C₁-C₄-alkyl)-, -O-C(R')₂-[C(R")₂]₁₋₄-N(C₁-C₄-alkyl)-, -O-C(R')₂-[C(R")₂]₁₋₄-O-, -CH₂-CH₂-CH₂-O- and -CH₂-CH₂-CH₂-N(C₁-C₄-alkyl)-, where R' and R" are each, independently of one another, hydrogen or C₁-C₄-alkyl. Other examples of cyclic phosphine groups having O atoms bound in the α position are the groups of the formulae

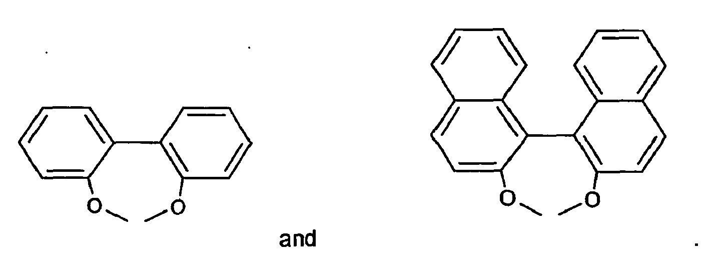

Preferred phosphine groups X₁ are ones which contain identical or different, preferably identical, radicals selected from the group consisting of C₁-C₆-alkyl, unsubstituted cyclopentyl or cyclohexyl and cyclopentyl or cyclohexyl bearing from 1 to 3 C₁-C₄-alkyl or C₁-C₄-alkoxy groups as substituents, benzyl and in particular phenyl, which are unsubstituted or substituted by from 1 to 3 C₁-C₄-alkyl. C₁-C₄-alkoxy, F, Cl, C₁-C₄-fluoroalkyl or C₁-C₄-fluoroalkoxy.

In the compounds of the formula I, X₁ is preferably the group -PR₁R₂, where R₁ and R₂ are each, independently of one another, a hydrocarbon radical which has from 1 to 20 C atoms and is unsubstituted or substituted by halogen, C₁-C₆-alkyl, C₁-C₆-haloalkyl, C₁-C₆-alkoxy, C₁-C₆-haloalkoxy, (C₆H₅)₃Si, (C₁-C₁₂-alkyl)₃Si or -CO₂-C₁-C₆-alkyl; or R₁ and R₂ together form unsubstituted or C₁-C₄-alkyl- or C₁-C₄-alkoxy-substituted dimethylene, trimethylene, tetramethylene or pentamethylene.

R₁ and R₂ are preferably identical or different, in particular identical, radicals selected from the group consisting of branched C₃-C₆-alkyl, unsubstituted cyclopentyl or cyclohexyl and cyclopentyl or cyclohexyl bearing from one to three C₁-C₄-alkyl or C₁-C₄-alkoxy groups as substituents, unsubstituted benzyl and benzyl bearing from one to three C₁-C₄-alkyl or C₁-C₄-alkoxy groups as substituents and in particular unsubstituted phenyl and phenyl substituted by from one to three C₁-C₄-alkyl, C₁-C₄-alkoxy, -NH₂, OH, F, Cl, C₁-C₄-fluoroalkyl or C₁-C₄-fluoroalkoxy groups.

R₁ and R₂ are particularly preferably identical or different, in particular identical, radicals selected from the group consisting of unsubstituted phenyl and phenyl substituted by from one to three C₁-C₄-alkyl, C₁-C₄-alkoxy or C₁-C₄-fluoroalkyl groups.

The radicals R₃ and R₄ can be unsubstituted or substituted, for example by C₁-C₆-alkyl, C₁-C₆-alkoxy, cyclohexyl, C₆-C₁₀-aryl, C₇-C₁₂-aralkyl, C₁-C₄-alkyl-C₆-C₁₀-aryl, C₁-C₄-alkoxy-C₆-C₁₀-aryl, C₁-C₄-alkyl-C₇-C₁₂-aralkyl, C₁-C₄-alkoxy-C₁-C₁₂-aralkyl, -CO-OR₅, halogen (preferably F or Cl), -CO-NR₆R₇ or-NR₆R₇, where R₅ is H, an alkali metal, C₁-C₆-alkyl, cyclohexyl, phenyl or benzyl, and R₆ and R₇ are each, independently of one another, hydrogen, C₁-C₆-alkyl, cyclohexyl, phenyl or benzyl, or R₆ and R₇ together form tetramethylene, pentamethylene or 3-oxapentylene.

The hydrocarbon radical R₃ preferably contains from 1 to 16 and particularly preferably from 1 to 12 C atoms. The hydrocarbon radical R₃ can be C₁-C₁₈-alkyl, preferably C₁-C₁₂-alkyl and particularly preferably C₁-C₈-alkyl; C₃-C₁₂-cycloalkyl, preferably C₄-C₈-cycloalkyl and particularly preferably C₅-C₆-cycloalkyl; or C₆-C₁₆-aryl and preferably C₆-C₁₂-aryl.

When R₃ is alkyl, it is preferably C₁-C₈-alkyl. Examples of alkyl are methyl, ethyl, propyl, butyl, pentyl, hexyl, heptyl, octyl, nonyl, decyl, undecyl, dodecyl, tridecyl, tetradecyl, pentadecyl, hexadecyl, heptadecyl, octadecyl and eicosyl. Examples of branched alkyl are isopropyl, isobutyl, tert-butyl, isopentyl, isohexyl and 1,1,2,2-tetramethylethyl.

When R₃ is cycloalkyl, it can be, for example, cyclopropyl, cyclobutyl, cyclopentyl, cyclohexyl, cycloheptyl, cyclooctyl, cyclodecyl or cyclododecyl.

The aromatic hydrocarbon radical R₃ preferably contains from 6 to 18 and particularly preferably from 6 to 14 C atoms. The heteroaromatic hydrocarbon radical R₃ preferably contains from 3 to 14 and particularly preferably from 3 to 11 C atoms. The hydrocarbon radical R₃ can be C₆-C₁₄-aryl and preferably C₆-C₁₀-aryl, or C₃-C₁₁-aryl and preferably C₄-C₁₀-heteroaryl.

Some examples of aryl are phenyl, naphthyl, anthracenyl, phenanthryl and biphenyl.

The heterohydrocarbon radical R₃ preferably contains a total of from 2 to 16 atoms, particularly preferably a total of from 2 to 12 atoms, and from 1 to 3 heteroatoms selected from the group consisting of O, S and NR. The heterohydrocarbon radical R₃ can be C₂-C₁₈-heteroalkyl, preferably C₂-C₁₂-heteroalkyl and particularly preferably C₂-C₈-heteroalkyl; C₃-C₁₂-heterocycloalkyl, preferably C₄-C₈-heterocycloalkyl and particularly preferably C₄-C₅-heterocycloalkyl; or C₃-C₁₆-heteroaryl and preferably C₄-C₁₁-heteroaryl.

When R₃ is heteroaryl, it is preferably C₂-C₈-alkyl. Examples of heteroalkyl are methoxymethyl, methoxyethyl, ethoxymethyl, ethoxyethyl, ethoxypropyl, isopropoxymethyl, isopropoxyethyl, isobutoxyethyl, tert-butoxyethyl, methylthioethyl, dimethylaminoethyl.

When R₃ is heterocycloalkyl, it can be, for example, oxetanyl, tetrahydrofuranyl, oxacyclohexyl, dioxanyl, pyrrolidinyl or N-methylazacyclohexyl.

When R₃ is heteroaryl, it can be, for example, furanyl, thiophenyl, pyrrolyl, imidizolinyl, oxazolinyl, thiazolyl, pyrazolinyl, benzofuranyl, pyridinyl, pyrimidinyl, pyridazinyl, pyrazinyl, quinazolinyl, quinoxalinyl, indolyl, benzimidazolyl, quinolinyl, isoquinolinyl or acridinyl.

Preferred substituents on R₃ are C₁-C₄-alkyl, C₁-C₄-alkoxy, cyclohexyl, C₆-C₁₀-aryl, C₇-C₁₂-aralkyl, C₁-C₄-alkyl-C₆-C₁₀-aryl, C₁-C₄-alkoxy-C₆-C₁₀-aryl, C₁-C₄-alkyl-C₇-C₁₂-aralkyl, C₁-C₄-alkoxy-C₇-C₁₂-aralkyl, -CO-OR₅, halogen (preferably F or Cl), -CO-NR₆R₇ or -NR₆R₇, where R₅ is C₁-C₆-alkyl, cyclohexyl, phenyl or benzyl, and R₆ and R₇ are each, independently of one another, hydrogen, C₁-C₆-alkyl, cyclohexyl, phenyl or benzyl, or R₆ and R₇ together form tetramethylene, pentamethylene or 3-oxapentylene.

In a preferred subgroup, R₃ is a hydrocarbon radical selected from the group consisting of C₁-C₁₂-alkyl, C₅-C₆-cycloalkyl and C₆-C₁₂-aryl, where the cyclic radicals are unsubstituted or substituted by halogen (F, Cl, Br), C₁-C₄-alkyl, C₁-C₄-perfluoroalkyl or C₁-C₄-alkoxy.

A hydrocarbon radical R₄ preferably contains from 1 to 16, particularly preferably from 1 to 12 and very particularly preferably from 1 to 8, C atoms. The hydrocarbon radical R₄ can be C₁-C₁₈-alkyl, preferably C₁-C₁₂-alkyl and particularly preferably C₁-C₈-alkyl; C₃-C₁₂-cycloalkyl, preferably C₄-C₈-cycloalkyl and particularly preferably C₅-C₆-cycloalkyl; C₆-C₁₆-aryl and preferably C₆-C₁₂-aryl, or C₇-C₁₆-aralkyl and preferably C₇-C₁₂-aralkyl.

When the two radicals R₄ together form a hydrocarbon radical, this is alkylene which preferably contains from 3 to 7 and particularly preferably from 4 to 6 C atoms. Examples are 1,3-propylene, 1,3-or 1,4-butylene, 1,3-, 1,4- or 1,5-pentylene and 1,3-, 1,4-, 1,5-, 2,5-, 2,6- or 1,6-hexylene.

When R₄ is alkyl, it is preferably linear or branched C₁-C₈-alkyl. Examples of alkyl are methyl, ethyl, propyl, butyl, pentyl, hexyl, heptyl, octyl, nonyl, decyl, undecyl, dodecyl, tridecyl, tetradecyl, pentadecyl, hexadecyl, heptadecyl, octadecyl and eicosyl. Examples of branched alkyl are isopropyl, isobutyl, tert-butyl, isopentyl, isohexyl and 1,1,2,2-tetramethylethyl.

When R₄ is cycloalkyl, it can be, for example, cyclopropyl, cyclobutyl, cyclopentyl, cyclohexyl, cycloheptyl, cyclooctyl, cyclodecyl or cyclododecyl.

When R₄ is aryl, it can be, for example, phenyl, naphthyl, anthracenyl, phenanthrenyl or biphenyl.

When R₄ is aralkyl, it can be benzyl or naphthylmethyl.

Preferred substituents on R₄ are halogen (F, Cl, Br), C₁-C₄-alkyl or C₁-C₄-alkoxy.

In a preferred subgroup, R₄ is a hydrocarbon radical selected from the group consisting of C₁-C₆-alkyl, C₅-C₆-cycloalkyl and benzyl, where the cyclic radicals are unsubstituted or substituted by halogen (F, Cl, Br), C₁-C₄-alkyl, C₁-C₄-haloalkyl (for example trifluoromethyl) or C₁-C₄-alkoxy.

A hydrocarbon radical R₀₁ preferably contains from 1 to 16, particularly preferably from 1 to 12 and very particularly preferably from 1 to 8, C atoms. The hydrocarbon radical R₀₁ can be C₁-C₁₈-alkyl, preferably C₁-C₁₂-alkyl and particularly preferably C₁-C₈-alkyl; C₃-C₁₂-cycloalkyl, preferably C₄-C₈-cycloalkyl and particularly preferably C₅-C₆-cycloalkyl; C₆-C₁₆-aryl and preferably C₆-C₁₂-aryl, or C₇-C₁₆-aralkyl and preferably C₇-C₁₂-aralkyl. The embodiments and preferences given for R₄ apply independently to R₀₁, R₀₂ and R'₀₂. In a particularly preferred embodiment, R₀₁ is α-branched alkyl having at least 3 C atoms, for example α-branched C₃-C₁₂-alkyl and more preferably C₃-C₈-alkyl. Examples of α-branched alkyl are i-propyl, but-2-yl, t-butyl, pent-2- or -3-yl, hex-2- or -3-yl, hept-2-, -3- or -4-yl and isooctyl (1,1,3,3,3-pentamethyl-prop-1-yl).

When R₀₁ and R₀₂ together with the C atoms to which they are bound form a three- to eight-membered hydrocarbon or heterohydrocarbon ring, the rings are aliphatic, olefinically unsaturated or aromatic fused ring systems preferably having from 3 to 8 and particularly preferably 5 or 6 ring atoms. Examples of fused aliphatic hydrocarbon rings are cyclopropane-1,2-diyl, cyclobutane-1,2-diyl, cyclopentane-1,2-diyl, cyclohexane-1,2-diyl, cycloheptane-1,2-diyl and cyclooctane-1,2-diyl. Examples of fused heteroaliphatic hydrocarbon rings are oxetane-1,2-diyl, tetrahydrofuran-1,2-diyl, oxacyclohex-1,2-diyl, dioxane-1,2-diyl, pyrrolidine-1,2-diyl and N-methylazacyclohex-1,2-diyl. Examples of fused aromatic hydrocarbon rings are 1,2-phenylene and 1,2-naphthylene. Examples of fused heteroaromatic hydrocarbon rings are furan-1,2-diyl, thiophene-1,2-diyl, pyrrole-1,2-diyl, imidazoline-1,2-diyl, oxazoline-1,2-diyl, thiazole-1,2-diyl, pyrazoline-1,2-diyl, benzofuran-1,2-diyl, pyridine-1,2-diyl, pyrimidine-1,2-diyl, pyridazine-1,2-diyl, pyrazine-1 ,2-diyl, quinazoline-1,2-diyl, quinoxaline-1,2-diyl, indole-1,2-diyl, benzimidazole-1,2-diyl, quinoline-1,2-diyl, isoquinoline-1,2-diyl and acridine-1,2-diyl.

When R₀₂ and R'₀₂ are different radicals or R₀₁ and R₀₂ together form a ring, the compounds of the formulae I and la contain a further chiral C atom. The invention encompasses racemates or diastereomers of these compounds. The relative configuration of the diastereomers can have a positive influence on the enantioselectivity in addition reactions which are catalyzed according to the invention. Preference is given to R₀₂ and R'₀₂ being hydrogen. In another preferred group, R₀₂ and R'₀₂ are each hydrogen and R₀₁ is α-branched C₃-C₈-alkyl.

A preferred subgroup of the compounds of the invention is made up of compounds of the formulae Ib and Ic, 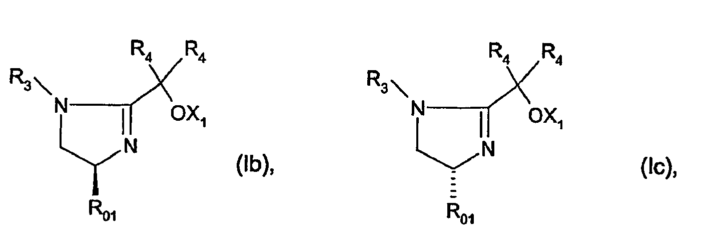 where
X₁ is -PR₁R₂,
R₁ and R₂ are identical or different and in particular identical radicals selected from the group consisting of α-branched C₃-C₆-alkyl, unsubstituted C₅-C₇-cycloalkyl and C₅-C₇-cycloalkyl bearing from one to three C₁-C₄-alkyl or C₁-C₄-alkoxy groups as substituents and
unsubstituted phenyl and phenyl bearing from one to three C₁-C₄-alkyl, C₁-C₄-alkoxy or C₁-C₄-fluoroalkyl groups as substituents and unsubstituted or C₁-C₄-alkyl- or C₁-C₄-alkoxy-substituted dimethylene, trimethylene, tetramethylene and hexamethylene;
R₃ is benzyl or C₆-C₁₂-aryl, and aryl and benzyl are unsubstituted or substituted by halogen, C₁-C₄-alkyl, C₁-C₄-haloalkyl or C₁-C₄-alkoxy;
R₄ is C₁-C₆-alkyl or benzyl, and
R₀₁ is α-branched C₃-C₈-alkyl.

The compounds of the formulae I and la can be prepared in a manner known per se by reacting imidazolinemethanols with secondary halophosphines in the presence of organic metal compounds, for example lithium alkyls. The preparation of imidazolinemethanols is described by M. Casey et al. in Synlett 2003, No. 1, pages 102 to 106.

The compounds of the formulae I and Ia can be prepared in a few process steps by a novel process via a haloimine ester as central intermediate. The novel process makes it possible to obtain different combinations of substituents.

The invention further provides a process for preparing compounds of the formulae I and la, 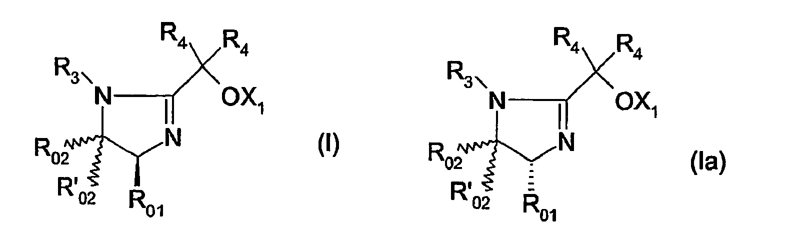 where R₀₁, R₀₂, R'₀₂, R₃, R₄ and X₁ are as defined above and - represents the R or S form, which is characterized in that
a) a compound of the formula II 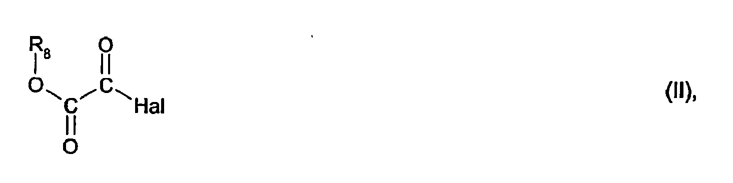 where R₈ is C₁-C₈-alkyl and Hal is Cl, Br or I, is reacted in the presence of a tertiary amine with at least an equivalent amount of a compound of the formula III, 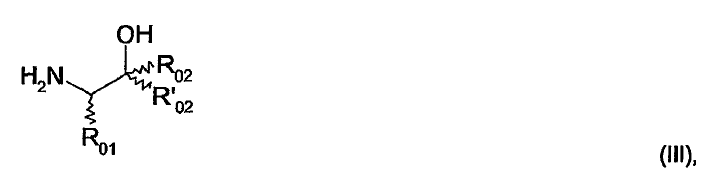 where R₀₁ and R₀₂ are as defined above, to form a compound of the formula IV, 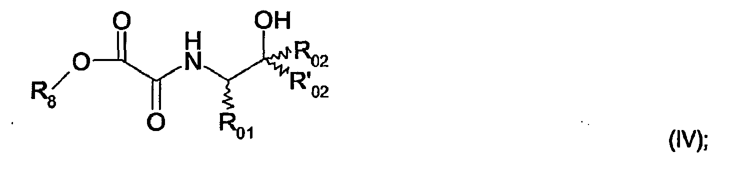
b) the compound of the formula IV is reacted with at least equivalent amounts of a halogenating agent to form a compound of the formula V, 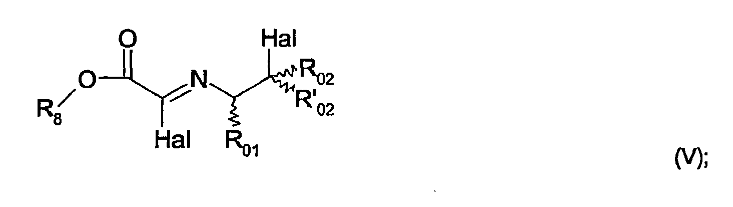
c) the compound of the formula V is cyclized with a primary amine of the formula R₃-NH₂ (X) in the presence of a tertiary amine to form a compound of the formula VI, 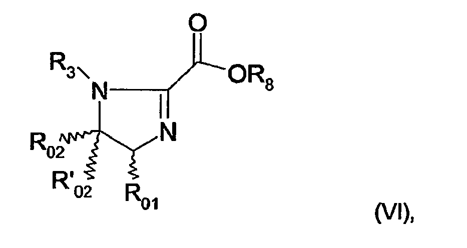
d) the compound of the formula VI is reacted with at least two equivalents of an organometallic compound of the formula VII or at least one equivalent of an organometallic compound of the formula VIIa

   R₄-X₂ (VII),

   R₄-(X₂)₂ (VIIa),

   where R₄ is as defined above, X₂ is an alkali metal or -Me₁X₃, Me₁ is Mg or Zn, and X₃ is Cl, Br or I, to form a compound of the formula VIII 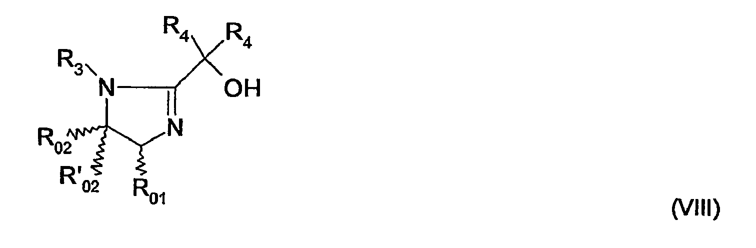 and
e) the hydroxyl group of the compound of the formula VIII is metallated and subsequently reacted with a halophosphine of the formula IX,

   X₁-Y₁ (IX),

   where X₁ is as defined above and Y₁ is Cl, Br or I, to give a compound of the formula la or Ib.

Hal is preferably Cl or Br and particularly preferably Cl. R₈ is preferably C₁-C₄-alkyl and particularly preferably isopropyl.

### Process step a

Oxalic monoester halides are known and some are commercially available, or they can be prepared in a simple manner by esterification of oxalic monohalides. The reaction is advantageously carried out at temperatures of from -20 to 20°C. The reaction is advantageously carried out without solvents.

Compounds of the formula III are likewise known and commercially available or can be prepared by known methods or methods analogous to these.

The reaction is advantageously carried out in inert solvents such as alkanols (methanol, ethanol, ethylene glycol, ethylene glycol monomethyl ether), ethers (diethyl ether, dibutyl ether, tetrahydrofuran and dioxane) or halogenated hydrocarbons (methylene chloride, chloroform, tetrachloroethane and chlorobenzene) at low temperatures (for example from -20 to 20°C).

The tertiary amines serve to bind hydrogen halide formed and are advantageously added in at least equimolar amounts. Examples of suitable tertiary amines are trialkylamines (trimethylamine, triethylamine, tripropylamine, tributylamine, methyldiethylamine or dimethylethylamine) and cyclic or polycyclic amines whose N atom(s) is/are substituted by C₁-C₄-alkyl (N-methylpiperidine and N-methylmorpholine).

The compounds of the formula IV are obtained in high yields. They can be isolated and purified in a known manner.

### Process step b)

The rearrangement of the compounds of the formula IV with halogenation to form haloimines of the formula V is advantageously carried out at relatively high temperatures, for example from 50 to 150°C. If the halogenating agent is liquid, no solvent has to be used. The reaction can and in the case of solid halogenating agents is carried out in the presence of an inert solvent such as a halogenated hydrocarbons (methylene chloride, chloroform, tetrachloroethane and chlorobenzene). To accelerate the reaction, it is possible to employ halogenation catalysts, for example tertiary amines, N,N-dialkylated acid amides or N-alkylated lactams (trimethylamine, triethylamine, tributylamine, diazabicycloundecane, dimethylformamide, dimethylacetamide, N-methylpyrrolidone). The amount is, for example, from 0.1 to 5 mol%, based on the compound of the formula IV. The halogenation catalyst can also be used simultaneously as solvent. Suitable halogenating agents are, for example, SOCl₂, SOBr₂, PCl₃, PCl₅ and OPCl₃. The halogenating agent is advantageously used in excess. The haloimines of the formula V are obtained in very high yields.

### Process step c)

The cyclization of the haloimines to form compounds of the formula IV is advantageously carried out at relatively high temperatures, for example from 70 to 150°C, and in the presence of an inert solvent. Suitable solvents are, for example, aromatic hydrocarbons (benzene, toluene, xylene) or halogenated hydrocarbons (methylene chloride, chloroform, tetrachloroethane and chlorobenzene). The tertiary amines serve to bind hydrogen halide formed and they are advantageously added in at least equimolar amounts. Suitable tertiary amines are, for example, trialkylamines (trimethylamine, triethylamine, tripropylamine, tributylamine, methyldiethylamine or dimethylethylamine) and cyclic or polycyclic amines whose N atom(s) is/are substituted by C₁-C₄-alkyl (N-methylpiperidine and N-methylmorpholine). The amines of the formula X are added in equimolar amounts or in a slight excess.

### Process step d)

The reaction of carboxylic esters with metal-hydrocarbon compounds or metal halide-hydrocarbon compounds is known per se. When X₂ is an alkali metal, it can be Na, K and in particular Li. In the group Me₁X₃, Me₁ can be, for example, Mg or Zn. The reaction is advantageously carried out by adding the compound of the formula VII or VIIa at low temperatures, for example from -30 to -80°C, to a solution of the compound of the formula VI and then allowing the mixture to warm up, for example to room temperature. The reaction can then be completed at this temperature or higher temperatures (up to the boiling point of solvents used). Suitable solvents are, in particular, ethers such as diethyl ether, dibutyl ether, tetrahydrofuran and dioxane.

### Process step e)

The metallation of the compound of the formula VIII to form a metal alkoxide can be effected by means of alkali metal alkyls and in particular a lithium alkyl, for example methyllithium, ethyllithium, propyllithium or butyllithium, or by means of Grignard reagents such as methylmagnesium, ethylmagnesium, propylmagnesium, butylmagnesium or benzylmagnesium halides. It is advantageous to use equivalent amounts or a slight excess of alkali metal alkyls or Grignard reagents. The addition is advantageously carried out at relatively low temperatures, for example from -20 to -80°C. The presence of tertiary amines such as trimethylamine, triethylamine, tributylamine or tetramethylethylenediamine can be advantageous. The reaction can subsequently be brought to completion at room temperature, the halophosphine of the formula IX is added and the reaction can be completed at this temperature. The reaction is preferably carried out in the presence of inert solvents, for example ethers or hydrocarbons (pentane, hexane, cyclohexane, methylcyclohexane, benzene, toluene or xylene).

The compounds of the formulae la and lb are obtained in good overall yields. Choice of the starting compounds enables the compounds of the invention to be built up in a modular fashion, with the simple starting compounds making possible a wide variety of substitutions in respect of R₃ and R₄.

The novel compounds of the formula I and la are ligands for complexes of metals selected from the group of TM8 metals, in particular from the group consisting of Ru, Rh and lr, which are excellent catalysts or catalyst precursors for asymmetric syntheses, for example the asymmetric hydrogenation of prochiral, unsaturated, organic compounds. If prochiral unsaturated organic compounds are used, a very high excess of optical isomer can be induced in the synthesis of organic compounds and a high chemical conversion can be achieved in short reaction times. The enantioselectivity in the case of selected substrates is very high in comparison with known ligands.

The invention further provides complexes of metals selected from the group of TM8 metals with compounds of the formulae I and la as ligands.

Possible metals are, for example, Cu, Ag, Au, Ni, Co, Rh, Pd, Ir, Ru and Pt. Preferred metals are rhodium and iridium and also ruthenium, platinum and palladium.

Particularly preferred metals are ruthenium, rhodium and iridium.

Depending on the oxidation number and coordination number of the metal atom, the metal complexes can contain further ligands and/or anions. They can also be cationic metal complexes. Such analogous metal complexes and their preparation are widely described in the literature.

The metal complexes can, for example, have the general formulae XI and XII,

A₁MeLₙ (XI),

(A₁MeLₙ)^{(z+)}(E⁻)_{z} (XII),

where A₁ is a compound of the formula I or la,
L represents identical or different monodentate, anionic or nonionic ligands, or two L together represent identical or different bidentate, anionic or nonionic ligands;
n is 2, 3 or 4 when L is a monodentate ligand, or n is 1 or 2 when L is a bidentate ligand;
z is 1,2 or 3;
Me is a metal selected from the group consisting of Rh and Ir, with the metal having the oxidation state 0, 1, 2, 3 or 4;
E⁻ is the anion of an oxo acid or complex acid; and
the anionic ligands balance the charge of the oxidation state 1, 2, 3 or 4 of the metal.

The above-described preferences and embodiments apply to the compounds of the formulae XI and XII.

Monodentate nonionic ligands can, for example, be selected from the group consisting of olefins (for example ethylene, propylene), allyls (allyl, 2-methallyl), solvating solvents (nitriles, linear or cyclic ethers, unalkylated or N-alkylated amides and lactams, amines, phosphines, alcohols, carboxylic esters, sulphonic esters), nitrogen monoxide and carbon monoxide.

Monodentate anionic ligands can, for example, be selected from the group consisting of halide (F, Cl, Br, I), pseudohalide (cyanide, cyanate, isocyanate) and anions of carboxylic acids, sulphonic acids and phosphonic acids (carbonate, formate, acetate, propionate, methylsulphonate, trifluoromethylsulphonate, phenylsulphonate, tosylate).

Bidentate nonionic ligands can, for example, be selected from the group consisting of linear or cyclic diolefins (for example hexadiene, cyclooctadiene, norbomadiene), dinitriles (malononitrile), unalkylated or N-alkylated diamides of carboxylic acids, diamines, diphosphines, diols, acetylacetonates, diesters of dicarboxylic acids and diesters of disulphonic acids.

Bidentate anionic ligands can, for example, be selected from the group consisting of the anions of dicaboxylic acids, disulphonic acids and diphosphonic acids (for example oxalic acid, malonic acid, succinic acid, maleic acid, methylenedisulphonic acid and methylenediphosphonic acid).

Preferred metal complexes also include those in which E is -Cl⁻, -Br⁻, -I⁻, ClO₄⁻, CF₃SO₃⁻, CH₃SO₃, HSO₄⁻, BF₄⁻, B(phenyl)₄⁻, B(C₆F₅)₄⁻, B(3,5-bistrifluoromethylphenyl)₄⁻ (BAR_{F}), tetra-(C₁-C₆-perfluoroalkyl)aluminates such as (CF₅CF₂O)₄Al⁻, PF₆⁻, SbCl₆⁻, AsF₆⁻ or SbF₆⁻.

Particularly preferred metal complexes which are particularly suitable for hydrogenations have the formulae XIII and XIV,

[A₁Me₁YZ] (XIII),

[A₁Me₁Y]⁺E₁⁻ (XIV),

where
A₁ is a compound of the formula I or la;
Me₁ is rhodium or iridium;
Y represents two olefins or a diene;
Z is Cl, Br or I; and
E₁⁻ is the anion of an oxo acid or complex acid.

The above-described embodiments and preferences apply to the compounds of the formulae I and Ia.

When Y is an olefin, it can be a C₂-C₁₂-, preferably C₂-C₆- and particularly preferably C₂-C₄-olefin. Examples are propene, 1-butene and in particular ethylene. The diene can contain from 5 to 12 and preferably from 5 to 8 C atoms and can be an open-chain, cyclic or polycyclic diene. The two olefin groups of the diene are preferably joined by one or two CH₂ groups. Examples are 1,3-pentadiene, cyclopentadiene, 1,5-hexadiene, 1,4-cyclohexadiene, 1,4- or 1,5-heptadiene, 1,4- or 1,5-cycloheptadiene, 1,4- or 1,5-octadiene, 1,4- or 1,5-cyclooctadiene and norbornadiene. Y preferably represents two ethylene or 1,5-hexadiene, 1,5-cyclooctadiene or norbornadiene.

Z in the formula XIII is preferably Cl or Br. Examples of E₁ are ClO₄⁻, CF₃SO₃⁻, CH₃SO₃⁻, HSO₄⁻, BF₄⁻, B(phenyl)₄⁻, BARF, PF₆⁻, SbCl₆⁻, AsF₆⁻ or SbF₆⁻.

Ruthenium complexes according to the invention can, for example, have the formula XV

[RuₐH_{b}Z_{c}(A₁)_{d}Lₑ]r(E^{k})_{g}(S)ₕ (XV),

where Z is Cl, Br or I; A₁ is a compound of the formula I or la; L represents identical or different ligands; E⁻ is the anion of an oxo acid, mineral acid or complex acid; S is a solvent capable of coordination as ligand; and a is from 1 to 3, b is from 0 to 4, c is from 0 to 6, d is from 1 to 3, e is from 0 to 4, f is from 1 to 3, g is from 1 to 4, h is from 0 to 6 and k is from 1 to 4, with the total charge on the complex being 0.

The above-described preferences for Z, A₁, L and E⁻ apply to the compounds of the formula XV. The ligands L can additionally be arenes or heteroarenes (for example benzene, naphthalene, methylbenzene, xylene, cumene, 1,3,5-mesitylene, pyridine, biphenyl, pyrrole, benzimidazole or cyclopentadienyl) and metal salts which act as Lewis acids (for example ZnCl₂, AlCl₃, TiCl₄ and SnCl₄). The solvent ligands can be, for example, alcohols, amines, acid amides, lactams and sulphones.

Complexes of this type are described in the references below and the references cited therein:
D. J. Ager, S. A. Laneman, Tetrahedron: Asymmetry, **8**, *1997*, 3327 - 3355;
T. Ohkuma, R. Noyori in Comprehensive Asymmetric Catalysis (E.N. Jacobsen, A. Pfaltz, H. Yamamoto, Eds.), Springer, Berlin, 1999, 199-246;
J. M. Brown in Comprehensive Asymmetric Catalysis (E.N. Jacobsen, A. Pfaltz, H. Yamamoto, Eds.), Springer, Berlin, 1999, 122 - 182;
T. Ohkuma, M. Kitamura, R. Noyori in Catalytic Asymmetric Synthesis, 2^{nd} Edition (I. Ojima, Ed.), Wiley-VCH New York, 2000, 1 - 110;
N. Zanetti, et al. Organometallics 15, 1996, 860.

The metal complexes of the invention are prepared by methods known from the literature (cf. US-A-5,371,256, US-A-5,446,844, US-A-5,583,241, and E. Jacobsen, A. Pfaltz, H. Yamamoto (Eds.), Comprehensive Asymmetric Catalysis I to III, Springer Verlag, Berlin, 1999, and references cited therein).

The metal complexes of the invention act as homogeneous catalysts or catalyst precursors which can be activated under the reaction conditions and can be used for asymmetric addition reactions of prochiral, unsaturated, organic compounds.

The metal complexes can, for example, be used for the asymmetric hydrogenation (addition of hydrogen) or transfer hydrogenation in the presence of hydrogen donors such as methanol, ethanol, isopropanol or formic acid, of prochiral compounds having carbon-carbon or carbon-heteroatom double bonds. Such hydrogenations using soluble homogeneous metal complexes are described, for example, in Pure and Appl. Chem., Vol. 68, No. 1, pp. 131-138 (1996). Preferred unsaturated compounds to be hydrogenated contain the groups C=C, C=N and/or C=O. According to the invention, the hydrogenation is preferably carried out using metal complexes of ruthenium, rhodium and iridium.

The metal complexes of the invention can also be used as catalysts for the asymmetric hydroboration (addition of boron hydrides) of prochiral organic compounds having carbon-carbon double bonds. Such hydroborations are described, for example, by Tamio Hayashi in E. Jacobsen, A. Pfaltz, H. Yamamoto (Eds.), Comprehensive Asymmetric Catalysis I to III, Springer Verlag, Berlin, 1999, pages 351 to 364. Suitable boron hydrides are, for example, catecholboranes. The chiral boron compounds can be used in syntheses and/or can be converted in a manner known per se into other chiral organic compounds which are valuable building blocks for the preparation of chiral intermediates or active substances. An example of such a reaction is the preparation of 3-hydroxytetrahydrofuran (as described in DE 19,807,330).

The metal complexes of the invention can also be used as catalysts for the asymmetric hydrosilylation (addition of silanes) of prochiral organic compounds having carbon-carbon or carbon-heteroatom double bonds. Such hydrosilylations are described, for example, by G. Pioda and A. Togni in Tetrahedron: Asymmetry, 1998, 9, 3093, or by S. Uemura, et al. in Chem. Commun. 1996, 847. Suitable silanes are, for example, trichlorosilane or diphenylsilane. The hydrosilylation of, for example, C=O and C=N groups is preferably carried out using metal complexes of rhodium and iridium. The hydrosilylation of, for example, C=C groups is preferably carried out using metal complexes of palladium. The chiral silyl compounds can be used in syntheses and/or can be converted in a manner known per se into other chiral organic compounds which are valuable building blocks for the preparation of chiral intermediates or active substances. An example of such a reaction is hydrolysis to form alcohols.

The metal complexes of the invention can also be used as catalysts for asymmetric allylic substitution reactions (addition of C-nucleophiles onto allyl compounds). Such allylations are described, for example, by A. Pfaltz and M. Lautens in E. Jacobsen, A. Pfaltz, H. Yamamoto (Eds.), Comprehensive Asymmetric Catalysis I to III, Springer Verlag, Berlin, 1999, pages 833 to 884. Suitable precursors for allyl compounds are, for example, 1,3-diphenyl-3-acetoxy-1-propene or 3-acetoxy-1-cyclohexene. This reaction is preferably carried out using metal complexes of palladium. The chiral allyl compounds can be used in syntheses for preparing chiral intermediates or active substances.

The metal complexes of the invention can also be used as catalysts for asymmetric amination (addition of amines onto allyl compounds) or etherification (addition of alcohols or phenols onto allyl compounds). Such aminations and etherifications are described, for example, by A. Pfaltz and M. Lautens in E. Jacobsen, A. Pfaltz, H. Yamamoto (Eds.), Comprehensive Asymmetric Catalysis I to III, Springer Verlag, Berlin, 1999, pages 833 to 884. Suitable amines include both ammonia and primary and secondary amines. Suitable alcohols are phenols and aliphatic alcohols. The amination or etherification of allyl compounds is preferably carried out using metal complexes of palladium. The chiral amines and ethers can be used in syntheses for preparing chiral intermediates or active substances.

The metal complexes of the invention can also be used as catalysts for asymmetric isomerization, cf. M. Beller et al. in Transition Metals for Organic Synthesis, Volume 1, Wiley-VCH, Weinheim 1998, pages 147-156.

The invention also provides for the use of the metal complexes of the invention as homogeneous catalysts for preparing chiral organic compounds by asymmetric addition of hydrogen, boron hydrides or silanes onto a carbon-carbon or carbon-heteroatom multiple bond in prochiral organic compounds, or the asymmetric addition of C-nucleophiles or amines onto allyl compounds.

A further aspect of the invention is a process for preparing chiral organic compounds by asymmetric addition of hydrogen, boron hydrides or silanes onto a carbon-carbon or carbon-heteroatom multiple bond in prochiral organic compounds, or the asymmetric addition of C-nucleophiles, alcohols or amines onto allyl compounds in the presence of a catalyst, which is characterized in that the addition reaction is carried out in the presence of catalytic amounts of at least one metal complex according to the invention.

Preferred prochiral, unsaturated compounds to be hydrogenated can contain one or more, identical or different groups C=C, C=N and/or C=O in open-chain or cyclic organic compounds, with the groups C=C, C=N and/or C=O being able to be part of a ring system or being exocyclic groups. The prochiral unsaturated compounds can be alkenes, cycloalkenes, heterocycloalkenes, fused heteroaromatics or open-chain or cyclic ketones, ketimines and hydrazones of ketones. They can have, for example, the formula XVI,

R₁₅R₁₆C=D (XVI),

where R₁₅ and R₁₆ are selected so that the compound is prochiral and are each, independently of one another, an open-chain or cyclic hydrocarbon radical or heterohydrocarbon radical containing heteroatoms selected from the group consisting of O, S and N, each of which contains from 1 to 30 and preferably from 1 to 20 carbon atoms;
D is O or a radical of the formula CR₁₇R₁₈ or NR₁₉;
R₁₇ and R₁₈ have, independently of one another, the same meanings as R₁₅ and R₁₆,
R₁₉ is hydrogen, C₁-C₁₂-alkyl, C₁-C₁₂-alkoxy, C₃-C₁₂-cycloalkyl, C₃-C₁₂-cycloalkyl-C₁-C₆-alkyl,
C₃-C₁₁-heterocycloalkyl, C₃-C₁₁-heterocycloalkyl-C₁-C₆-alkyl, C₆-C₁₄-aryl, C₅-C₁₃-heteroaryl, C₇-C₁₆-aralkyl or C₆-C₁₄-heteroaralkyl,
R₁₅ and R₁₆ together with the C atom to which they are bound form a hydrocarbon ring or heterohydrocarbon ring having from 3 to 12 ring atoms;
R₁₅ and R₁₇ together with the C=C group to which they are bound form a hydrocarbon ring or heterohydrocarbon ring having from 3 to 12 ring atoms;
R₁₅ and R₁₉ together with the C=N group to which they are bound form a hydrocarbon ring or heterohydrocarbon ring having from 3 to 12 ring atoms;
the heteroatoms in the heterocyclic rings are selected from the group consisting of O, S and N;
and R₁₅, R₁₆, R₁₇, R₁₈ and R₁₉ are unsubstituted or substituted by C₁-C₆-alkyl, C₁-C₆-alkoxy, cyclohexyl, C₆-C₁₀-aryl, C₇-C₁₂-aralkyl, C₁-C₄-alkyl-C₆-C₁₀-aryl, C₁-C₄-alkoxy-C₆-C₁₀-aryl, Ci-C₄-alkyl-C₇-C₁₂-aralkyl, C₁-C₄-alkoxy-C₇-C₁₂-aralkyl, -OH, =O, -NR₂₁R₂₂, -CO-OR₂₀ or
-CO-NR₂₁R₂₂, where R₂₀ is H, an alkali metal, C₁-C₆-alkyl, cyclohexyl, phenyl or benzyl, and and R₂₁ and R₂₂ are each, independently of one another, hydrogen, C₁-C₆-alkyl, cyclohexyl, phenyl or benzyl, or R₂₁ and R₂₂ together form tetramethylene, pentamethylene or 3-oxtapentylene.

Examples of and preferences for substituents have been mentioned above.

R₁₅ and R₁₆ can each be, for example, C₁-C₂₀-alkyl and preferably C₁-C₁₂-alkyl, C₁-C₂₀-heteroalkyl and preferably C₁-C₁₂-heteroalkyl containing heteroatoms selected from the group consisting of O, S and N, C₃-C₁₂-cycloalkyl and preferably C₄-C₈-cycloalkyl, C-bonded C₃-C₁₁-heterocycloalkyl and preferably C₄-C₈-heterocycloalkyl containing heteroatoms selected from the group consisting of O, S and N, C₃-C₁₂-cycloalkyl-C₁-C₆-alkyl and preferably C₄-C₈-cycloalkyl-C₁-C₆-alkyl, C₃-C₁₁-heterocycloalkyl-C₁-C₆-alkyl and preferably C₄-C₈-heterocycloalkyl-C₁-C₆alkyl containing heteroatoms selected from the group consisting of O, S and N, C₆-C₁₄-aryl and preferably C₆-C₁₀-aryl, C₅-C₁₃-heteroaryl and preferably C₅-C₉-heteroaryl containing heteroatoms selected from the group consisting of O, S and N, C₇-C₁₅-aralkyl and preferably C₇-C₁₁-aralkyl, C₆-C₁₂-heteroaralkyl and preferably C₆-C₁₀-heteroaralkyl containing heteroatoms selected from the group consisting of O, S and N.

When R₁₅ and R₁₆, R₁₅ and R₁₇, or R₁₅ and R₁₉ in each case together with the group to which they are bound form a hydrocarbon ring or a heterohydrocarbon ring, the ring preferably contains from 4 to 8 ring atoms. The heterohydrocarbon ring can, for example, contain from 1 to 3 and preferably one or two heteroatoms.

R₁₉ is preferably hydrogen, C₁-C₆-alkyl, C₁-C₆-alkoxy, C₄-C₈-cycloalkyl, C₄-C₈-cycloalkyl-C₁-C₄-alkyl, C₄-C₁₀-heterocycloalkyl, C₄-C₁₀-heterocycloalkyl-C₁-C₄-alkyl, C₆-C₁₀-aryl, C₅-C₉-heteroaryl, C₇-C₁₂-aralkyl and C₅-C₁₃-heteroaralkyl.

Some examples of unsaturated organic compounds are imines of acetophenone, 4-methoxyacetophenone, 4-trifluoromethylacetophenone, 4-nitroacetophenone, 2-chloro-acetophenone, unsubstituted or substituted benzocyclohexanone or benzocyclopentanone, imines from the group consisting of unsubstituted or substituted tetrahydroquinoline, tetrahydropyridine and dihydropyrrole, and cis and trans isomers of prochiral olefins such as methylstilbene, methoxyphenylbutene, unsaturated carboxylic esters, amides and salts, for example α- and if appropriate β-substituted acrylic acids, crotonic acids or cinnamic acids, and olefinically unsaturated alcohols or ethers. Preferred carboxylic esters are those of the formula

R₂₃-CH=C(R₂₄)-C(O)OR₂₅

and also salts and amides of the acid, where R₂₃ is C₁-C₆-alkyl, unsubstituted C₃-C₈-cycloalkyl or C₃-C₈-cycloalkyl bearing from 1 to 4 C₁-C₆-alkyl, C₁-C₆-alkoxy, C₁-C₆-alkoxy-C₁-C₄-alkoxy groups as substituents, or unsubstituted C₆-C₁₀-aryl, preferably phenyl, or C₆-C₁₀-aryl, preferably phenyl, bearing from 1 to 4 C₁-C₆-alkyl, C₁-C₆-alkoxy, C₁-C₆-alkoxy-C₁-C₄-alkoxy groups as substituents, R₂₄ is linear or branched C₁-C₆-alkyl (for example isopropyl), cyclopentyl, cyclohexyl or phenyl each of which may be unsubstituted or substituted as defined above or protected amino (for example acetylamino), and R₂₅ is C₁-C₄-alkyl. Further suitable substrates for hydrogenation are, for example, prochiral allyl alcohols and β-enamides.

Suitable substrates for hydrogenation using ruthenium complexes are, for example, prochiral α- and β-ketocarboxylic salts, esters and amides, prochiral 1,3-diketones and prochiral ketones, α- and β-alkoxyketones and α- and β-hydroxyketones, α- and β-haloketones and α- and β-aminoketones.

The process of the invention can be carried out at low or elevated temperatures, for example temperatures of from -40 to 150°C, preferably from -20 to 100°C and particularly preferably from 0 to 80°C. The optical yields can be influenced by the choice of temperature, with relatively high optical yields also being achieved at relatively high temperatures.

The process of the invention can be carried out at atmospheric pressure or superatmospheric pressure. The pressure can be, for example, from 10⁵ to 2x10⁷ Pa (pascal). Hydrogenations are preferably carried out at atmospheric pressure or superatmospheric pressure.

Catalysts are preferably used in amounts of from 0.00001 to 10 mol%, particularly preferably from 0.0001 to 5 mol% and in particular from 0.01 to 5 mol%, based on the compound to be hydrogenated.

The preparation of the ligands and catalysts and also the addition reaction can be carried out without solvent or in the presence of an inert solvent, with one solvent or a mixture of solvents being able to be used. Suitable solvents are, for example, aliphatic, cycloaliphatic and aromatic hydrocarbons (pentane, hexane, petroleum ether, cyclohexane, methylcyclohexane, benzene, toluene, xylene), aliphatic halogenated hydrocarbons (methylene chloride, chloroform, dichloroethane and tetrachloroethane), nitriles (acetonitrile, propionitrile, benzonitrile), ethers (diethyl ether, dibutyl ether, t-butyl methyl ether, ethylene glycol dimethyl ether, ethylene glycol diethyl ether, diethylene glycol dimethyl ether, tetrahydrofuran, dioxane, diethylene glycol monomethyl or monoethyl ether), ketones (acetone, methyl isobutyl ketone), carboxylic esters and lactones (ethyl or methyl acetate, valerolactone), N-substituted lactams (N-methylpyrrolidone), carboxamides (dimethylacetamide, dimethylformamide), acyclic ureas (dimethylimidazoline) and sulphoxides and sulphones (dimethyl sulphoxide, dimethyl sulphone, tetramethylene sulphoxide, tetramethylene sulphone) and alcohols (methanol, ethanol, propanol, butanol, ethylene glycol monomethyl ether, ethylene glycol monoethyl ether, diethylene glycol monomethyl ether) and water. The solvents can be used alone or as mixtures of at least two solvents.

The reactions can be carried out in the presence of cocatalysts, for example quaternary ammonium halides (tetrabutylammonium iodide) and/or in the presence of protic complex acids, for example HBAr_{F} (cf., for example, US-A-5,371,256, US-A-5,446,844 and US-A-5,583,241 and EP-A-0 691 949). The cocatalysts are particularly useful for hydrogenations.

The metal complexes used as catalysts can be added as separately prepared, isolated compounds, or can be formed in situ prior to the reaction and then mixed with the substrate to be hydrogenated. It can be advantageous to add additional ligands in the reaction using isolated metal complexes, or to use an excess of the ligands in the in-situ preparation. The excess can be, for example, from 1 to 10 and preferably from 1 to 5 mol, based on the metal compound used for the preparation.

The catalysts to be used according to the invention can be prepared in situ prior to the reaction. For this purpose, it is possible, and this is a further subject-matter of the invention, for metal compound and ligand according to the invention to be marketed separately, if appropriate in solution, as a kit in one container each.

The process of the invention is generally carried out by placing the catalyst in a reaction vessel and then adding the substrate, if desired reaction auxiliaries and the compound to be added on, and subsequently start the reaction. Gaseous compounds to be added on, for example hydrogen or ammonia, are preferably introduced under pressure. The process can be carried out continuously or batchwise in various types of reactor.

The chiral organic compounds which can be prepared according to the invention are active substances or intermediates for the preparation of such substances, in particular in the field of production of pharmaceuticals, fragrances and agrochemicals.

The following examples illustrate the invention.

### A) Preparation of precursors and intermediates

Example A1: Preparation of 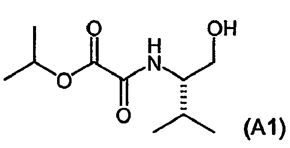
a) Oxalyl chloride (40 ml, 0.47 mol) is placed in a three-necked flask and cooled to 0°C by means of an ice bath. Isopropanol (18 ml, 0.24 mol) is slowly added from a dropping funnel. After warming to room temperature, the product (26.0 g, 36%, colourless oil) is distilled off by means of fractional distillation (atmospheric pressure) at 132°C.
   ¹H-NMR (400.1 MHz, CDCl₃, 300 K): δ=1.39 (d, J = 6.4 Hz, 6H, CH(CH₃)₂), 5.18 (sep, *J =* 6.4 Hz, 1H, CH(CH₃)₂) ppm.
   ¹³C-NMR (100.6 MHz, CDCl₃, 300 K): δ = 21.7 (2 x CH₃), 74.4 (CH), 155.6 (Cl-C=O), 161.7 (O-C=O) ppm.
b) (S)-Valinol (3.00 g, 29 mmol) is dissolved in 50 ml of isopropanol and admixed with triethylamine (4.10 ml, 29 mmol). While cooling in ice, the monoisopropyl ester chloride of oxalic acid (3.73 ml, 29 mmol) is slowly added. After stirring for 4 hours, the solution is evaporated under reduced pressure and then taken up in 110 ml of ethyl acetate/H₂O (12:1). The aqueous phase is extracted once with 15 ml of ethyl acetate and the combined organic extracts are washed three times with 5 ml each time of 2N aqueous HCl solution. The solution is subsequently dried over MgSO₄ and evaporated on a rotary evaporator. This gives 5.92 g (27.26 mmol, 94%) of a colourless solid.
   ¹H-NMR (400.1 MHz, CDCl₃, 300 K): δ = 0.93 (d, *J* = 6.8 Hz, 3H, CH(CH₃)₂), 0.96 (d, *J* = 6.8 Hz, 3H, CH(CH₃)₂), 1.36 (d, *J* = 6.4 Hz, 6H, OCH(CH₃)₂), 1.94 (sep, *J* = 6.8 Hz, 1H, CH(CH₃)₂), 2.31 (br s, 1H, OH), 3.72 (m, 3H, CH₂ and CH), 5.12 (sep, *J* = 6.4 Hz, 1H, OCH(CH₃)₂), 7.28 (br s, 1H, NH) ppm.
   ¹³C-NMR (100.6 MHz, CDCl₃, 300 K): δ=18.8 (CH(CH₃)₂), 19.5 (CH(CH₃)₂), 21.6 (OCH(CH₃)₂), 28.9 (CH(CH₃)₂), 57.8 (CH(CH₂), 63.1 (CH₂), 71.7 (OCH), 157.5 (N-C=O), 160.4 (O-C=O) ppm.

Example A2: Preparation of

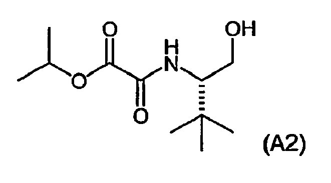

The procedure of Example A1b is repeated using 4.00 g (34 mmol) of (*S*)-*tert*-leucinol, giving 5.37 g (23.12 mmol, 68%) of the amide A2.
¹H-NMR (400.1 MHz, CDCl₃, 300 K): δ = 0.96 (s, 9H, C(CH₃)₃), 1.35 (d, *J* = 7 Hz, 6H, CH(CH₃)₂, 2.24 (br s, 1H, OH), 3.61 (t, *J* = 7.6 Hz, 1H, CHC(CH₃)₃), 3.85 (m, 2H, CH₂), 5.12 (m, 1H, OCH), 7.27 (br s, 1H, NH) ppm.
¹³C-NMR (100.6 MHz, CDCl₃, 300 K): δ = 21.6 (CH(CH₃)₂), 26.9 (C(CH₃)₃), 33.8 (C(CH₃)₃), 60.5 (CH₂), 62.4 (NCH), 71.8 (CH(CH₃)₂), 157.8 (N-C=O), 160.5 (O-C=O) ppm.

Example A3: Preparation of 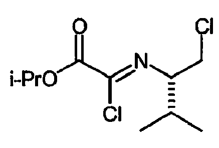 i-Pr = isopropyl (A3)

The amide A1 (0.50 g, 2.2 mmol) is dissolved in SOCl₂ (3.0 ml), dimethylformamide (DMF) (4 µl, 2.5 mol%) is added and the mixture is refluxed at 85°C for 16 hours. Removal of the SOCl₂ in a high vacuum gives a quantitative yield of the chlorimine A3 in the form of a colourless oil.
¹H-NMR (400.1 MHz, CDCl₃, 300 K): δ = 0.91 (d, *J* = 6.8 Hz, 3H, CH(CH₃)₂), 0.95 (d, *J* = 6.8 Hz, 3H, CH(CH₃)₂), 2.05 (sep, *J* = 6.8 Hz, 1H, CH(CH₃)₂), 3.67 (m, 2H, CH₂), 3.93 (m, 1H, CH(CH₂)), 5.16 (sep, *J* = 6.0, 1H, OCH(CH₃)₂) ppm.
¹³C-NMR (100.6 MHz, CDCl₃, 300 K): δ = 18.3 (CH(CH₃)₂), 19.3 (CH(CH₃)₂), 21.6 (OCH(CH₃)₂), 31.4 (CH(CH₃)₂), 45.3 (CH₂), 71.0 (CH(CH₂)), 72.0 (OCH(CH₃)₂), 136.5 (COCCl), 158.6 (CO) ppm.

Example A4: Preparation of

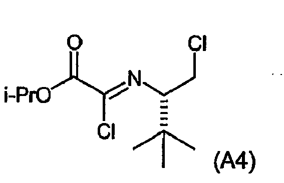

The amide A2 (5.00 g, 21.6 mmol) is dissolved in SOCl₂ (12.0 ml), DMF (40 µl, 2.5 mol%) is added and the mixture is refluxed at 85°C for 16 hours. After the SOCl₂ has been removed in a high vacuum, the crude product is purified by means of bulb tube distillation (oven temperature: 100°C / 0.15 mbar). The product is obtained in the form of a colourless oil (5.21 g/19.4 mmol/90%).
¹H-NMR (400.1 MHz, CDCl₃, 300 K): δ = 0.96 (s, 9H, C(CH₃)₃), 1.35 (d, *J* = 12 Hz, 6H, CH(CH₃)₂), 3.67 (t, *J* =10.6 Hz, 1H, CH₂), 3.87 (m, 2H, CH₂ and CH), 5.16 (sep, *J* = 6.4 Hz, 1H, OCH(CH₃)₂) ppm.
¹³C-NMR (100.6 MHz, CDCl₃, 300 K): *δ =* 21.6 (CH(CH₃)₂), 26.6 (C(CH₃)₃), 35.6 (C(CH₃)₃), 44.8 (CH₂), 71.8 (CH(CH₂)), 74.6 (OCH(CH₃)₂), 136.6 (COCCl), 158.7 (O-C=O) ppm.

Example A5 (comparison): Preparation of 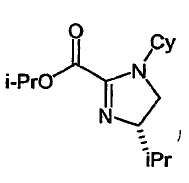 Cy = cyclohexyl (A5)

Chlorimine A3 (560 mg, 2.2 mmol) is dissolved in absolute toluene (5 ml) and admixed with triethylamine (2 ml). After the cyclohexylamine (290 µl, 2.5 mmol) dissolved in 3 ml of toluene has been added dropwise, the mixture is heated at 110°C for 12 hours. The solution is cooled to room temperature for the work-up. The mixture is washed twice with 3 ml each time of 1 N aqueous KOH solution, shaken and the aqueous phase is extracted twice with 10 ml each time of toluene. After drying over MgSO₄ and filtration, the solvent is removed on a rotary evaporator. This leaves a yellow oil which is purified by column chromatography (pentane/diethyl ether/triethylamine, 8:1:1). This gives 360 mg of the pure imidazoline A5 (1.28 mmol, 56%).
¹H-NMR (400.1 MHz, CDCl₃, 300 K): δ = 0.84 (d, *J* = 6.8 Hz, 3H, CH(CH₃)₂), 0.95 (d, *J* = 7.2 Hz, 3H, CH(CH₃)₂), 1.27-1.25 (m, 5H, Cy), 1.32 (d, *J* = 6.0 Hz, 6H, O-CH(CH₃)₂) 1.55-1.90 (m, 6H; 5H Cy, 1H CH(CH₃)₂)), 3.08 (t, *J* = 9.6 Hz, 1H, CH₂), 3.36 (t, *J* = 9.6 Hz, 1H, CH₂), 3.57 (t, *J =* 9.6 Hz, 1H, CH, Im), 3.81 (q, *J* = 4.8 Hz, 1H, CH, Cy), 5.18 (sep, *J =* 6.0 Hz, 1H, OCH(CH₃)₂) ppm.
¹³C-NMR (100.6 MHz, CDCl₃, 300K): δ = 17.9 (CH(CH₃)₂), 19.2 (CH(CH₃)₂, 21.7 (OCH(CH₃)₂), 25.5 (CH₂, Cy), 25.6 (CH₂, Cy), 25.8 (CH₂, Cy), 30.8 (CH₂, Cy), 31.6 (CH₂, Cy), 33.0 (CH(CH₃)₂), 46.7 (CH₂, lm), 54.8 (CH, Cy), 69.9 (CH, lm), 70.5 (OCH(CH₃)₂), 156.6 (C=N), 161.5 (C=O) ppm.

Example A6: Preparation of 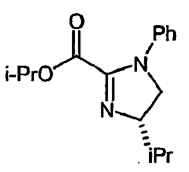 Ph = phenyl (A6)

The procedure of Example A5 is repeated using chlorimine A3 (406 mg, 1.6 mmol) and aniline (290 µl, 3.2 mmol), giving, after purification by column chromatography (pentane/diethyl ether/triethylamine 8:1:1), the imidazoline A6 in the form of a yellow oil (275 mg, 1.00 mmol, 63%).
¹H-NMR (400.1 MHz, CDCl₃, 300 K): δ = 0.93 (d, *J* = 6.8 Hz, 3H, CH(CH₃)₂), 1.03 (d, *J* = 6.8 Hz, 3H, CH(CH₃)₂), 1.15 (d, *J*= 6.4 Hz, 3H, OCH(CH₃)₂), 1.18 (d, *J*= 6.4 Hz, 3H, OCH(CH₃)₂), 1.91 (sep, *J* = 6.4 Hz, 1H, CH(CH₃)₂), 3.59 (t, *J* = 9.2 Hz, 1H, CH₂), 3.92 (t, *J* = 9.2 Hz, 1H, CH₂), 4.10 (m, 1H, CH, Im), 5.10 (sep, *J* = 6.4 Hz, 1H, OCH(CH₃)₂), 6.95 (d, *J* = 8.0 Hz, 2H, Ar-H), 7.09 (t, *J* = 7.6 Hz, 1H, Ar-H), 7.28 t, *J* = 7.6 Hz, 2H, Ar-H) ppm.
¹³C-NMR (100.6 MHz, CDCl₃; 300 K): δ = 18.1 (CH(CH₃)₂), 19.1 (CH(CH₃)₂), 21.4 (OCH(CH₃)₂), 32.9 (CH(CH₃)₂), 54.9 (CH₂), 70.4 (CH), 71.6 (OCH(CH₃)₂), 121.1 (CH, Ar), 124.5 (CH, Ar-C), 129.2 (CH, Ar-C), 141.5 (C-N, Ar-C), 154.3 (N-C=N), 161.0 (C=O) ppm.

Example A7 (comparison): Preparation of

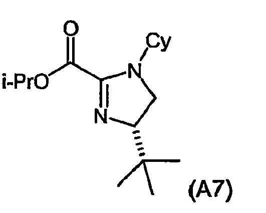

The procedure of Example A5 is repeated using chlorimine A4 (400 mg, 1.49 mmol) and cyclohexylamine (256 µl, 2.24 mmol), giving, after purification by column chromatography (pentane/diethyl ether (Et₂O)/triethylamine 7:2:1), the imidazoline A7 in the form of a colourless oil (294 mg, 1.00 mmol, 67%).
¹H-NMR (400.1 MHz, CDCl₃, 300 K): δ = 0.87 (s, 9H, C(CH₃)₃), 1.01 -1.85 (m, 10H, Cy-H), 1.32 (d, *J* = 6.3 Hz, 6H, OCH(CH₃)₂), 3.16 (m, 1H, lm-H), 3.31 (m, 1H, lm-H), 3.48 (m, 1H, Cy-H), 3.76 (m, 1H, 1m-H), 5.19 (sep, *J* = 6.3 Hz, 1H, OCH(CH₃)₂) ppm.
¹³C-NMR (100.6 MHz, CDCl₃, 300K): δ = 22.3 (OCH(CH₃)₂), 25.8 (CH₂, Cy), 25.9 (CH₂, Cy), 26.1 (CH₂, Cy), 26.3 (C(CH₃)), 30.8 (CH₂, Cy), 31.6 (CH₂, Cy), 34.7 (C(CH₃)₃), 45.6 (CH₂, lm), 55.1 (CH, Cy), 70.2 (CH, lm), 74.4 (OCH(CH₃)₂), 157.0 (C=N), 162.0 (C=O) ppm.

Example A8: Preparation of 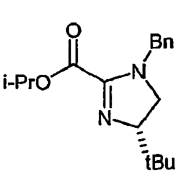 (Bn = benzyl; tBu = tert-butyl) (A8)

The procedure of Example A5 is repeated using chlorimine A4 cf. Example A13 (590 mg, 2.2 mmol) and benzylamine (280 µl, 2.6 mmol), giving, after purification by column chromatography (pentane/Et₂O/triethylamine 8:1:1), 280 mg of a yellow oil (0.93 mmol, 43%).
¹H-NMR (400.1 MHz, CDCl₃, 300 K): δ = 0.86 (s, 9H, C(CH₃)₃); 1.32 (dd, *J* = 6.4, 3.5 Hz, 6H, CH(CH₃)₂), 3.06 (t, *J* = 9.6 Hz, 1H, CH₂, Im), 3.26 (t, *J* = 9.6 Hz, 1H, CH, lm), 3.83 (t, *J* = 9.6 Hz, 1H, CH₂, lm), 4.43 (s br, 2H, CH₂Ph), 5.20 (sep, *J* = 6.4 Hz, 1H, CH(CH₃)₂), 7.25-7.45 (m, 5H, Ph-H) ppm.
¹³C-NMR (100.6 MHz, CDCl₃, 300 K): δ = 21.7 (CH(CH₃)₂), 26.0 (C(CH₃)₃), 34.2 (C(CH₃)₃), 51.0 (CH₂, Bn-H), 51.4 (CH₂), 70.4 (CH(CH₃)₂), 74.5 (CH), 127.6 (CH, Ar-C), 127.8 (CH, ArC), 128.7 (CH, Ar-C), 137.3 (C, ipso, Ar-C), 156.5 (N-C=N), 161.0 (C=O) ppm.

Example A9: Preparation of

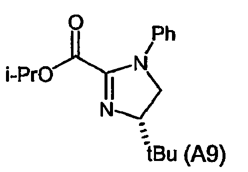

The procedure of Example A2 is repeated using chlorimine A4 (1.15 g, 4.3 mmol) and aniline (475 µl, 5.2 mmol), giving, after column chromatography (pentane/Et₂O/triethylamine 4:5:1), the imidazoline A9 as an orange oil (900 mg, 3.12 mmol, 72%).
¹H-NMR (400.1 MHz, CDCl₃, 300K): δ = 0.96 (s, 9H, C(CH₃)₃), 1.11 (d, *J* = 6.0 Hz, 3H, CH(CH₃)₂), 1.16 (d, *J* = 6 Hz, 3H, CH(CH₃)₂), 3.65 (t, *J* = 9.4 Hz, 1H, CH₂), 3.87 (t, *J* = 9.2 Hz, 1H, CHC(CH₃)₃), 4.00 (t, *J* = 9.2 Hz, 1H, CH₂), 5.09 (sep, *J* = 6.4 Hz, 1H, OCH(CH₃)₂), 6.94 (dd, J= 8.8, 1.0 Hz, 2H, Ar-H), 7.08 (t, *J* = 7.2 Hz, 1H, para-Ar-H), 7.27 (t, *J* = 7.6 Hz, 2H, Ar-H) ppm.
¹³C-NMR (100.6 MHz, CDCl₃, 300 K): *δ* = 21.3 and 21.4 (OCH(CH₃)₂), 26.0 (C(CH₃)), 34.2 (C(CH₃)₃), 53.5 (CH₂), 70.3 (OCH(CH₃)₂), 75.1 (CHC(CH₃)₃), 121.0 (CH, Ar), 124.4 (CH, para, Ar), 129.2 (CH, Ar), 141.5 (C, Ar), 154.3 (C=N), 161.0 (C=O) ppm.

Example A10: Preparation of 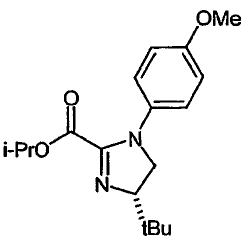 (Me = methyl) (A10)

The procedure of Example A5 is repeated using chlorimine A4 (1.15 g, 4.3 mmol) and p-anisidine (1.07 g, 8.6 mmol), giving, after purification by column chromatography (pentane/Et₂O/triethylamine 8:1:1), the imidazoline A10 as a yellow, viscous oil (420 mg, 1.32 mmol, 31%).
¹H-NMR (400.1 MHz, CDCl₃, 300 K): δ = 0.96 (s, 9H, C(CH₃)₃), 1.07 (d, *J* = 6.0 Hz, 3H, CH(CH₃)₂), 1.13 (d, *J* = 6.0 Hz, 3H, CH(CH₃)₂), 3.54 (t, *J* = 9.2 Hz, 1H, CH₂), 3.77 (s, 3H, OCH₃), 3.81 (t, *J* = 9.2 Hz, 1H, CH), 3.97 (t, *J* = 9.2 Hz, 1H, CH₂), 5.02 (sep, *J* = 6.4 Hz, 1H, OCH(CH₃)₂), 6.81 (d, *J* = 8.8 Hz, 2H, Ar-H), 6.94 (d, *J* = 9.0 Hz, 2H, Ar-H) ppm.
¹³C-NMR (100.6 MHz, CDCl₃, 300 K): δ = 21.4 and 21.5 (CH(CH₃)₂), 26.0 (C(CH₃)₃), 34.2 (C(CH₃)₃), 54.7 (CH₂), 55.6 (OCH₃), 70.0 (OCH(CH₃)₂), 75.3 (CH), 114.5 (CH, Ar-C), 124.1 (CH, Ar-C), 135.2 (N-C, Ar-C), 155.2 (C-O, Ar-C), 157.3 (N-C=N), 160.9 (C=O) ppm.

Example A11: Preparation of

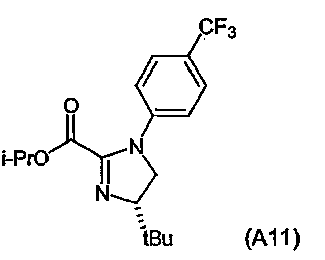

The procedure of Example A5 is repeated using chlorimine A4 (1.15 g, 4.3 mmol) and 4-aminobenzotrifluoride (1.07 ml, 8.6 mmol), giving, after purification by column chromatography (pentane/triethylamine 9:1), the imidazoline A11 as a red oil (1.10 g, 3.07 mmol, 71 %).
¹H-NMR (400.1 MHz, CDCl₃, 300 K): δ = 0.96 (s, 9H, C(CH₃)₃), 1.23 (d, *J* = 6.4 Hz, 3H, CH(CH₃)₂), 1.25 (d, *J* = 6.4 Hz, 3H, CH(CH₃)₂), 3.72 (t, *J* = 8.8 Hz, 1H, CH₂), 3.90 (t, *J* = 9.2 Hz, 1H, CHC(CH₃)₃), 4.03 (t, *J* = 8.8 Hz, 1H, CH₂), 5.17 (sep, *J* = 6.4 Hz, 1H, OCH(CH₃)₂), 6.96 (d, *J* = 8.8 Hz, 2H, Ar-H), 7.52 (d, *J* = 8.8 Hz, 2H, Ar-H) ppm.
¹³C-NMR (100.6 MHz, CDCl₃, 300 K): δ = 21.4 (CH(CH₃)₂), 25.9 (C(CH₃)₃), 34.2 (C(CH₃)₃), 52.6 (CH₂), 65.9 (OCH), 75.1 (CH, lm), 119.2 (CH, Ar), 126.4 (CH, Ar), 143.9 (C-N, Ar), 152.8 (N-C=N), 160.8 (C=O) ppm.
¹⁹F-NMR (376.4 MHz, CDCl3, 300 K): δ = - 63.2 ppm.

Example A12: Preparation of 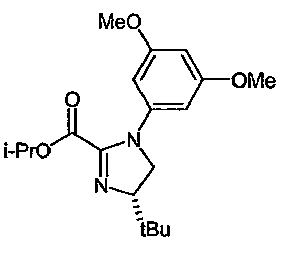 (MeO = methoxy) (A12)

The procedure of Example A5 is repeated using chlorimine A4 (400 mg, 1.49 mmol) and 3,5-dimethoxyaniline (342 mg, 2.24 mmol), giving, after purification by column chromatography (pentane/triethylamine 9:1), the imidazoline A12 as a colourless oil (117 mg, 0.336 mmol, 23%).
¹H-NMR (400.1 MHz, CDCl₃, 300 K): δ **=** 0.96 (s, 9H, C(CH₃)₃), 1.19 (d, *J* = 6.6 Hz, 3H, CH(CH₃)₂), 1.22 (d, *J* = 7.0 Hz, 3H, CH(CH₃)₂), 3.64 (t, *J*= 8.8 Hz, 1H, CH₂), 3.85 (dd, *J* = 10.9 Hz, 9.2 Hz, 1H, CHC(CH₃)₃), 3.74 (s, 6H, MeO), 3.99 (dd, *J* = 11.1Hz, 8.8 Hz, 1H, CH₂), 5.13 (sep, *J* = 6.3 Hz), 6.11 (d, *J* = 2.3 Hz, 2H, Ar-H), 6.20 (t, *J* = 2.0 Hz, 1H, Ar-H) ppm.
¹³C-NMR (100.6 MHz, CDCl₃, 300 K): δ **=** 21.8 (CH(CH₃)₂), 26.9 (C(CH₃)₃), 34.5 (C(CH₃)₃), 53.6 (CH₂), 55.7 (CH₃O), 70.8 (OCH), 75.3 (CH, Im), 96.5 (CH, Ar), 99.7 (CH, Ar), 143.9 (C-N, Ar), 154.5 (N-C=N), 161.6 (C=O and C-O, Ar) ppm.
¹³C-NMR (100.6 MHz, CDCl₃, 300 K): δ = 18.1 (Ar-CH₃), 21.4 and 21.6 (je CH(CH₃)₂), 26.5 (C(CH₃)₃), 34.6 (C(CH₃)₃), 54.8 (CH₂), 70.1 (OCH), 76.0 (CH, Im), 126.4 (C, Ar), 127.3 (CH, Ar), 127.8 (C, Ar), 131.4 (CH, Ar), 136.4 (C), 157.1 (C) ppm.

Example A13: Preparation of

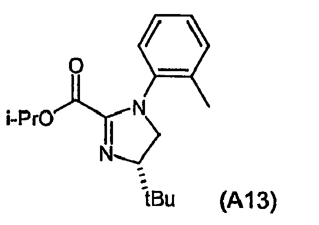

The procedure of Example A5 is repeated using chlorimine A4 (0.50 g, 1.86 mmol) and *o*-toluidine (0.30 ml, 2.80 mmol), giving, after purification by column chromatography (pentane/triethylamine 9:1), the imidazoline A1 as a colourless oil (160 mg, 0.652 mmol, 35%).
¹H-NMR (400.1 MHz, CDCl₃, 300 K): δ= 0.92 (d, *J*= 6.0 Hz, 3H, CH(CH₃)₂), 1.00 (br, 12H, C(CH₃)₃ and CH(CH₃)₂), 2.32 (s, 3H, Ar-CH₃), 3.45 (br, 1H, CH₂), 3.72 (br, 1H, CHC(CH₃)₃), 4.06 (dd, *J*= 11.4 Hz, 9.6 Hz, 1H, CH₂), 4.91 (sep, *J* = 6.3 Hz), 6.96 (m br, 1H, Ar-H), 7.11-7.24 (m, 3H, Ar-H) ppm.
¹³C-NMR (100.6 MHz, CDCl₃, 300 K): δ = 18.1 (Ar-CH₃), 21.4 and 21.6 (each CH(CH₃)₂), 26.5 (C(CH₃)₃), 34.6 (C(CH₃)₃), 54.8 (CH₂), 70.1 (OCH), 76.0 (CH, lm), 126.4 (C, Ar), 127.3 (CH, Ar), 127.8 (C, Ar), 131.4 (CH, Ar), 136.4 (C), 157.1 (C) ppm.

Example A14: Preparation of

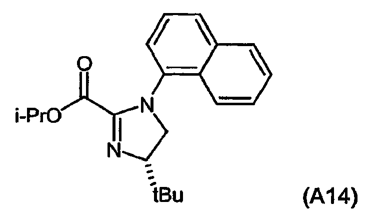

The procedure of Example A5 is repeated using chlorimine A4 (0.50 g, 1.86 mmol) and 1-naphthylamine (0.347 g, 2.42 mmol) with addition of tetrabutylammonium iodide (0.343 g, 0.93 mmol), giving, after purification by column chromatography (pentane/diethyl ether/triethylamine 8:1:1), the imidazoline A14 as a colourless solid (0.445 g, 1,31 mmol, 71%).
¹H-NMR (250.1 MHz, CDCl₃, 300K): δ = 0.55 - 0.92 (m br, 6H, CH(CH₃)₂), 1.08 (s, 9H, C(CH₃)₃), 3.35 - 4.27 (br, 3H, CH₂ and CHC(CH₃)₃), 4.82 (sep, *J* = 6.4 Hz, 1H, OCH(CH₃)₂), 7.21 (br, 1H, Ar-H), 7.43 (pt, 1H, Ar-H), 7.56 (m, 2H, Ar-H), 7.78 (d, J= 13.1Hz, 1H, Ar-H), 7.90 (m, 1H, Ar-H), 8.09 (br, 1H, Ar-H) ppm.
¹³C-NMR (100.6 MHz, CDCl₃, 300 K): δ=21.3 and 22.0 (OCH(CH₃)₂), 26.6 (C(CH₃)), 35.6 (C(CH₃)₃), 56.5 (CH₂), 69.9 (OCH(CH₃)₂), 76.4 (CHC(CH₃)₃), 123.7, 126.0, 126.8, 127.0, 127.8, 128.6, 131.4, 134.8 (each Ar-C), 157.4 (C=N), 160.4 (C=O) ppm.

Example A15: Preparation of

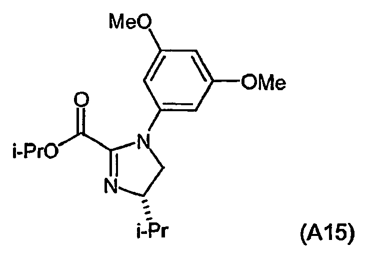

The procedure of Example A5 is repeated using chlorimine A3 (584 mg, 2.30 mmol) and 3,5-dimethoxyaniline (458 mg, 2.99 mmol), giving, after purification by column chromatography (pentane/triethylamine 9:1), the imidazoline A15 as a colourless oil (339 mg, 1.01 mmol, 44%).
¹H-NMR (400.1 MHz, CDCl₃, 300 K): δ = 0.91 (d, *J* = 6.8 Hz, 3H, CHCH(CH₃)₂), 1.02 (d, *J* = 6.8 Hz, 3H, CHCH(CH₃)₂), 1.20 (d, *J* = 6.3 Hz, 3H, OCH(CH₃)₂), 1.22 (d, *J* = 6.3 Hz, 3H, OCH(CH₃)₂), 1.89 (sept, *J* = 6.8 Hz, 1H, CHCH(CH₃)₂) 3.56 (pt, 1H, CH₂), 3.73 (s, 6H, OCH₃), 3.88 (dd, *J*=10.6 Hz, 9.1 Hz, 1H, CHCH(CH₃)₂), 4.00 (m, 1H, CH₂), 5.12 (sept, *J* = 6.3 Hz), 6.10 (d, *J* = 2.0 Hz, 2H, Ar-H), 6.19 (t, *J* = 2.3 Hz, 1H, Ar-H) ppm.
¹³C-NMR (100.6 MHz, CDCl₃, 300 K): δ = 18.4 (CH(CH₃)₂), 19.5 (CH(CH₃)₂), 21.8 (2C, OCH(CH₃)₂), 33.2 (CH(CH₃)₂), 55.8 (CH₂, lm), 55.7 (2C, CH₃O), 70.8 (OCH), 75.3 (CH, lm), 96.6 (CH, Ar), 99.7 (CH, Ar), 143.4 (C-N, Ar), 154.5 (N-C=N), 161.5, 161.6 (C=O and C-O, Ar) ppm.

### B) Preparation of imidazolinemethanols

Example B1: Preparation of

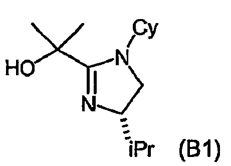

The imidazoline A5 (200 mg, 0.71 mmol) is placed in a baked Schlenk flask and dissolved in absolute diethyl ether (8 ml). A solution of methylmagnesium bromide (3 M, in Et₂O, 0.72 ml, 2.14 mmol) is then slowly added dropwise at -78°C while stirring vigorously. The reaction solution is allowed to warm slowly to room temperature and is stirred for another 14 h. For the work-up, cold aqueous NH₄Cl solution (8 ml) is added. After phase separation, the aqueous phase is extracted twice with Et₂O (10 ml). The combined organic extracts are dried over MgSO₄. The crude product is used without further purification for preparing the phosphinite.
¹H-NMR (250 MHz, CDCl₃, 300 K, crude product spectrum): δ *=* 0.85 (d, *J* = 6.5 Hz, 3H, CH(CH₃)₂), 0.95 (d, *J* = 6.5 Hz, 3H, CH(CH₃)₂), 1.00 -1.50 (m, 5H, CH₂, Cy), 1.50 (s, 6H, HOC(CH₃)₂), 1.50 - 2.0 (m, 6H, CH₂ (Cy), CH(CH₃)₂), 3.30 (t, J = 9.5 Hz, 1H, Im), 3.40 (m, 2H, Im), 3.80 (m, 1H, CH, Cy) ppm.

Example B2: Preparation of

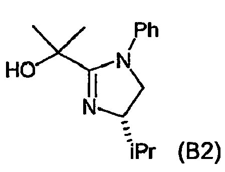

Using the procedure of Example B1, imidazoline A6 (200 mg, 0.73 mmol) is reacted with methylmagnesium bromide solution (0.73 ml, 2.19 mmol) to form the alcohol B2. A very pure crude product is obtained (165 mg, 0.67 mmol, 92%).
¹H-NMR (400.1 MHz, CDCl₃, 300 K): δ = 0.93 (d, *J* = 6.8 Hz, 3H, CH(CH₃)₂), 1.00 (d, *J* = 6.8 Hz, 3H, CH(CH₃)₂), 1.15 (s, 3H, CH₃), 1.20 (s, 3H, CH₃), 1.81 (sep, *J* = 6.8 Hz, 1H, CH(CH₃)₂), 3.59 (t, *J* = 7.2 Hz, 1H, CH₂), 3.84 (m, 2H, CH₂ and CH, Im), 4.77 (s br, 1H, OH), 7.23 (d, *J* = 4.0 Hz, 2H, Ar-H), 7.30 (t, *J* = 6.8 Hz, 1H, Ar-H), 7.37 (t, *J* = 7.2 Hz, 2H, Ar-H) ppm.
¹³C-NMR (100.1 MHz, CDCl₃, 300 K): δ = 18.1 (CH(CH₃)₂), 18.7 (CH(CH₃)₂), 28.8 (HO-C(CH₃)₂), 29.3 (HO-C(CH₃)₂), 61.4 (CH₂), 68.0 (CH, Im), 69.5 (HO-C(CH₃)₂), 127.8 (CH, ArC), 129,1 and 129.5 (CH, Ar-C), 142.8 (C, ipso, Ar), 170.5 (C=N) ppm.

Example B3: Preparation of

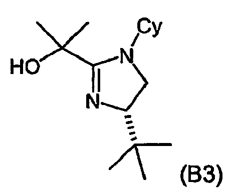

Using the procedure of Example B1, imidazoline A7 (260 mg, 0.88 mmol) is reacted with methylmagnesium bromide solution (0.88 ml, 2.65 mmol) to form the alcohol B3. A very pure crude product is obtained (180 mg, 0.67 mmol, 77%).
¹H-NMR (400.1 MHz, CDCl₃, 300 K): δ = 0.84 (s, 9H, C(CH₃)₃), 1.09 (m, 1H, Cy), 1.26 (m, 2H, Cy), 1.42 (s, 3H, CH₃), 1.44 (s, 3H, CH₃), 1.46 -1.84 (br m, 7H, Cy), 3.31 - 3.42 (m, 2H, Cy and Im), 3.47 (m, 1H, CH₂), 3.58 (m, 1H, Im) ppm.
¹³C-NMR (100.1 MHz, CDCl₃, 300 K): *δ* = 25.7 (Cy), 25.9 (C(CH₃)₃), 26.2 (Cy), 26.3 (Cy), 28.2 (HO-C(CH₃)₂), 29.0 (HO-C(CH₃)₂), 34.9 (C(CH₃)₃), 47.7 (C(CH₃)₂), 68.0 (Im), 70.2 (Im), 170.5 (C=N) ppm.

Example B4: Preparation of

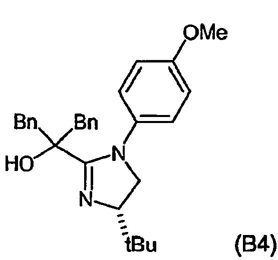

Imidazoline A10 (250 mg, 0.83 mmol) is reacted as described in Example B1 with benzylmagnesium bromide (1 M, in diethyl ether, 2.5 ml, 2.5 mmol). Purification is carried out by means of column chromatography (pentane/diethyl ether/triethylamine 8:1:1), giving 250 mg (0.566 mmol, 68%) of a yellow oil.
¹H-NMR (400.1 MHz, CDCl₃, 300 K): δ = 0.86 (s br, 9H, C(CH₃)₃), 2.16 (s br, 1H, OH), 2.77 (m, 2H, CH₂, Bn-H), 3.00 (m, 1H, CH₂, CH₂Ph), 3.45 (t, *J* = 9.0 Hz, 1H, lm), 3.67 (m, 2H, lm), 3.81 (s, 3H, OCH₃), 4.40 (s br, 1H, CH₂, Bn-H), 6.90 (m, 2H, Ar-H), 7.10-7.40 (m, 12H, Ar-H) ppm.

Example B5: Preparation of

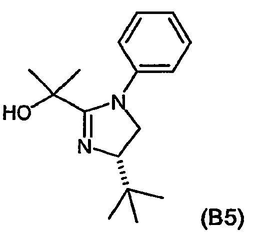

Using the procedure of Example B1, imidazoline A9 (200 mg, 0.73 mmol) is reacted with methylmagnesium bromide solution (0.73 ml, 2.19 mmol) to form alcohol B4. A very pure crude product is obtained (165 mg, 0.67 mmol, 92%).
¹H-NMR (400.1 MHz, CDCl₃, 300 K): δ = 0.93 (d, *J* = 6.8 Hz, 3H, CH(CH₃)₂), 1.00 (d, *J* = 6.8 Hz, 3H, CH(CH₃)₂), 1.15 (s, 3H, CH₃), 1.20 (s, 3H, CH₃), 1.81 (sep, *J* = 6.8 Hz, 1H, CH(CH₃)₂), 3.59 (t, *J* = 7.2 Hz, 1H, CH₂), 3.84 (m, 2H, CH₂ and CH, Im), 4.77 (s br, 1H, OH), 7.23 (d, *J* = 4.0 Hz, 2H, Ar-H), 7.30 (t, *J* = 6.8 Hz, 1H, Ar-H), 7.37 (t, *J* = 7.2 Hz, 2H, Ar-H) ppm.
¹³C-NMR (100.1MHz, CDCl₃, 300 K): *δ =* 18.1 (CH(CH₃)₂), 18.7 (CH(CH₃)₂), 28.8 (HO-C(CH₃)₂), 29.3 (HO-C(CH₃)₂), 61.4 (CH₂), 68.0 (CH, Im), 69.5 (HO-C(CH₃)₂), 127.8 (CH, ArC), 129.1 and 129.5 (CH, Ar-C), 142.8 (C, ipso, Ar), 170.5 (C=N) ppm.

Example B6: Preparation of

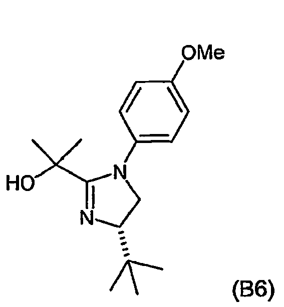

Using the procedure of Example B1, imidazoline A10 (400 mg, 1.26 mmol) is reacted with methylmagnesium bromide solution (1.26 ml, 3.78 mmol, in Et₂O). This gives a yellow oil (130 mg, 36%).
¹H-NMR (400.1 MHz, CDCl₃, 300 K): δ = 0.95 (s, 9H, C(CH₃)₃), 1.16 (s, 3H, CH₃), 1.20 (s, 3H, CH₃), 3.64 (t, *J =* 8.8 Hz, 1H, CH₂), 3.78 (m, 2H, CH and CH₂, imidazoline), 3.81 (s, 3H, OCH₃), 5.23 (br s, 1H, OH), 6.87 (d, *J =* 8.8 Hz, 2H, Ar-H), 7.13 (d, *J =* 8.8 Hz, 2H, Ar-H) ppm.
¹³C-NMR (100.6 MHz, CDCl₃, 300 K): δ = 25.7 (C(CH₃)₃), 28.5 (CH₃), 29.3 (CH₃), 34.4 (C(CH₃)₃), 55.6 (OCH₃), 60.1 (CH₂), 69.4 (C-OH), 114.7 (CH, Ar-C), 130.3 (CH, Ar-C), 159.1 (N-C=N) ppm.

Example B7: Preparation of

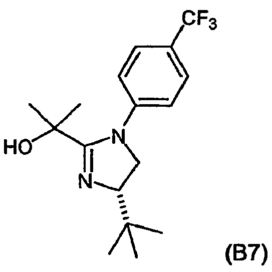

Using the procedure of Example B1, the imidazoline B7 (200 mg, 0.56 mmol) is reacted with methylmagnesium bromide solution (3 M in Et₂O, 0.56 ml, 1.68 mmol). The alcohol obtained (165 mg, 90%) is used further as crude product.
¹H-NMR (400.1 MHz, CDCl₃, 300 K): δ = 0.95 (s, 9H, C(CH₃)₃), 1.19 (s, 3H, CH₃), 1.23 (s, 3H, CH₃), 3.67 (t, J= 8.4 Hz, 1H, CH₂), 3.83 (m, 2H, CH₂ and CH, lm), 4.73 (s br, 1H, OH), 7.34 (d, *J* = 8.8 Hz, 2H, Ar-H), 7.63 (d, *J* = 8.0 Hz, 2H, Ar-H) ppm.
¹³C-NMR (100.6 MHz, CDCl₃, 300 K): δ=25.8 (C(CH₃)₃), 28.9 (CH₃), 29.6 (CH₃), 34.3 (C(CH₃)₃), 59.7 (CH₂), 69.6 (C-OH), 71.7 (CH, lm), 122.5 (CCF₃), 125.2 (CCF₃), 126.7 (CH, Ar-C), 129.0 (CH, Ar-C), 146.3 (C-N, Ar), 169.6 (C=N) ppm.
¹⁹F-NMR (376.4 MHz, CDCl3, 300 K): δ = - 63.6 ppm.

Example B8: Preparation of

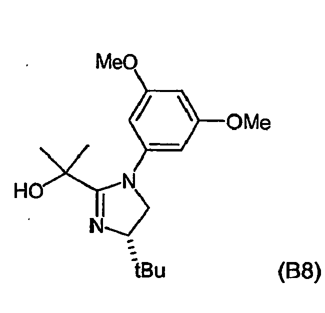

Using the procedure of Example B1, the imidazoline A12 (98 mg, 0.28 mmol) is reacted with methylmagnesium bromide solution (3 M in Et₂O, 0.34 ml, 1.03 mmol). The alcohol obtained (83 mg, 92%) is used further as crude product.
¹H-NMR (500.1 MHz, CDCl₃, 295 K): δ = 0.94 (s, 9H, C(CH₃)₃), 1.23 (s, 3H, CH₃), 1.27 (s, 3H, CH₃), 3.64 (dd, J= 9.0 Hz, 8.0 Hz, 1H, CH₂), 3.77 (m, 7H, CHC(CH₃)₃and CH₃O), 3.85 (dd, *J*=10.8 Hz, 9.0 Hz, 1H, CH₂), 5.10 (br, 1H, OH), 6.36 - 6.41 (m, 3H, Ar-H) ppm.
¹³C-NMR (125.8 MHz, CDCl₃, 295 K): δ = 25.7 (C(CH₃)₃), 28.7 and 29.5 (each OCH₃), 34.2 (C(CH₃)₃), 55.5 (CH₂ and OCH₃), 59.6 (CCH₃)₂), 69.5 (OCH), 71.1 (CH, lm), 99.4 (CH, Ar), 107.1 (CH, Ar), 144.4 (C-N, Ar), 161.2 (N-C=N), 170.2 (C-OMe, Ar) ppm.

Example B9: Preparation of

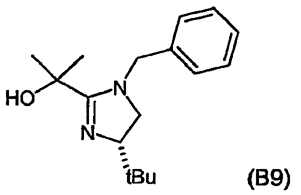

Using the procedure of Example B1, the imidazoline A8 (111 mg, 0.367 mmol) is reacted with methylmagnesium bromide solution (3 M in Et₂O, 0.37 ml, 1.10 mmol). The alcohol obtained (80 mg, 0.292 mmol, 80%) is used further as crude product.
¹H-NMR (400.1 MHz, CDCl₃, 300 K): δ = 0.85 (s, 9H, C(CH₃)₃), 1.53 (s, 6H, CH₃), 3.09 (m, 1H, CH₂), 3.29 (m, 1H, CH₂), 3.64 (dd, *J* = 11.1 Hz, 8.3 Hz, 1H, CH₂), 4.37 (d, *J* = 15.2 Hz, CH₂Ph), 4.48 (d, *J*= 15.4 Hz, CH₂Ph), 7.24 - 7.38 (m, 5H, Ar-H) ppm.
¹³C-NMR (125.8 MHz, CDCl₃, 295 K): δ = 26.1 (C(CH₃)₃), 28.5 and 29.2 (each CH₃), 34.2 (C(CH₃)₃), 52.4 (CH₂Ph), 54.0 (C(CH₃)₂), 69.0 (OCH), 71.4 (CH, lm), 127.4, 128.0, 129.2, 137.7 (each Ar-C), 170.9 (N-C=N) ppm.

Example B10: Preparation of

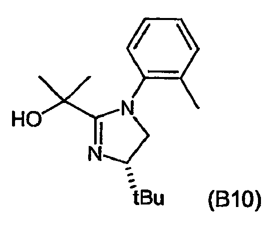

Using the procedure of Example B1, the imidazoline A13 (149 mg, 0.493 mmol) is reacted with methylmagnesium bromide solution (3 M in Et₂O, 0.49 ml, 1.48 mmol). The alcohol obtained (94 mg, 0.343 mmol, 70%) is used further as crude product. Owing to the formation of two diastereomers, some doubled sets of signals are observed in the NMR spectra. The mixture could not be separated.
¹H-NMR (400.1 MHz, CDCl₃, 300 K): δ = 0.94 (s, 9H, C(CH₃)₃), 1.08 (s, 3H, CH₃), 1.23 (s, 3H, CH₃), 2.28 (s, 3H, PhCH₃), 3.38, 3.53, 3.75 - 3.90 (each m, total of 3H, CH and CH₂), 5.12 (br s, 1H, OH), 7.08 - 7.29 (m, 4H, Ar-H) ppm.
¹³C-NMR (125.8 MHz, CDCl₃, 295 K): δ *=* 18.1 and 18.5 (PhCH₃), 26.1 and 26.2 (C(CH₃)₃), 27.1, 28.0, 29.0, 30.6 (CH₃), 34.6 and 34.7 (C(CH₃)₃), 58.1 and 58.3 (C(CH₃)₂), 69.6 and 69.7 (OCH), 72.0 and 72.1 (CH, lm), 127.2, 128.6, 130.2,130.5, 131.6, 137.6, 138.0, 140.9, 141.1 (each Ar-C), 170.6 and 171.0 (N-C=N) ppm.

Example B11: Preparation of

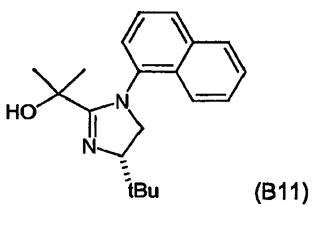

Using the procedure of Example B1, the imidazoline A14 (80 mg, 0.236 mmol) is reacted with methylmagnesium bromide solution (3 M in Et₂O, 0.29 ml, 0.863 mmol). The alcohol obtained (73 mg, 0.235 mmol, 99%) is used further as crude product. Diastereomer formation leads to a doubling of the signals in the NMR spectrum.
¹H-NMR (400.1 MHz, CDCl₃, 300 K): δ = 0.96 and 0.97 (s, 9H, C(CH₃)₃), 1.20 and 1.22 (s, 3H, CH₃), 1.27 and 1.29 (s, 3H, CH₃), 3.61, 3.74, 4.00, 4.12 (each m, total of 3H, CH and CH₂), 5.20 (brs, 1H, OH), 7.34, 7.43 - 7.59, 7.84 - 7.93, 8.00 (m, total of 7H, Ar-H) ppm. ¹³C-NMR (100.6 MHz, CDCl₃, 300 K): δ = 25.7 and 25.9 (C(CH₃)₃), 26.7, 27.7, 28.5, 30.1 (CH₃), 34.3, 34.4 (s, C(CH₃)₃), 59.3 (CH₂), 65.9 and 69.5 (C-OH), 71.4 and 71.6 (CH), 123.2, 125.5, 125.6, 126.6, 126 .7, 127.0, 127.1, 127.2, 127.6,128.6, 128.7, 128.8, 131.7, 134.6 (Ar-C) ppm (C-N, Ar and C=N not detected).

Example B12: Preparation of

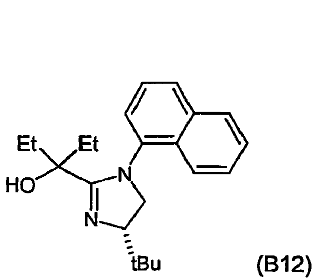

Using the procedure of Example B1, the imidazoline A14 (140 mg, 0.40 mmol) is reacted with ethylmagnesium chloride solution (3 M in Et₂O, 0.40 ml, 1.20 mmol). After work-up, the crude product is purified by means of column chromatography (pentane/ethyl ether/ triethylamine 8:1:1). The desired product is obtained in the form of a colourless oil (60 mg, 0.177 mmol, 44%). Diastereomer formation leads to a doubling of the signals in the NMR spectrum.
¹H-NMR (400.1 MHz, CDCl₃, 300 K): δ = 0.83 -1.39 (m, 19H, Et and C(CH₃)₃), 3.56, 3.72, 3.98 - 4.10 (each m, total of 3H, CH and CH₂), 5.04 (br s, 1H, OH), 7.37, 7.41 - 7.56, 7.83 - 7.95, 8.06 (m, total of 7H, Ar-H) ppm.
¹³C-NMR (100.6 MHz, CDCl₃, 300 K): δ = 8.7 and 8.8 (each CH₃), 26.5 and 26.6 (C(CH₃)₃), 31.9, 32.1, 32.7, 32.7 (CH₂), 34.2, 34.6 (s, C(CH₃)₃), 59.7 and 60.3 (C-OH), 72.5 (br, CH₂), 75.4 and 75.5 (CH), 123.4, 123.5, 125.9, 126.0, 126.3, 126.9, 127.0, 127.1, 127.2, 128.9, 128.9, 129.0, 132.0, 132.3, 134.9, 135.0(Ar-C) 168.1 (C=N) ppm.

Example B13: Preparation of

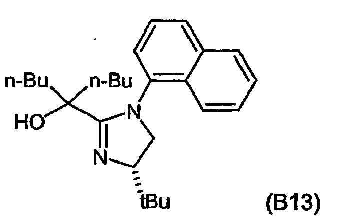

Using the procedure of Example B1, the imidazoline A14 (150 mg, 0.44 mmol) is reacted with n-butylmagnesium chloride solution (1 M in Et₂O, 1.53 ml, 1.33 mmol). After work-up, the crude product is purified by means of column chromatography (pentane/ethyl ether/ triethylamine 8:1:1). The desired product is obtained in the form of a colourless oil (15 mg, 0.038 mmol, 9%). Diastereomer formation leads to a doubling of the signals in the NMR spectrum.
¹H-NMR (500.1 MHz, CDCl₃, 295 K): δ = 0.68 -1.64 (m, 27H, n-Bu and C(CH₃)₃), 3.57, 3.74, 3.96 - 4.17 (each br m, total of 3H, CH and CH₂), 5.07 (br s, 1H, OH), 7.34, 7.39, 7.44, 7.82 - 7.95, 8.07 (m, total of 7H, Ar-H) ppm.
¹³C-NMR (125.8 MHz, CDCl₃, 295 K): δ=14.0 and 14.2 (each CH₃), 22.5, 22.8, 25.6, 25.8, 25.9, 26.1, 26.5, 26.9 (CH₃ and CH₂), 33.9 and 34.2 (s, C(CH₃)₃), 59.3 and 59.8 (C-OH), 72.1 (br, CH₂), 74.5 and 76.7 (CH), 122.9, 125.4, 125.5, 126.0, 126.6, 126.9, 128.5, 128.6, 134.6, 138.7 (Ar-C) 165.7 (C=N) ppm.

Example B14: Preparation of

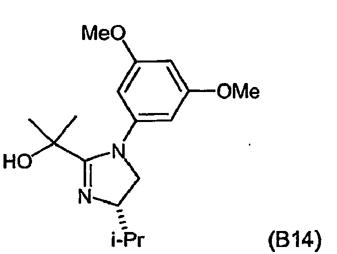

Using the procedure of Example B1, imidazoline A15 (147 mg, 0.44 mmol) is reacted with methylmagnesium bromide solution (0.44 ml, 1.32 mmol, 3 M in Et₂O). This gives a yellow oil (120 mg, 89%).
¹H-NMR (500.1 MHz, CDCl₃, 295 K): δ = 0.96 (d, *J* = 6.8 Hz, 3H, CHCH(CH₃)₂), 1.23 (d, *J* = 6.8 Hz, 3H, CHCH(CH₃)₂), 1.28 (s, 3H, CH₃), 1.31 (s, 3H, CH₃), 1.88 (sept, *J* = 6.5 Hz, 1H, CHCH(CH₃)₂), 3.63 (m, 1H, CH₂), 3.78 (s, 6H, OCH₃), 3.91 (m, 1H, CH, Im), 3.96 (m, 1H, CH₂, Im) 5.21 (br s, 1H, OH), 6.38 (d, *J* = 2.2 Hz, 2H, Ar-H), 6.43 (t, *J* = 2,1 Hz, 1H, Ar-H) ppm.
¹³C-NMR (100.6 MHz, CDCl₃, 300 K): *δ* = 17.7, 18.4 (each CH(CH₃)₂), 28.7 (CH₃), 29.1 (CH₃), 33.0 (CH(CH₃)₂), 55.6 (OCH₃), 60.8 (CH₂), 66.3 (CH, Im), 69.9 (C-OH), 99.9 (CH, ArC), 106.7 (CH, Ar-C), 114.7 (CH, Ar-C), 161.3 (C-N, Ar-C), 171.4 (N-C=N) ppm.

### C) Preparation of phosphinites

Example C1: Preparation of

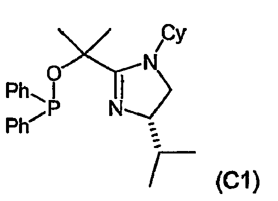

The alcohol B1 (60 mg, 0.24 mmol) is suspended in 15 ml of pentane. At -78°C, *n*-butylLi (1.6 M in hexane, 0.20 ml, 0.31 mmol) and subsequently tetramethylethylenediamine (TMEDA) (62 µl) are added dropwise. After removing the cooling bath, this solution is stirred at room temperature for 1 h. Diphenylchlorophosphane (Ph₂PCl) (57 µl, 0.31 mmol) is subsequently added at 0°C. The solution is stirred overnight.

For the work-up, the suspension is firstly evaporated to about 1 ml. This residue is subsequently applied directly to the prepared silica gel column. Purification of the crude product is carried out by means of column chromatography (pentane/triethylamine 9:1). The phosphinite C1 is isolated as a colourless oil (32 mg, 30%).
¹H-NMR (400.1 MHz, CDCl₃, 300 K): δ = 0.83 (d, *J* = 6.4 Hz, 3H, CH(CH₃)₂), 0.88 (d, 6.8 Hz, 3H, CH(CH₃)₂), 1.20 -1.37 (m, 11H, CH₂ Cy and CH(CH₃)₂), 1.67 (s, 6H, OC(CH₃)₂), 3.12 (s br, 1H, Cy), 3.34 (s br, 1H, lm), 3.76 (s br, 2H, lm), 7.30 (m, 6H, Ar-H), 7.51 (m, 4H, Ar-H) ppm.
³¹P-NMR (161.9 MHz, CDCl₃, 300 K): δ= 86.9 (96%) and - 23.9 (phosphinate, 4%) ppm.

Example C2: Preparation of

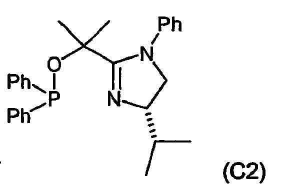

Using the procedure of Example C1, the alcohol B2 (135 mg, 0.49 mmol) is reacted with Ph₂PCl (120 µl, 0.64 mmol). After purification by column chromatography (pentane/Et₂O/triethylamine 8:1:1), a light-yellow liquid (80 mg, 0.19 mmol, 38%) is obtained. ¹H-NMR (400.1 MHz, CDCl₃, 300 K):δ = 0.91 (d, *J* = 6.4 Hz, 3H, CH(CH₃)₂), 0.95 (d, *J* = 6.8 Hz, 3H, CH(CH₃)₂), 1.87 (sep, *J* = 6.8 Hz, 1H, CH(CH₃)₂), 3.45 (q, J= 7.2 Hz, 1H, CH₂), 3.77 (t, *J* = 9.6 Hz, 1H, CH), 3.88 (m, 1H, CH₂), 6.95 - 7.10 (m, 5H, Ar-H), 7.26 (m br, 10H, Ar-H) ppm.
³¹P-NMR (161.9 MHz, CDCl₃, 300 K): δ = 87.9 ppm.

Example C3: Preparation of

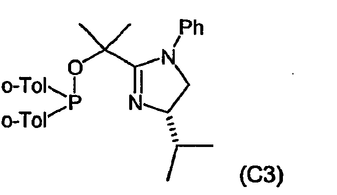

Using the procedure of Example C1, alcohol B2 (120 mg, 0.487 mmol) was reacted with bis(*ortho*-tolyl)chlorophosphane (172 mg, 0.633 mmol). Column chromatography gives a colourless oil (71 mg, 0.155 mmol, 32%).
¹H-NMR (400.1 MHz, CDCl₃, 300 K): δ = 0.97 (d, *J =* 7.0 Hz, 3H, CH(CH₃)₂), 1.01 (d, 7.0 Hz, 3H, CH(CH₃)₂), 1.89 (sep, *J* = 7.0Hz, 1H, CH(CH₃)₂), 1.60 (s, 6H, OC(CH₃)₂), 2.29 (s, 3H, CH₃, Tol), 2.34 (s, 3H, CH₃, Tol), 3.50 (dd, *J*=9.0 Hz, 7.4 Hz, CH₂, lm-H), 3.82 (dd, *J*=10.5 Hz, 9.0 Hz, CH₂, Im-H), 3.94 (m, 1H, CH, lm-H), 6.85 - 7.34 (m, 13H, Ar-H) ppm.
³¹P-NMR (161.9 MHz, CDCl₃, 300 K): δ = 72.8 and -35.3 (phosphinate, 5%) ppm.

Example C4: Preparation of

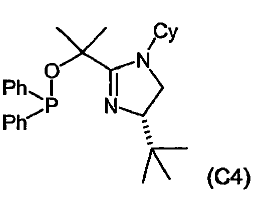

Using the procedure of Example C1, the alcohol B3 (70 mg, 0.26 mmol) is reacted with diphenylchlorophosphane (63 µl, 0.34 mmol). Column chromatography gives a colourless oil (38 mg, 0.087 mmol, 33%).
¹H-NMR (250.1 MHz, CDCl₃, 300 K): δ=0.67 (m, 2H, Cy), 0.90 (s br, 9H, C(CH₃)₃), 1.27-1.77 (m br, 14H, CH₂, Cy-H and C(CH₃)₂), 1.65 (s br, 6H, OC(CH₃)₂), 3.15 -3.47 (m br, 2H, Cy-H and lm-H), 3.55 - 3.75 (s br, 2H, lm-H), 7.31 - 7.58 (m, 10H, Ar-H) ppm.
³¹P-NMR (101.2 MHz, CDCl₃, 300 K): δ = 86.6 ppm.

Example C5: Preparation of

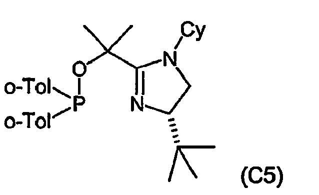

Using the procedure of Example C1, the alcohol B3 (70 mg, 0.26 mmol) is reacted with bis(*ortho*-tolyl)chlorophosphane (93 mg, 0.34 mmol). Column chromatography gives a colourless oil (29 mg, 0.062 mmol, 24%).
¹H-NMR (250.1 MHz, CDCl₃, 300 K): δ = 0.88 (s br, 11H, C(CH₃)₃ and Cy-H), 1.20 - 1.64 (m, 8H, CH₂, Cy-H), 1.65 (s br, 6H, C(CH₃)₂), 2.35 (s, 3H, CH₃, *o*-Tol), 2.42 (s, 3H, CH₃, *o*-Tol), 3.12 -3.35 (m br, 2H, Cy-H and lm-H), 3.57 - 3.76 (s br, 2H, lm-H), 6.95 - 7.27 (m, 6H, Ar-H), 7.45 - 7.56 (m, 2H, Ar-H) ppm.
³¹P-NMR (101.3 MHz, CDCl₃, 300 K): δ = 71.8 ppm.

Example C6: Preparation of

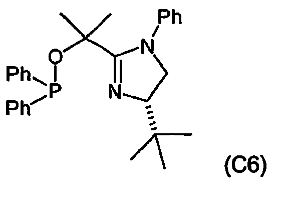

Using the procedure of Example C1, the alcohol B5 (150 mg, 0.58 mmol, 79%) is reacted with Ph₂PCl (106 µl, 0.57 mmol). After column chromatography (pentane/triethylamine 9:1), a light-yellow liquid (125 mg, 64%) is obtained.
¹H-NMR (250.1 MHz, CDCl₃, 300 K): δ = 0.96 (s, 9H, C(CH₃)₃), 1.60 (s, 3H, CH₃), 1.63 (s, 3H, CH₃), 3.55 (m, 1H, lm), 3.80 (d, *J*= 2.5 Hz, 2H, lm), 7.05 (d, *J* = 2.3 Hz, 5H, Ar-H), 7.29 (m, 10H, Ar-H) ppm.
¹³P-NMR (101.2 MHz, CDCl₃, 300 K): δ =87.5 ppm.

Example C7: Preparation of

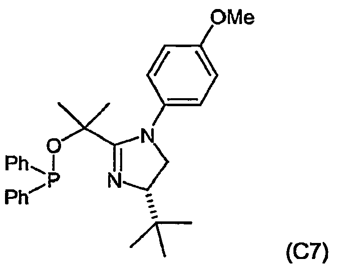

Using the procedure of Example C1, the alcohol B6 (175 mg, 0.60 mmol) is reacted with Ph₂PCl (0.78 mmol). After purification by column chromatography (pentane/Et₂O/triethylamine 8:1:1), a clear oil (162 mg, 0.34 mmol, 57%) is obtained.
¹H-NMR (250.1 MHz, CDCl₃, 300 K): δ = 0.93 (s, 9H, C(CH₃)₃), 1.59 (s, 3H, CH₃), 1.62 (s, 3H, CH₃), 3.50 (m, 1H, CH₂), 3.73 (s, 3H, OCH₃), 3.76 (m, 2H, CH₂ and CH), 6.52 (d, *J* = 9.0 Hz, 2H, p-MeOPh), 6.97 (d, *J* = 9.0 Hz, 2H, *p*-MeOPh), 7.29 (br s, 10H, Ar-H) ppm.
³¹P-NMR (101.2 MHz, CDCl₃, 300 K): δ=87.2.

Example C8: Preparation of

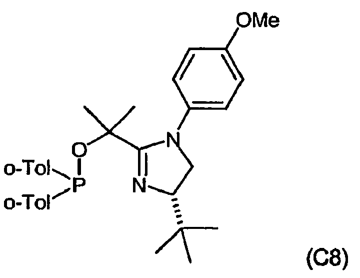

Using the procedure of Example C1, the alcohol B6 (90 mg, 0.31 mmol) is reacted with o-tolyl₂PCl (100 mg, 0.40 mmol). After column chromatography (pentane/triethylamine 9:1), the product is obtained as a colourless oil (40 mg, 26%).
¹H-NMR (400.1 MHz, CDCl₃; 300 K): δ *=* 0.95 (s, 9H, C(CH₃)₃), 1.55 (s, 6H, *o*-tolyl), 2.23 (d, *J* = 1.2 Hz, 3H, CH₃), 2.32 (d, *J* = 1.0 Hz, 3H, CH₃), 3.48 (t, *J* = 5.6 Hz, 1H, CH₂), 3.67 (s, 3H, OCH₃), 3.74 (m, 2H CH₂ and CH), 6.37 (d, *J* = 9.2 Hz, 2H, *p*-MeOPh), 6.85 (d, *J=* 8.8 Hz, 2H, *p*-MeOPh), 7.04 - 7.26 (various m, 8H, *o*-tolyl) ppm.
³¹P-NMR (161.9 MHz, CDCl₃, 300 K): δ =72.5 ppm.

Example C9: Preparation of

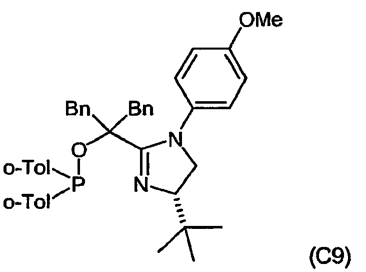

Using the procedure of Example C1, the alcohol B4 (150 mg, 0.34 mmol) is reacted with Ph₂PCl (0.442 mmol). The phosphinite C9 is obtained as a colourless oil (200 mg, 29 mmol, 86%, crude product).
¹H-NMR (250 MHz, CDCl₃, 300 K): δ = 0.70 (s, 9H, C(CH₃)₃), 2.30 (s, 6H, tolyl-CH₃), 3.15 - 3.80 (m br, 10H, OCH₃, CH₂Ph and lm-H), 6.60 - 7.80 (m, 22H, Ar-H) ppm.
³¹P-NMR (101.2 MHz, CDCl₃, 300 K): δ=87.4.

Example C10: Preparation of

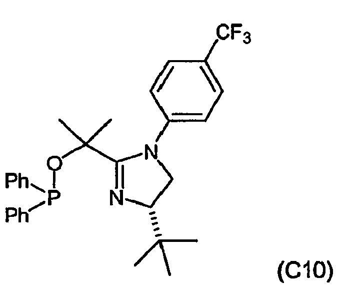

Using the procedure of Example C1, the alcohol B7 (109 mg, 0.33 mmol) is reacted with Ph₂PCl (80 µl, 0.43 mmol). After purification by column chromatography (pentane/ triethylamine 9:1), the phosphinite C10 is isolated as a pale yellow oil (113 mg, 0.22 mmol, 66%).
¹H-NMR (250.1 MHz, CDCl₃, 300 K): δ = 0.96 (s, 9H, C(CH₃)₃), 1.65 (s, 3H, CH₃),1.70 (s, 3H, CH₃), 3.57 (m, 1H, CH₂), 3.83 (m, 2H, CH and CH₂), 7.13 (s, 4H, Ar-H), 7.30 (d, *J* = 2.5 Hz, 10H, Ar-H) ppm.
³¹P-NMR (101.2 MHz, CDCl₃, 300 K): δ = 88.3 ppm.

Example C11: Preparation of

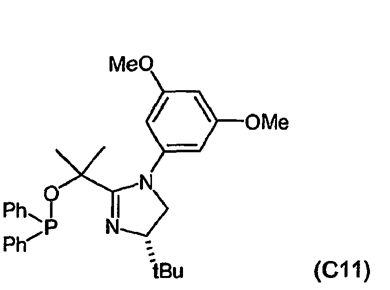

Using the procedure of Example C1, the alcohol B8 (77 mg, 0.24 mmol) is reacted with Ph₂PCl (60 µl, 0.327 mmol). After purification by column chromatography (pentane/ triethylamine 9:1), the phosphinite C11 is isolated as a pale yellow oil (38 mg, 0.075 mmol, 31%).
¹H-NMR (250.1 MHz, CDCl₃, 295 K): *δ =* 0.94 (s, 9H, C(CH₃)₃), 1.74 (s, 6H, CH₃), 3.52-3.87 (br m, 9H, CH, CH₂ and CH₃O), 6.15 (s, 1H, Ar-H), 6.37 (s, 2H, Ar-H), 7.20 - 7.68 (m, 10H, Ar-H) ppm.
³¹P-NMR (101.2 MHz, CDCl₃, 300 K): δ =88.5 ppm.

Example C12: Preparation of

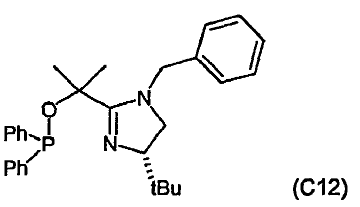

Using the procedure of Example C1, the alcohol B9 (80 mg, 0.29 mmol) is reacted with Ph₂PCl (70 µl, 0.38 mmol). After purification by column chromatography (pentane/diethyl ether/triethylamine 8:1:1), the phosphinite C12 is isolated as a pale yellow oil (51 mg, 0.11 mmol, 38%).
¹H-NMR (250.1 MHz, CDCl₃, 300 K): δ = 0.93 (s, 9H, C(CH₃)₃), 1.78 (s, 6H, CH₃), 2.89 - 3.78 (br m, 3H, CH₂), 4.06 (d, *J =* 15.2 Hz, CH₂Ph), 4.28 (d, *J* = 15.9 Hz, CH₂Ph), 6.97 - 7.90 (m, 15H, Ar-H) ppm.
³¹P-NMR (101.2 MHz, CDCl₃, 300 K): δ = 88.4 ppm.

Example C13: Preparation of

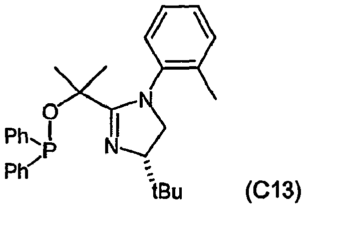

Using the procedure of Example C1, the alcohol B10 (94 mg, 0.343 mmol) is reacted with Ph₂PCl (83 µl, 0.45 mmol). After purification by column chromatography (pentane/diethyl ether/triethylamine 8:1:1), the phosphinite C13 is isolated as a pale yellow oil (61 mg, 0.132 mmol, 39%).
¹H-NMR (250.1 MHz, CDCl₃, 300 K): δ = 0.96 (s, 9H, C(CH₃)₃), 1.67 (s, 6H, CH₃), 2.15 and 2.18 (each s, total of 3H, PhCH₃), 3.11 - 3.97 (br m, total of 3H, CH and CH₂), 6.83 - 7.67 (m, 14H, Ar-H) ppm.
³¹P-NMR (101.2 MHz, CDCl₃, 300 K): δ=87.8 ppm.

Example C14: Preparation of

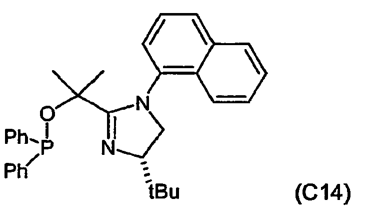

Using the procedure of Example C1, the alcohol B11 (60 mg, 0.20 mmol) is reacted with Ph₂PCl (48 µl, 0.26 mmol). After purification by column chromatography (pentane/diethyl ether/triethylamine 8:1:1), the phosphinite C14 is isolated as a pale yellow oil (48 mg, 0.097 mmol, 48 %).
¹H-NMR (400.1 MHz, CDCl₃, 300 K): δ = 1.00 and 1.03 (s, 9H, C(CH₃)₃), 1.54, 1.60 and 1.70 (s, 6H, CH₃), 3.40 - 4.16 (br m, total of 3H, CH and CH₂), 6.96 - 8.10 (total of 17H, Ar-H) ppm.
³¹P-NMR (101.2 MHz, CDCl₃, 300 K): δ = 87.5 ppm.

Example C15: Preparation of

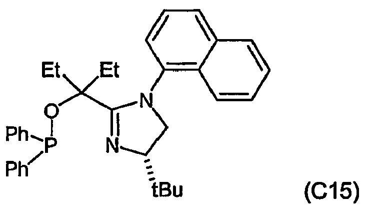

Using the procedure of Example C1, the alcohol B12 (60 mg, 0.18 mmol) is reacted with Ph₂PCl (43 µl, 0.26 mmol). After removal of the solvent, the phosphinite C15 is converted in situ into the corresponding iridium complex.

Example C16: Preparation of

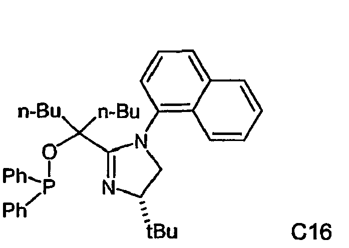

Using the procedure of Example C1, the alcohol B13 (15 mg, 0.063 mmol) is reacted with Ph₂PCl (9.2 µl, 0.082 mmol). After removal of the solvent, the phosphinite C16 is converted in situ into the corresponding iridium complex.

Example C17: Preparation of

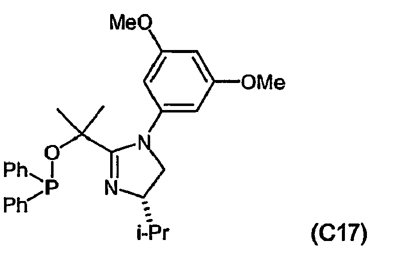

Using the procedure of Example C1, the alcohol B14 (120 mg, 0.392 mmol) is reacted with Ph₂PCl (94 µl, 0.51 mmol). After purification by column chromatography (pentane/diethyl ether/triethylamine 9:1), the phosphinite C17 is isolated as a pale yellow oil (20 mg, 0.041 mmol, 10%).
¹H-NMR (400.1 MHz, CDCl₃, 300 K): δ = 0.96 (pt, 6H, CH(CH₃)₂), 1.73 (br s, 6H, CH₃), 1.96 (br, 1H, CH(CH₃)₂), 3.52 - 3.97 (br m, 9H, CH, CH₂ and CH₃O), 6.13 (t, *J* = 2.0 Hz, 1H, Ar-H), 6.37 (d, *J* = 2.3 Hz, 2H, Ar-H), 7.20 - 7.68 (m, 10H, Ar-H) ppm.
³¹P-NMR (101.2 MHz, CDCl₃, 300 K): δ=89.2 ppm.

### D) Preparation of metal complexes

### Example D1: Ir complex D1 with phosphinite C1 (COD is cyclooctadiene)

[lr(COD)Cl]₂ (27 mg, 0.039 mmol) is placed in a reaction vessel together with dichloromethane (0.5 ml). The phosphinite C1 (32 mg, 0.071 mmol, dissolved in dichloromethane, 4.0 ml) is added dropwise to the solution and the mixture is subsequently heated to 45°C. After 2 h, the solution is admixed with sodium tetra(bistrifluoromethyl)phenyl)borate (NaBAr_{F}) (74 mg, 0.078 mmol) and water. After phase separation and extraction of the aqueous phase with dichloromethane (10 ml), the combined organic extracts are dried over MgSO₄ and the dichloromethane is subsequently removed on a rotary evaporator. The orange foam formed is purified by column chromatography on silica gel (dichloromethane). This gives the complex D1 as an orange solid (105 mg, 91%).
¹H-NMR (500.1 MHz, CDCl₃, 295 K): δ=- 0.04 (d, *J* = 6.5 Hz, 3H, CH(CH₃)₂), 0.74 (d, *J* = 7.0 Hz, 3H, CH(CH₃)₂), 1.05 (m, 1H, CH₂), 1.23 (m, 2H, CH₂), 1.40-1.70 (m, 5H, COD and Cy), 1.70 -1.80 (m, 5H, COD and Cy), 1.80 (m, 1H, CH(CH₃)₂), 1.90 (d, J = 2.0 Hz, 3H, CH₃), 1.94 (m, 1H, CH₂), 2.10 (m, 1H, CH₂), 2.34 (m, 1H, CH₂), 2.34 (s, 3H, CH₃), 2.41 (m, 1H, CH₂), 2.55 (m, 2H, CH₂), 3.23 (m, 1H, CH, COD), 3.39 (dd, *J* =11.0, 5.0 Hz, 1H, CH₂, lm), 3.46 (t, *J =* 11.5 Hz, 1H, CH₂, lm), 3.64 (m, 1H, CH, lm), 3.85 (m, 1H, CH, Cy), 5.04 (m, 1H, CH, COD), 5.20 (m, 1H, CH, COD), 7.11 (m, 2H, Ar-H), 7.41-7.47 (m, 6H, Ar-H), 7.52 (s br, 3H, BAr_{F}), 7.54 (m, 1H, Ar-H), 7.71 (s, 8H, BAr_{F}), 7.83 (2H, N-Ar-H) ppm.
³¹P-NMR (161.9 MHz, CDCl₃, 300 K): *δ* = 94.7 ppm.

### Example D2: Ir complex D2 with phosphinite C2

The procedure of Example D1 is repeated using the phosphinite C2 (80 mg, 0.186 mmol), [Ir(COD)Cl]₂ (69 mg, 0.102 mmol) and NaBAr_{F} (193 mg, 0.205 mmol), giving the complex D2 (210 mg, 71%).
¹H-NMR (400.1 MHz, CDCl₃, 300 K): δ = 0.10 (d, *J =* 6.4 Hz, 3H, CH(CH₃)₂), 0.77 (d, *J* = 7.2 Hz, 3H, CH(CH₃)₂), 1.17 (s, 3H, CH₃), 1.60 (m, 1H, CH₂, COD), 1.80 (m, 1H, CH₂, COD), 1.91 (m, 1H, CH₂, COD), 1.99 (m, 1H, CH₂, COD), 2.16 (m, 1H, CH₂, COD), 2.38 (m, 2H, CH₂, COD), 2.42 (s, 3H, CH₃), 2.59 (m, 3H: CH (COD) and CH₂, (COD) and CH(CH₃)₂), 3.37 (m, 1H, CH, COD), 3.72 (d, *J* = 8.8 Hz, 2H, lm), 3.85 (t, *J* = 8.5 Hz, 1H, lm), 5.08 (m, 1H, CH, COD), 5.31 (m, 1H, CH, COD), 7.09 (m, 4H, Ar-H), 7.41 (s, 6H, Ar-H), 7.52 (s, 7H, Ar-H and BAr_{F}), 7.71 (s, 8H, BAr_{F}), 7.86 (m, 2H, Ar-H) ppm.
³¹P-NMR (161.9 MHz, CDCl₃, 300 K): δ=93.8 ppm.

### Example D3: Ir complex D3 with phosphinite C3

The procedure of Example D1 is repeated using the phosphinite C3 (72 mg, 0.157 mmol), [Ir(COD)Cl]₂ (58 mg, 0.0864 mmol) and NaBAr_{F} (161 mg, 0.173 mmol), giving the complex D3 (198 mg, 78%).
¹H-NMR (400.1 MHz, CDCl₃, 300 K): δ = 0.30 (d, *J* = 6.6 Hz, 3H, CH(CH₃)₂), 0.85 (d, *J* = 6.8 Hz, 3H, CH(CH₃)₂), 1.26 (s br, 4H, CH₃ and CH(CH₃)), 1.72 (m, 1H, CH₂, COD), 1.80 (m, 1H, CH₂, COD), 2.05 - 2.50 (m, 15H, COD, C(CH₃)₂ and tolyl-CH₃), 3.12 (m, 3H, CH(COD), 3.67 - 3.91 (m, 3H, Im-H), 5.06 (m, 1H, CH, COD), 5.30 (m, 1H, CH, COD), 6.97 (m, 2H, Ar-H), 7.19 - 7.52 (m br, 16H, Ar-H and BAr_{F}), 7.71 (s, 8H, BAr_{F}), 8.31 (m, 1H, Ar-H) ppm.
³¹P-NMR (161.9 MHz, CDCl₃, 300 K): δ = 101.0 ppm.

### Example D4: Ir complex D4 with phosphinite C4

The procedure of Example D1 is repeated using the phosphinite C4 (38 mg, 0.087 mmol), [Ir(COD)Cl]₂ (32 mg, 0.0479 mmol) and NaBAr_{F} (89 mg, 0.0957 mmol), giving the complex D4 (82 mg, 0.0508 mmol, 58%).
¹H-NMR (400.1 MHz, CDCl₃, 300 K): δ = 0.53 (s, 9H, C(CH₃)), 1.06 (m, 1H, CH₂), 1.29 (m, 2H, CH₂), 1.46 - 2.10 (m, 12H, CH₂ and CH₃), 2.36 (m, 6H, CH₂ and CH₃), 2.55 (m, 3H, CH and CH₂(COD)), 3.37 - 3.58 (m, 4H, CH, Im-H, Cy-H, COD), 3.80 (m, 1H, CH), 5.15 (s br, 2H, CH(COD)), 7.18 (m, 2H, Ar-H), 7.40 (m, 3H, Ar-H), 7.51 (s br, 7H, Ar-H and BAr_{F}), 7.71 (s, 8H, BAr_{F}), 7.75 (m, 2H, Ar-H) ppm.
³¹P-NMR (161.9 MHz, CDCl₃, 300 K): δ=94.6 ppm.

### Example D5: lr complex D5 with phosphinite C5

The procedure of Example D1 is repeated using the phosphinite C5 (29 mg, 0.0624 mmol), [Ir(COD)Cl]₂ (23 mg, 0.0343 mmol) and NaBAr_{F} (64 mg, 0.0686 mmol), giving the complex D5 (65 mg, 0.0396 mmol, 63%).
¹H-NMR (400.1 MHz, CDCl₃, 300 K): δ = 0.89 (s, 9H, C(CH₃)), 1.20 (m, 4H, CH₂), 1.45 - 2.05 (m, 19H), 2.20 - 2.47 (m, 12H, CH₂ and CH₃), 3.12 (m, 1H, CH(COD)), 3.45 - 3.62 (m, 4H, CH, lm-H, Cy-H), 4.88 (m, 1H, CH(COD), 5.34 (s br, 1H, CH(COD)), 7.03 - 7.52 (m, 13H, Ar-H and BAr_{F}), 7.71 (s, 8H, BAr_{F}), 7.98 (m, 1H, Ar-H) ppm.
³¹P-NMR (161.9 MHz, CDCl₃, 300 K): δ = 106.2 ppm (impurity at 38.8 ppm).

### Example D6: Ir complex D6 with phosphinite C6

The procedure of Example D1 is repeated using the phosphinite C6 (125 mg, 0.28 mmol), [Ir(COD)Cl]₂ (103 mg, 0.154 mmol) and NaBAr_{F} (264 mg, 0.28 mmol), giving the complex D6 (260 mg, 58%).
¹H-NMR (500.1 MHz, CDCl₃, 295 K): δ = 0.68 (s, 9H, C(CH₃)₃), 1.26 (s, 3H, CH₃), 1.54 (m, 1H, CH₂, COD), 1.73 (m, 1H, CH₂, COD), 2.00 (m, 1H, CH₂, COD), 2.08 (m, 1H, CH₂, COD), 2.38 (m, 2H, CH₂, COD), 2.53 (s, 3H, CH₃), 2.62 (m, 3H, CH₂ and CH, COD), 3.61 (d, *J* = 11.5 Hz, 1H, CH, Im), 3.64 (m, 1H, CH, COD), 3.80 (t, *J* = 11.5 Hz, 1H, CH₂, Im), 3.88 (d, *J* =11.5 Hz, 1H, CH₂, Im), 5.02 (m, 1H, CH, COD), 5.29 (m, 1H, CH, COD), 7.14 (m, 4H, Ar-H), 7.39 - 7.43 (m, 6H, Ar-H), 7.54 (s br, 7H, BAr_{F} and Ar-H), 7.71 (s br, 9H, BAr_{F} and Ar-H), 7.76 (m, 1H, Ar-H) ppm.
³¹P-NMR (161.9 MHz, CDCl₃, 300 K): δ = 91.1 ppm.

### Example D7: Ir complex D7 with phosphinite C7

The procedure of Example D1 is repeated using the phosphinite C7 (80 mg, 0.17 mmol), [Ir(COD)Cl]₂ (63 mg, 0.0935 mmol) and NaBAr_{F} (159 mg, 0.17 mmol), giving the complex D7 (175 mg, 63%).
¹H-NMR (400.1 MHz, CDCl₃, 300 K): δ = 0.67 (s, 9H, C(CH₃)₃), 1.30 (s, 3H, CH₃), 1.53 (m, 1H, CH₂, COD), 1.70 (m, 1H, CH₂, COD), 2.0 (m, 2H, CH₂, COD), 2.37 (m, 2H, CH₂, COD), 2.51 (s, 3H, CH₃), 2.61 (m, 3H, CH₂ and CH, COD), 3.60 (m, 2H, CH (COD), CH₂ (lm)), 3.84 (s, 3H, OCH₃), 3.85 (m, 1H; CH, lm), 3.87 (d, *J* = 7.6 Hz, 1H, CH₂, lm), 5.03 (m, 1H, CH, COD), 5.28 (m, 1H, CH, COD), 6.92 (d, *J* = 8.8 Hz, 2H, 4-MeOPh), 7.05 (m, 2H, Ar-H), 7.15 (d x d, *J* = 11.0 Hz, 2H, 4-MeOPh), 7.39 (s br, 4H, BAr_{F}, Ar-H), 7.52 (s br, 7H, BARF, Ar-H), 7.71 (s br, 8H, BAr_{F}) ppm.
³¹P-NMR (161.9 MHz, CDCl₃, 300 K): δ= 91.1 ppm.

### Example D8: Ir complex D8 with phosphinite C8

The procedure of Example D1 is repeated using the phosphinite C8 (40 mg, 0.082 mmol), [Ir(COD)Cl]₂ (30 mg, 0.045 mmol) and NaBAr_{F} (77 mg, 0.082 mmol), giving the complex D8 (50 mg, 0.030 mmol, 37%).
¹H-NMR (400.1 MHz, CDCl₃, 300 K): δ=0.88 (s br, 9H, C(CH₃)₃), 1.26 (s, 3H, *o*-tolyl), 1.52 (s, 3H, *o*-tolyl), 1.66 (m, 1H, COD), 1.90 - 2.20 (m, 6H, COD), 2.29 (m, 1H, COD), 2.31 (s, 3H, CH₃), 2.35 (s, 3H, CH₃), 2.55 (m, 2H, COD), 3.72 (m, 1H, Im), 3.78 (s, 3H, OCH₃), 3.82 (m, 2H, Im), 4.91 (m, 1H, CH, COD), 5.33 (m, 1H, CH, COD), 6.89 (d, *J* = 8.8 Hz, 2H, meta, 4-MeOPh), 7.00 - 7.30 (m, 9H, Ar-H), 7.40 (t, *J* = 8.0 Hz, 1H, Ar-H), 7.52 (s, br, 4H, BAr_{F}), 7,72 (s, br, 8H, BAr_{F}) ppm.
³¹P-NMR (161.9 MHz, CDCl₃, 300 K): δ = 101.8 ppm.

### Example D9: Ir complex D9 with phosphinite C9

The procedure of Example D1 is repeated using the phosphinite C9 (213 mg, 0.34 mmol), [Ir(COD)Cl]₂ (126 mg, 0.187 mmol) and NaBAr_{F} (352 mg, 0.37 mmol), giving the complex D9 (380 mg, 62%).
¹H-NMR (500.1 MHz, CDCl₃, 295 K): δ = 0.78 (s, 9H, C(CH₃)₃), 1.58 (m, 1H, CH₂, COD), 1.85 (m, 1H, CH₂, COD), 1.95 (m, 1H, CH₂, COD), 2.22 (m, 1H, CH₂, COD), 2.37 (m, 1H, CH₂, COD), 2.45 (m, 1H, CH₂, COD), 2.60 (m, 2H: 1H CH₂, COD; 1H of CH₂, Bn-H), 2.65 (m, 1H, CH₂, COD), 2.75 (m, 1H, CH, COD), 2.97 (d, J=15.0 Hz, 1H, CH₂, Bn-H), 3.60 (m, 4H: 1H of CH, COD; 1H of CH₂, Bn-H; 1H of CH, lm; 1H of CH₂, lm), 3.80 (s, 3H, OCH₃), 3.88 (t, *J*=11.5 Hz, 1H, CH₂, lm), 4.80 (dd, *J* = 9.0, 3.0 Hz, 1H, Ph-H) 5.36 (m, 1H, CH, COD), 5.37 (d, *J*=12.0 Hz, 1H, CH₂, Bn-H), 5.51 (m, 1H, CH, COD), 6.43 (dd, *J*= 9.0, 3.0 Hz, 1H, Ar-H), 6.61 (dd, *J* = 9.0, 3.0 Hz, 1H, Ar-H), 6.71 (dd, *J* = 9.0 Hz, 3.0 Hz, 1H, Ar-H), 6.84 (m, 2H, Ar-H), 7.16-7.31 (m, 9H, Ar-H), 7.32 (td, J= 7.5, 1.0 Hz, 1H, Ar-H), 7.51 (s br, 4H, BAr_{F}), 7.53-7.59 (m, 10H, Ar-H), 7.72 (s br, 8H, BAr_{F}), 7.92 (m, 2H, Ar-H) ppm.
³¹P-NMR (161.9 MHz, CDCl₃, 300 K): δ=89.6 ppm.

### Example D10: Ir complex D10 with phosphinite C10

The procedure of Example D1 is repeated using the phosphinite C10 (110 mg, 0.22 mmol), [Ir(COD)CI]₂ (81 mg, 0.121 mmol) and NaBAr_{F} (226 mg, 0.24 mmol) giving the complex D10 (200 mg, 55%).
¹H-NMR (400.1 MHz, CDCl₃, 300 K): δ = 0.69 (s, 9H, C(CH₃)₃), 1.28 (s, 3H, CH₃), 1.53 (m, 1H, CH₂, COD), 1.73 (m, 1H, CH₂, COD), 1.97 (m, 1H, CH₂, COD), 2.07 (m, 1H, CH₂, COD), 2.34 (m, 2H, CH₂, COD), 2.55 (s, 3H, CH₃), 2.55 (m, 3H, CH and CH₂, COD), 3.66 (m, 2H, 1H (COD) and 1H (Im)), 3.80 (t, *J* = 11.0 Hz, 1H, Im), 3.87 (d, *J* = 11.0 Hz, 1H, Im), 5.00 (m, 1H, CH, COD), 5.28 (m, 1H, CH, COD), 7.14 (t, *J* = 9.5 Hz, 2H, meta, Ar-H), 7.29 (d, *J* = 7.6 Hz, 2H, Ar-H), 7.40 (m, 3H, Ar-H), 7.51 (d, *J* = 10.8 Hz, 7H, BAr_{F} and Ar-H), 7.71 (s br, 8H, BAr_{F}), 7.75 (d, *J* = 10.5 Hz, 4H, Ar-H) ppm.
³¹P-NMR (161.9 MHz, CDCl₃, 300 K): δ = 91.7 ppm.
¹⁹F-NMR (376.4 MHz, CDCl₃, 300 K): δ = - 64.1 (*p*-CF₃-phenyl), - 63.5 (CF₃, BAr_{F}) ppm.

### Example D11: Ir complex D11 with phosphinite C11

The procedure of Example D1 is repeated using the phosphinite C11 (38 mg, 0.075 mmol), [Ir(COD)Cl]₂ (28 mg, 0.0414 mmol) and NaBAr_{F} (77 mg, 0.0825 mmol), giving the complex D11 (98 mg, 0.058 mmol, 78%).
¹H-NMR (400.1 MHz, CDCl₃, 300 K): δ = 0.67 (s, 9H, C(CH₃)₃), 1.42 (d, *J* = 2.0 Hz, 3H, CH₃), 1.56 (m, 1H, CH₂, COD), 1.72 (m, 1H, CH₂, COD), 1.91-2.13 (m, 2H, CH₂, COD), 2.37 (m, 2H, CH₂, COD), 2.56 (s, 3H, CH₃), 2.62 (m, 3H, CH₂ and CH, COD), 3.59 (m, 2H, CH (COD), CH₂(lm)), 3.79 - 3.90 (s, 8H, CH, CH₂ (lm) and OCH₃), 5.01 (m, 1H, CH, COD), 5.28 (m, 1H, CH, COD), 6.23 and 6.27 (each s, 2H, 3,5-DiMeOPh), 6.51 (t, *J* = 2.3 Hz, 1H, 3,5-DiMeOPh) 7.15 (m, 2H, Ar-H), 7.40 (m, 3H, Ar-H), 7.52 (m, 7H, BAr_{F}, Ar-H), 7.70 - 7.81 (br, 10H, BAr_{F}) ppm.
³¹P-NMR (161.9 MHz, CDCl₃, 300 K): *δ* = 91.1 ppm.

### Example D12: Ir complex D12 with phosphinite C12

The procedure of Example D1 is repeated using the phosphinite C12 (51 mg, 0.11 mmol), [Ir(COD)Cl]₂ (41 mg, 0.061 mmol) and NaBAr_{F} (114 mg, 0.122 mmol), giving the complex C12 (54 mg, 0.033 mmol, 30%).
¹H-NMR (500.1 MHz, CDCl₃, 295 K): δ=0.61 (s, 9H, C(CH₃)₃), 1.62 (m, 1H, CH₂, COD), 1.80 (m, 1H, CH₂, COD), 2.01 (d, *J =* 2.3 Hz, 3H, CH₃), 2.16 (m, 1H, CH₂, COD), 2.36 (m, 1H, CH₂, COD), 2.40 (m, 2H, CH₂, COD), 2.51 (s, 3H, CH₃), 2.66 (m, 3H, CH₂ and CH, COD), 3.43 (m, 1H, COD), 3.61 (m, 3H, CH₂ and CH, Im), 4.52 (d, *J* = 16.4 Hz, 1H, PhCH₂), 4.97 (d, *J* = 16.4 Hz, 1H, PhCH₂), 5.11 (m, 1H, CH, COD), 5.20 (m, 1H, CH, COD), 5.33 (m, 1H, COD), 7.18 - 7.47 (m, 10H, Ar-H), 7.51 (s br, 7H, BAr_{F} and Ar-H), 7.71 (s br, 8H, BAr_{F} and Ar-H), 7.84 (m, 2H, Ar-H) ppm.
³¹P-NMR (161.9 MHz, CDCl₃, 300 K): δ = 93.1 ppm.

### Example D13: Ir complex D13 with phosphinite C13

The procedure of Example D1 is repeated using the phosphinite C13 (61 mg, 0.132 mmol), [lr(COD)Cl]₂ (49 mg, 0.073 mmol) and NaBAr_{F} (135 mg, 0.145 mmol), giving the complex D13 (153 mg, 0.093 mmol, 71%).
¹H-NMR (500.1 MHz, CDCl₃, 295 K): δ = 0.73 and 0.74 (s, 9H, C(CH₃)₃), 1.29 (d, *J* = 2.3 Hz, 3H, CH₃), 1.63 (m, 1H, CH₂, COD), 1.78 (m, 1H, CH₂, COD), 1.97 - 2.17 (m, 2H, CH₂, COD), 2.29 - 2.33 (m, 3H, CH₂, COD), 2.45 (m, 2H, COD), 2.60 (m, 1H, CH₂, COD), 2.68 (m, 3H, CH and CH₂, COD), 3.45 - 3.97 (m, 3H, CH₂ and CH, lm), 5.06 (m, 1H, CH, COD), 5.33 - 5.40 (m, 3H, CH, COD), 7.05 (m, 1H, Ar-H), 7.22 (2H, Ar-H), 7.42 (m, 6H, Ar-H), 7.51 (s br, 7H, BAr_{F} and Ar-H), 7.71 (s br, 8H, BAr_{F} and Ar-H), 7.84 (m, 4H, Ar-H) ppm.
³¹P-NMR (161.9 MHz, CDCl₃, 300 K): δ = 89.9 ppm.

### Example D14: Ir complex D14 with phosphinite C14

The procedure of Example D1 is repeated using the phosphinite C14 (48 mg, 0.097 mmol), [Ir(COD)Cl]₂ (36 mg, 0.053 mmol) and NaBAr_{F} (100 mg, 0.107 mmol), giving the complex D14 (98 mg, 0.059 mmol, 61%).
¹H-NMR (400.1 MHz, CDCl₃, 295 K): δ=0.75 and 0.80 (s, 9H, C(CH₃)₃), 1.16 (s, 3H, CH₃), 1.58 (m, 1H, CH₂, COD), 1.76 (m, 1H, CH₂, COD), 2.00 (m, 1H, CH₂, COD), 2.11 (m, 1H, CH₂, COD), 2.40 (m, 2H, CH₂, COD), 2.50 and 2.60 (each s, together 3H, CH₃), 2.65 (m, 3H, CH₂ and CH, COD), 3.64 - 3.91 (m, 3H, CH lm and COD), 4.12 - 4.24 (m, 1H, CH lm), 5.06 (m, 1H, CH, COD), 5.38 (m, 1H, CH, COD), 7.16 (m, 2H, Ar-H), 7.28 (m, 1H, Ar-H), 7.39 (m, 3H, Ar-H), 7.46 - 7.68 (m, 10H, Ar-H and BAr_{F}-H), 7.72 (s br, 8H, BAr_{F}), 7.78 (m, 1H, Ar-H), 7.96 (m, 2H, Ar-H) ppm.
³¹P-NMR (161.9 MHz, CDCl₃, 300 K): δ = 91.7 ppm.

### Example D15: Ir complex D15 with phosphinite C15

Using the procedure of Example D1, the phosphinite C15 is reacted in situ with [Ir(COD)Cl]₂ (65 mg, 0.097 mmol) and NaBAr_{F} (182 mg, 0.195 mmol), to give the complex D15 (131 mg, 46%).
¹H-NMR (400.1 MHz, CDCl₃, 295 K): δ = 0.75 - 0.89 (m, 12H, CH₃ and C(CH₃)₃), 1.11 (pt, 2H, CH₃), 1.28 (pt, 1H, CH₃), 1.58 (m, 4H, CH₂, COD and Et), 1.76 (m, 1H, CH₂, COD), 2.00 (m, 1H, CH₂, COD), 2.11 (m, 1H, CH₂, COD), 2.40 (m, 2H, CH₂, COD), 2.65 (m, 3H, CH₂, COD and Et), 3.61 - 4.07 (m, 3H, CH lm and COD), 4.45 (m, 1H, CH lm), 4.98 (m, 1H, CH, COD), 5.37 (m, 1H, CH, COD), 7.06 (m, 1H, Ar-H), 7.15 (m, 2H, Ar-H), 7.39 (m, 3H, Ar-H), 7.46 - 7.68 (m, 10H, Ar-H and BAr_{F}-H), 7.72 (s br, 8H, BAr_{F}), 7.78 (m, 1H, Ar-H), 7.96 (m, 2H, Ar-H) ppm.
³¹P-NMR (161.9 MHz, CDCl₃, 300 K): δ = 90.9 ppm.

### Example D16: Ir complex D16 with phosphinite C16

Using the procedure of Example D1, the phosphinite C16 is reacted in situ with [Ir(COD)Cl]₂ (14 mg, 0.021 mmol) and NaBAr_{F} (39 mg, 0.042 mmol), to give the complex D16 (45 mg, 74%).
¹H-NMR (400.1 MHz, CDCl₃, 295 K): δ = 0.28 and 0.55 (pt, together 3H, CH₃ Bu) 0.77 - 2.71 (m, 31H, CH₂ and CH₃ Bu, C(CH₃)₃, COD), 3.61 - 4.07 (m, 3H, CH Im and COD), 4.41 (m, 1H, CH lm), 5.04 (m, 1H, CH, COD), 5.38 (m, 1H, CH, COD), 7.12 (m, 3H, Ar-H), 7.39 (m, 3H, Ar-H), 7.46 - 7.68 (m, 10H, Ar-H and BAr_{F}-H), 7.72 (s br, 8H, BAr_{F}), 7.78 (m, 1H, Ar-H), 7.95 (m, 2H, Ar-H) ppm.
³¹P-NMR (161.9 MHz, CDCl₃, 300 K): δ = 92.1 and 92.8 ppm.

### Example D17: Ir complex D17 with phosphinite C17

The procedure of Example D1 is repeated using the phosphinite C17 (20 mg, 0.041 mmol), [Ir(COD)Cl]₂ (15 mg, 0.0224 mmol) and NaBAr_{F} (42 mg, 0.045 mmol), giving the complex D17 (36 mg, 0.022 mmol, 53%).
¹H-NMR (400.1 MHz, CDCl₃, 300 K): δ = 0.07 (d, *J* = 6.6 Hz, 3H, CH(CH₃)₂), 0.77 (d, *J* = 6.8 Hz, 3H, CH(CH₃)₂), 1.34 (d, *J* = 1.8 Hz, 3H, CH₃), 1.55 - 2.67 (br m, 10H, CH(CH₃)₂), CH and CH₂ (COD)), 2.44 (s, 3H, CH₃), 3.36 (m, 1H, CH, COD), 3.71- 3.82 (m, 9H, CH and CH₂(lm), OCH₃), 5.06 (m, 1H, CH, COD), 5.28 (m, 1H, CH, COD), 6.12 and 6.24 (each s, 1H, Ar-H, 3,5-DiMeOPh), 6.48 (t, *J* = 2.3 Hz, 1H, 3,5-DiMeOPh) 7.10 (m, 2H, Ar-H), 7.41 (m, 3H, Ar-H), 7.52 (m, 7H, BAr_{F}, Ar-H), 7.71 (br, 8H, BAr_{F}), 7.85 (m, 2H, Ar-H) ppm.
³¹P-NMR (161.9 MHz, CDCl₃, 300 K): δ = 93.8 ppm.

### E) Use examples

### Example E1: Hydrogenation of trans-α-methylstilbene

19.4 mg (0.1 mmol) of α-trans-methylstilbene are dissolved together with 1.6 mg (0.002 mmol) 6 h in 0.5 ml of dichloromethane and the solution is transferred to a steel autoclave provided with a glass insert and a magnetic stirrer. The autoclave is then pressurized at room temperature (RT) with 50 bar of H₂. After 2 hours, the autoclave is depressurized, the solvent is removed, the residue is taken up in heptane and filtered through a syringe filter (CHROMAFIL O-20/15 MS 0.2 µm, *Macherey-Nagel).* GC/MS analysis (100°C for 2 minutes, 7°C/min to 250°C) of the solution indicates complete conversion. The enantiomeric excess is determined by means of chiral HPLC (flow rate: 0.5 ml/min at 20°C; stationary phase: Daicel Chiralcel OJ, heptane/isopropanol 99:1) as 85% (tᵣ: 13.4 *(R),* 20.4 (*S*) minutes).
The results are shown in Table 1.

**Table 1:**

| Catalyst | Mol % | Time [h] | Conversion [%] | ee [%] |
|---|---|---|---|---|
| D1 | 1 | 2 | 30 | 21 |
| D2 | 1 | 2 | 72 | 79 |
| D3 | 1 | 2 | >99 | 86 |
| D4 | 1 | 2 | 38 | 4 |
| D6 | 1 | 2 | 92 | 55 |
| D7 | 1 | 2 | >99 | 61 |
| D8 | 1 | 2 | 42 | 90 |
| D10 | 1 | 2 | 97 | 71 |
| D11 | 1 | 2 | 15 | 60 |
| D12 | 1 | 2 | 8 | 35 |
| D13 | 1 | 2 | 39 | 19 |
| D14 | 1 | 2 | 31 | 7 |
| D15 | 1 | 2 | 18 | 3 |

### Example E2: Hydrogenation of trans-2-(4-methoxyphenyl)-2-butene

The procedure was analogous to Example E1. The determination of the enantiomeric excess is carried out by means of chiral HPLC (Daicel Chiracel OD-H, 100% heptane) (tᵣ: 13.8 (S), 15.5 (R)).
The results are shown in Table 2.

**Table 2:**

| Catalyst | Mol % | Time [h] | Conversion [%] | ee [%] |
|---|---|---|---|---|
| D2 | 1 | 2 | >99 | 90 |
| D3 | 1 | 2 | >99 | 91 |
| D6 | 1 | 2 | 26 | 25 |
| D7 | 1 | 2 | 85 | 50 |
| D8 | 1 | 2 | >99 | 84 |
| D9 | 1 | 2 | 5 | 45 |
| D14 | 1 | 2 | >99 | 61 |
| D15 | 1 | 2 | >99 | 32 |
| D 17 | 1 | 2 | >99 | 88 |

### Example E3: Hydrogenation of cis-2-(4-methoxyphenyl)-2-butene

The procedure was analogous to Example E1. The determination of the enantiomeric excess is carried out by means of chiral HPLC (Daicel Chiracel OD-H, 100% heptane) (tᵣ: 13.8 (S), 15.5 (R)).
The results are shown in Table 3a.

**Table 3a:**

| Catalyst | Mol % | Time [h] | Conversion [%] | ee [%] |
|---|---|---|---|---|
| D2 | 1 | 2 | >99 | 94 |
| D3 | 1 | 2 | >99 | 92 |
| D4 | 1 | 2 | >99 | 4 |
| D5 | 1 | 2 | >99 | 82 |
| D6 | 1 | 2 | >99 | 55 |
| D7 | 1 | 2 | >99 | 61 |
| D8 | 1 | 2 | 97 | 76 |
| D9 | 1 | 2 | 18 | 17 |
| D10 | 1 | 2 | 97 | 71 |
| D11 | 1 | 2 | 75 | 73 |
| D12 | 1 | 2 | 48 | 40 |
| D13 | 1 | 2 | >99 | 32 |
| D14 | 1 | 2 | >99 | 45 |
| D15 | 1 | 2 | >99 | 7 |
| D17 | 1 | 2 | 99 | 89 |

### Comparative example:

Hydrogenation of cis-2-(4-methoxyphenyl)-2-butene using analogous phosphinite-oxazoline ligands (structure (D)) , Pfaltz et al., Adv. Synth. Catal. 2003, 345, numbers 1 + 2, pages 33 to 43): 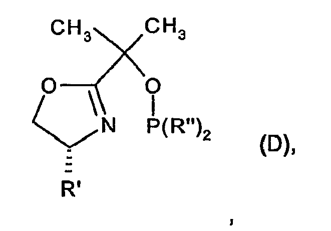 S: R' is i-propyl, R" is phenyl, T: R' is i-propyl, R" is o-tolyl, U: R' is t-butyl, R" is o-tolyl.

The results are shown in Table 3b.

**Table 3b:**

| Catalyst | Mol % | Time [h] | Conversion [%] | ee [%] |
|---|---|---|---|---|
| S (comparison) | 1 | 2 | >99 | 89 |
| D2 | 1 | 2 | >99 | 94 |
| T (comparison) | 1 | 2 | >99 | 85 |
| D3 | 1 | 2 | >99 | 92 |
| U (comparison) | 1 | 2 | >99 | 66 |
| D8 | 1 | 2 | >99 | 76 |

An enantiomeric excess of 90% and more is of great economic importance since the pure diastereomer can be obtained in only one or very few recrystallization steps.

### Example E4: Hydrogenation of 2-(4-methoxyphenyl)-1-butene

The hydrogenation is carried out in a manner analogous to Example E2.
The results are shown in Table 4.

**Table 4:**

| Catalyst | Mol % | Time [h] | Conversion [%] | ee [%] | T [°C] | p [bar] |
|---|---|---|---|---|---|---|
| D2 | 1 | 2 | >99 | 44 | 25 | 50 |
| D3 | 1 | 2 | >99 | 37 | 25 | 50 |
| D6 | 1 | 2 | >99 | 36 | 25 | 50 |
| D7 | 1 | 2 | >99 | 36 | 25 | 50 |
| D5 | 1 | 2 | >99 | 2 | 25 | 50 |
| D10 | 1 | 2 | >99 | 46 | 25 | 50 |
| D14 | 1 | 2 | >99 | 22 | 25 | 50 |
| D15 | 1 | 2 | >99 | 11 | 25 | 50 |

### Example E5: Hydrogenation of ethyl trans-β-methylcinnamate

The procedure is analogous to Example E1. The determination of the enantiomeric excess is carried out by means of chiral HPLC (Daicel Chiracel OB-H, 100% heptane/isopropanol 99.5:0.5) (tᵣ: 24.3 (S), 29.4 (R)).
The results are shown in Table 5.

**Table 5:**

| Catalyst | Mol % | Time [h] | Conversion [%] | ee [%] |
|---|---|---|---|---|
| D2 | 1 | 2 | >99 | 85 |
| D3 | 1 | 2 | >99 | 91 |
| D6 | 1 | 2 | 87 | 30 |
| D7 | 1 | 2 | >99 | 31 |
| D8 | 1 | 2 | 8 | 3 |
| D9 | 1 | 2 | 61 | 48 |
| D14 | 1 | 2 | >99 | 24 |
| D15 | 1 | 2 | >99 | 23 |

### Example E6: Hydrogenation of cis-2-methyl-3-phenylprop-2-enol

The procedure is analogous to Example E1. The determination of the enantiomeric excess is carried out by means of chiral HPLC (Daicel Chiracel OD-H, 100% heptane/isopropanol 95:5) (tᵣ: 15.4 (+), 17.7 (-)).
The results are shown in Table 6.

**Table 6:**

| Catalyst | Mol % | Time [h] | Conversion | ee [%] |
|---|---|---|---|---|
| D2 | 2 | 2 | >99 | 95 |
| D3 | 1 | 2 | >99 | 94 |
| D6 | 2 | 2 | >99 | 78 |
| D14 | 1 | 2 | >99 | 68 |
| D15 | 1 | 2 | 65 | 29 |

### Example D7: Hydrogenation of 6-methoxy-1-methyl-3,4-dihydronaphthalene

The procedure is analogous to Example E1. The determination of the enantiomeric excess is carried out by means of chiral HPLC (Daicel Chiracel OD-H, 100% heptane) (tᵣ: 24.8 (R), 29.7 (S)).
The results are shown in Table 7.

**Table 7:**

| Catalyst | Mol % | Time [h] | Conversion [%] | ee [%] |
|---|---|---|---|---|
| D2 | 1 | 2 | >99 | 88 |
| D4 | 1 | 2 | 20 | 70 |
| D6 | 1 | 2 | >99 | 83 |
| D9 | 1 | 2 | 54 | 14 |
| D14 | 2 | 2 | 95 | 71 |
| D15 | 2 | 2 | 76 | 25 |

## Claims

1. Compounds of the formulae I and la, 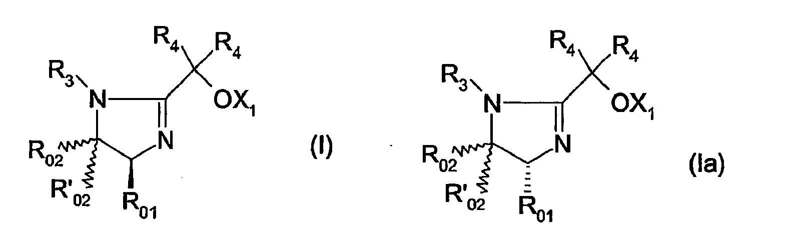 where
X₁ is secondary phosphino;
R₃ is a hydrocarbon radical having from 1 to 20 C atoms, a heterohydrocarbon radical which is bound via a C atom and has from 2 to 20 atoms and at least one heteroatom selected from the group consisting of O, S, NH and NR, or an -SO₂-R radical;
R is C₁-C₁₈-alkyl, phenyl or benzyl;
the radicals R₄ are each, independently of one another, hydrogen or a hydrocarbon radical having from 1 to 20 C atoms, or the two radicals R₄ together with the C atom to which they are bound form a three- to eight-membered hydrocarbon ring;
R₀₁ is a hydrocarbon radical having from 1 to 20 C atoms; and
R₀₂ and R'₀₂ are each a hydrogen atom or independently have the meaning of R₀₁, or
R₀₁ and R₀₂ together with the C atom to which they are bound form a three- to eight-membered hydrocarbon or heterohydrocarbon ring.

2. Compounds according to Claim 1, **characterized in that** X₁ as phosphine group contains two identical or two different hydrocarbon radicals having from 1 to 22 C atoms, or the two hydrocarbon atoms together with the P atom form a 3- to 8-membered ring.

3. Compounds according to Claim 2, **characterized in that** X₁ is the group -PR₁R₂, where R₁ and R₂ are each, independently of one another, a hydrocarbon radical which has from 1 to 20 C atoms and is unsubstituted or substituted by halogen, C₁-C₆-alkyl, C₁-C₆-haloalkyl, C₁-C₆-alkoxy, C₁-C₆-haloalkoxy, (C₆H₅)₃Si, (C₁-C₂-alkyl)₃Si or -CO₂-C₁-C₆-alkyl; or R₁ and R₂ together form an unsubstituted or C₁-C₄-alkyl- or C₁-C₄-alkoxy-substituted dimethylene, trimethylene, tetramethylene or pentamethylene.

4. Compounds according to Claim 3, **characterized in that** R₁ and R₂ are identical or different radicals selected from the group consisting of branched C₃-C₆-alkyl, unsubstituted cyclopentyl or cyclohexyl and cyclopentyl or cyclohexyl bearing from one to three C₁-C₄-alkyl or C₁-C₄-alkoxy groups as substituents, unsubstituted benzyl and benzyl bearing from one to three C₁-C₄-alkyl or C₁-C₄-alkoxy groups as substituents and unsubstituted phenyl and phenyl bearing from one to three C₁-C₄-alkyl, C₁-C₄-alkoxy, -NH₂, OH, F, Cl, C₁-C₄-fluoroalkyl or C₁-C₄-fluoroalkoxy groups as substituents.

5. Compounds according to Claim 3, **characterized in that** R₁ and R₂ are identical or different radicals selected from the group consisting of unsubstituted phenyl and phenyl substituted by from one to three C₁-C₄-alkyl, C₁-C₄-alkoxy or C₁-C₄-fluoroalkyl groups.

6. Compounds according to Claim 1, **characterized in that** the hydrocarbon radical R₃ is C₁-C₁₈-alkyl; C₃-C₁₂-cycloalkyl or C₆-C₁₆-aryl; and the heterohydrocarbon radical R₃ is C₂-C₁₈-heteroalkyl; C₃-C₁₂-heterocycloalkyl or C₃-C₁₆-heteroaryl containing from 1 to 3 heteroatoms selected from the group consisting of O, S and NR, and R is C₁-C₄-alkyl.

7. Compounds according to Claim 6, **characterized in that** the aromatic hydrocarbon radical R₃ is C₆-C₁₄-aryl.

8. Compounds according to Claim 7, **characterized in that** R₃ is C₆-C₁₀-aryl which is unsubstituted or substituted by halogen, CF₃, OCF₃, C₁-C₄-alkyl or C₁-C₄-alkoxy.

9. Compounds according to Claim 1, **characterized in that** R₄ is a hydrocarbon radical selected from the group consisting of C₁-C₁₈-alkyl, C₃-C₁₂-cycloalkyl, C₆-C₁₆-aryl or C₇-C₁₆-aralkyl.

10. Compounds according to Claim 1, **characterized in that** R₀₁ is α-branched alkyl having at least 3 C atoms, and R₀₂ and R'₀₂ are each hydrogen.

11. Compounds according to Claim 1, **characterized in that** they have the formulae Ib and Ic, 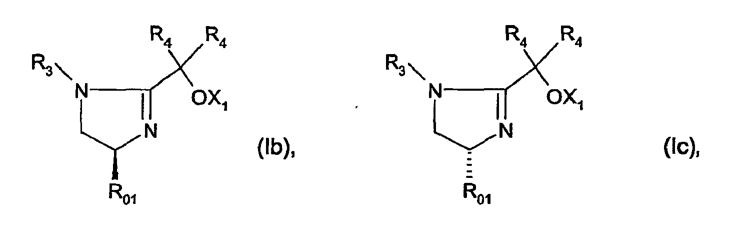 where
X₁ is -PR₁R₂,
R₁ and R₂ are identical or different and in particular identical radicals selected from the group consisting of α-branched C₃-C₆-alkyl, unsubstituted C₅-C₇-cycloalkyl and C₅-C₇-cycloalkyl bearing from one to three C₁-C₄-alkyl or C₁-C₄-alkoxy groups as substituents and
unsubstituted phenyl and phenyl bearing from one to three C₁-C₄-alkyl, C₁-C₄-alkoxy or C₁-C₄-fluoroalkyl groups as substituents and unsubstituted or C₁-C₄-alkyl- or C₁-C₄-alkoxy-substituted dimethylene, trimethylene, tetramethylene and hexamethylene;
R₃ is benzyl or C₆-C₁₂-aryl, and aryl and benzyl are unsubstituted or substituted by halogen, C₁-C₄-alkyl, C₁-C₄-haloalkyl or C₁-C₄-alkoxy;
R₄ is C₁-C₆-alkyl or benzyl, and
R₀₁ is α-branched C₃-C₈-alkyl.

12. Process for preparing compounds of the formulae I and la, 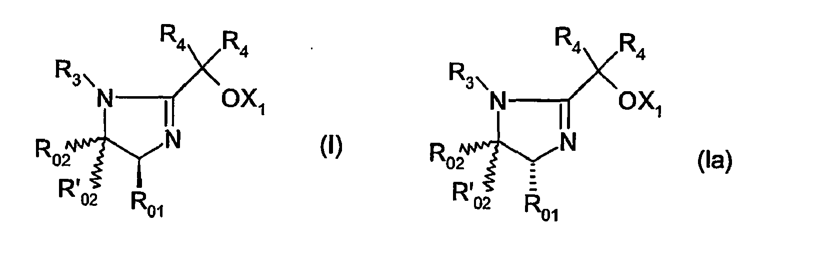 where R₀₁, R₀₂, R'₀₂, R₃, R₄ and X₁ are as defined in Claim 1, and ~ represents the R or S form, **characterized in that**
a) a compound of the formula II 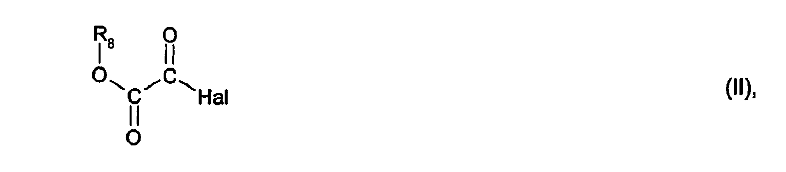 where R₈ is C₁-C₈-alkyl and Hal is Cl, Br or I, is reacted in the presence of a tertiary amine with at least an equivalent amount of a compound of the formula III, 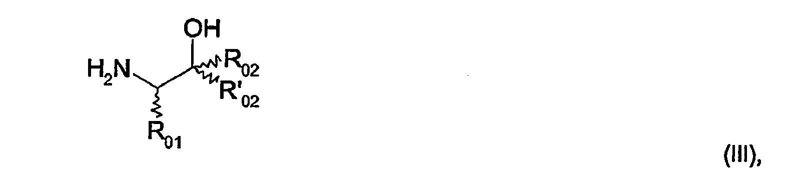 where R₀₁ and R₀₂ are as defined in Claim 1, to form a compound of the formula IV, 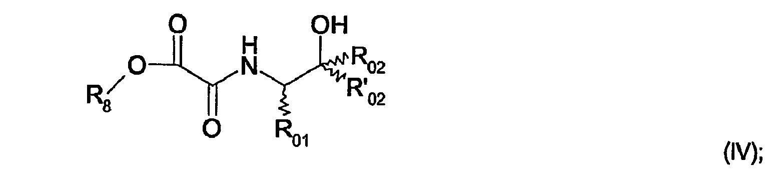
b) the compound of the formula IV is reacted with at least equivalent amounts of a halogenating agent to form a compound of the formula V, 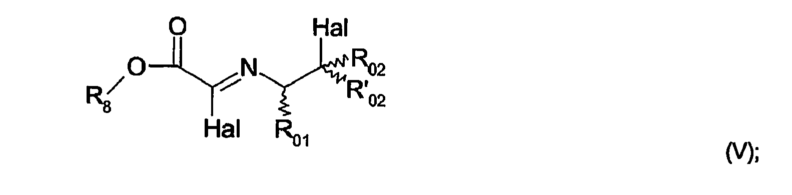
c) the compound of the formula V is cyclized with a primary amine of the formula R₃-NH₂ (X) in the presence of a tertiary amine to form a compound of the formula VI, 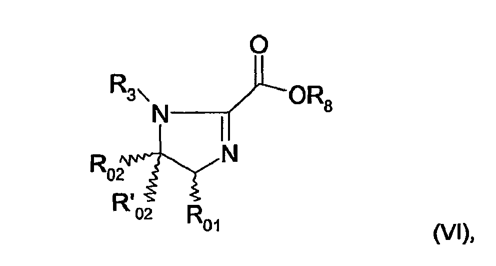
d) the compound of the formula VI is reacted with at least two equivalents of an organometallic compound of the formula VII or VIIa
R₄-X₂ (VII), R₄-(X₂)₂ (VIIa),
where R₄ is as defined in Claim 1, X₂ is an alkali metal or -Me₁X₃, Me₁ is Mg or Zn, and X₃ is Cl, Br or I, to form a compound of the formula VIII 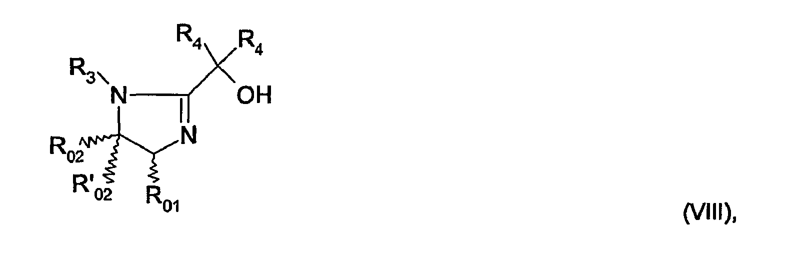 and
e) the hydroxyl group of the compound of the formula VIII is metallated and subsequently reacted with a halophosphine of the formula IX,
X₁-Y₁ (IX),
where X₁ is as defined in Claim 1 and Y₁ is Cl, Br or I, to give a compound of the formula la or Ib.

13. Complexes of metals selected from the group of TM8 metals with compounds of the formulae I and Ia according to claim 1 as ligands.

14. Metal complexes according to Claim 13, **characterized in that** the TM metals are Cu, Ag, Au, Ni, Co, Rh, Ru, Pd, lr and Pt.

15. Metal complexes according to Claim 14, **characterized in that** the TM metals are rhodium, iridium, ruthenium, platinum and palladium.

16. Metal complexes according to Claim 13, **characterized in that** the metal complexes have the formulae XI and XII,
A₁MeLₙ (XI),
(A₁MeLₙ)^{(z+)}(E⁻)_{z} (XII),
where A₁ is a compound of the formula I or la,
L represents identical or different monodentate, anionic or nonionic ligands, or two L together represent identical or different bidentate, anionic or nonionic ligands;
n is 2, 3 or 4 when L is a monodentate ligand, or n is 1 or 2 when L is a bidentate ligand;
z is 1, 2 or 3;
Me is a metal selected from the group consisting of Rh, lr and Ru, with the metal having the oxidation state 0, 1, 2, 3 or 4;
E⁻ is the anion of an oxo acid or complex acid; and
the anionic ligands balance the charge of the oxidation state 1, 2, 3 or 4 of the metal.

17. Metal complexes according to Claim 16, **characterized in that** E is -Cl⁻, -Br, -l⁻, ClO₄⁻, CF₃SO₃, CH₃SO₃ , HSO₄⁻, (CF₃SO₂)₂N⁻, (CF₃SO₂)₃C⁻, B(phenyl)₄⁻, B[bis(3,5-trifluoromethyl)phenyl]₄⁻ , B[bis(3,5-dimethyl)phenyl]₄⁻, B(C₆F₅)₄⁻, B(4-methylphenyl)₄⁻, tetra-(C₁-C₅-perfluoroalkyl)aluminate, BF₄⁻ , PF₆⁻, SbCL₆⁻, AsF₆⁻ or SbF₆⁻.

18. Metal complexes according to Claim 13**, characterized in that** they have the formulae XIII and XIV,
[A₁Me₂YZ] (XIII),
[A₁Me₂Y]⁺E₁⁻ (XIV),
where
A₁ is a compound of the formula I or la;
Me₁ is rhodium or iridium;
Y represents two olefins or a diene;
Z is Cl, Br or I; and
E₁⁻ is the anion of an oxo acid or complex acid.

19. Metal complexes according to Claim 18, **characterized in that** Y is a C₂-C₁₂-olefin, the diene contains from 5 to 12 C atoms, Z is Cl or Brand E₁ is BF₄⁻, ClO₄⁻, CF₃SO₃⁻, CH₃SO₃⁻, HSO₄⁻, B(phenyl)₄⁻, B[bis(3,5-trifluoromethyl)phenyl]₄⁻, PF₆⁻, SbCl₆⁻, AsF₆⁻ or SbF₆⁻.

20. Process for preparing chiral organic compounds by asymmetric addition of hydrogen, boron hydrides or silanes onto a carbon-carbon or carbon-heteroatom multiple bond in prochiral organic compounds, or the asymmetric addition of C-nucleophiles onto allyl compounds in the presence of a catalyst, **characterized in that** the addition reaction is carried out in the presence of catalytic amounts of at least one metal complex according to Claim 14.

21. Use of the metal complexes according to Claim 13 as homogeneous catalysts for preparing chiral organic compounds by asymmetric addition of hydrogen, boron hydrides or silanes onto a carbon-carbon or carbon-heteroatom multiple bond in prochiral organic compounds, or the asymmetric addition of C-nucleophiles or amines onto allyl compounds..

## Patentansprüche

1. Verbindungen der Formeln I und la, 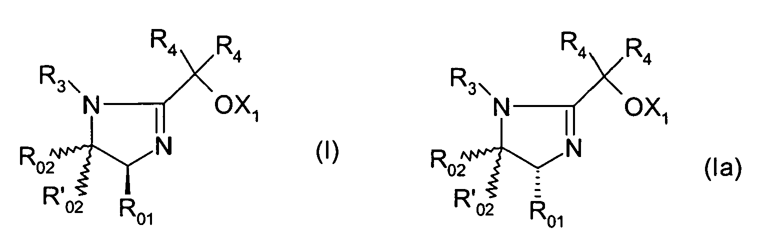 worin
X₁ Sekundärphosphino bedeutet;
R₃ einen Kohlenwasserstoffrest mit 1 bis 20 C-Atomen, einen über ein C-Atom gebundenen Heterokohlenwasserstoffrest mit 2 bis 20 Atomen und wenigstens einem Heteroatom ausgewählt aus der Gruppe O, S, NH und NR, oder einen -SO₂-R Rest darstellt;
R C₁-C₁₈-Alkyl, Phenyl oder Benzyl bedeutet;
die R₄ unabhängig voneinander für Wasserstoff oder einen Kohlenwasserstoffrest mit 1 bis 20 C-Atomen stehen, oder beide R₄ zusammen mit dem C-Atom, an das sie gebunden sind, einen drei- bis achtgliedrigen Kohlenwasserstoffring bilden;
R₀₁ einen Kohlenwasserstoffrest mit 1 bis 20 C-Atomen darstellt; und
R₀₂ und R'₀₂ für ein Wasserstoffatom stehen oder unabhängig die Bedeutung von R₀₁ haben, oder
R₀₁ und R₀₂ zusammen mit den C-Atomen, an das sie gebunden sind, einen drei- bis achtgliedrigen Kohlenwasserstoff- oder Heterokohlenwasserstoffring bilden.

2. Verbindungen gemäss Anspruch 1, **dadurch gekennzeichnet, dass** X₁ als Phosphingruppe zwei gleiche oder zwei verschiedene Kohlenwasserstoffreste mit 1 bis 22 C-Atomen enthalten, oder die beiden Kohlenwasserstoffreste mit dem P-Atom einen 3- bis 8-gliedrigen Ring bilden.

3. Verbindungen gemäss Anspruch 2, **dadurch gekennzeichnet, dass** X₁ die Gruppe -PR₁R₂ darstellt, worin
R₁ und R₂ unabhängig voneinander einen Kohlenwasserstoffrest mit 1 bis 20 C-Atomen darstellen, der unsubstituiert oder substituiert ist mit Halogen, C₁-C₆-Alkyl, C₁-C₆-Halogenalkyl, C₁-C₆-Alkoxy, C₁-C₆-Halogenalkoxy, (C₆H₅)₃Si, (C₁-C₁₂-Alkyl)₃Si, oder -CO₂-C₁-C₆-Alkyl; oder worin R₁ und R₂ je zusammen unsubstiuiertes oder mit C₁-C₄-Alkyl oder C₁-C₄-Alkoxy substituiertes Dimethylen, Trimethylen, Tetramethylen, oder Pentamethylen bedeuten.

4. Verbindungen gemäss Anspruch 3, **dadurch gekennzeichnet, dass** R₁ und R₂ gleiche oder verschiedene Reste bedeuten, ausgewählt aus der Gruppe verzweigtes C₃-C₆-Alkyl, unsubstituiertes oder mit ein bis drei C₁-C₄-Alkyl oder C₁-C₄-Alkoxy substituiertes Cyclopentyl oder Cyclohexyl, unsubstituiertes oder mit ein bis drei C₁-C₄-Alkyl oder C₁-C₄-Alkoxy substituiertes Benzyl, und unsubstituiertes oder mit ein bis drei C₁-C₄-Alkyl, C₁-C₄-Alkoxy, - NH₂, OH, F, Cl, C₁-C₄-Fluoralkyl oder C₁-C₄-Fluoralkoxy substituiertes Phenyl.

5. Verbindungen gemäss Anspruch 3, **dadurch gekennzeichnet, dass** R₁ und R₂ gleiche oder verschiedene Reste bedeuten, ausgewählt aus der Gruppe unsubstituiertes oder mit ein bis drei C₁-C₄-Alkyl, C₁-C₄-Alkoxy oder C₁-C₄-Fluoralkyl substituiertes Phenyl.

6. Verbindungen gemäss Anspruch 1, **dadurch gekennzeichnet, dass** es sich bei dem Kohlenwasserstoffrest R₃ um C₁-C₁₈-Alkyl; C₃-C₁₂-Cycloalkyl; oder C₆-C₁₆-Aryl; und bei dem Heterokohlenwasserstoffrest R₃ um C₂-C₁₈-Heteroalkyl; C₃-C₁₂-Heterocyclolkyl; oder C₃-C₁₆-Heteroaryl mit 1 bis 3 Heteroatomen, ausgewählt aus der Gruppe O, S und NR handelt, und R C₁-C₄-Alkyl ist.

7. Verbindungen gemäss Anspruch 6, **dadurch gekennzeichnet, dass** es sich bei um einen aromatischen Kohlenwasserstoffrest R₃ handelt, der C₆-C₁₄-Aryl ist.

8. Verbindungen gemäss Anspruch 7, **dadurch gekennzeichnet, dass** R₃ C₆-C₁₀-Aryl darstellt, das unsubstituiert oder mit Halogen, CF₃, OCF₃, C₁-C₄-Alkyl oder C₁-C₄-Alkoxy substituiert ist.

9. Verbindungen gemäss Anspruch 1, **dadurch gekennzeichnet, dass** R₄ einen Kohlenwasserstoffrest bedeutet, ausgewählt aus der Gruppe C₁-C₁₈-Alkyl, C₃-C₁₂-Cycloalkyl, C₆-C₁₆-Aryl oder C₇-C₁₆-Aralkyl.

10. Verbindungen gemäss Anspruch 1, **dadurch gekennzeichnet, dass** R₀₁ α-verzeigtes Alkyl mit wenigstens 3 C-Atomen, und R₀₂ und R'₀₂ für Wasserstoff steht.

11. Verbindungen gemäss Anspruch 1, **dadurch gekennzeichnet, dass** sie den Formeln Ib und Ic entsprechen, 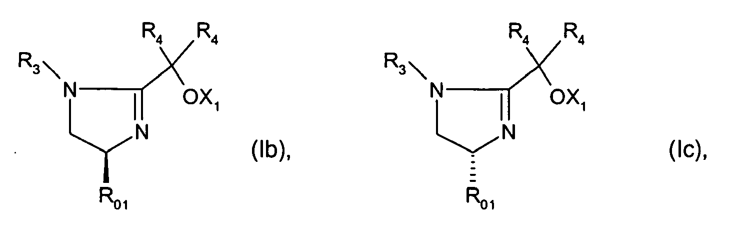 worin
X₁ für -PR₁R₂ steht,
R₁ und R₂ gleiche oder verschiedene und insbesondere gleiche Reste darstellen, ausgewählt aus der Gruppe α-verzweigtes C₃-C₆-Alkyl, unsubstituiertes oder mit ein bis drei C₁-C₄-Alkyl oder C₁-C₄-Alkoxy substituiertes C₅-C₇-Cycloalkyl, oder unsubstituiertes, mit ein bis drei C₁-C₄-Alkyl, C₁-C₄-Alkoxy oder C₁-C₄-Fluoralkyl substituiertes Phenyl, oder unsubstituiertes oder mit C₁-C₄-Alkyl oder C₁-C₄-Alkoxy substituiertes Dimethylen, Trimethylen, Tetramethylen oder Hexamethylen;
R₃ Benzyl oder C₆-C₁₂-Aryl ist, und Aryl und Benzyl unsubstituiert oder mit Halogen, C₁-C₄-Alkyl, C₁-C₄-Halogenalkyl oder C₁-C₄-Alkoxy substituiert sind;
R₄ C₁-C₆-Alkyl oder Benzyl darstellt, und
R₀₁ α-verzweigtes C₃-C₈-Alkyl bedeutet.

12. Verfahren zur Herstellung von Verbindungen der Formeln I und la, 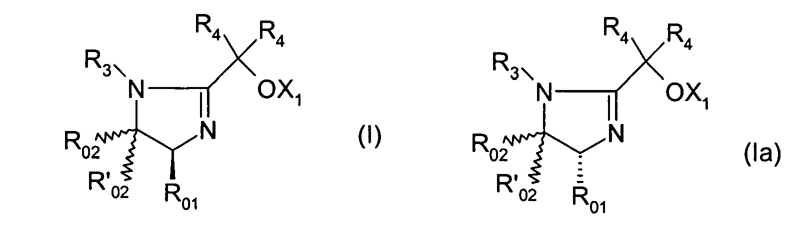 worin R₀₁, R₀₂, R'₀₂, R₃, R₄ und X₁ die in Anspruch 1 angegebenen Bedeutungen haben, und - für die R- oder S-Form steht, **dadurch gekennzeichnet, dass** man
a) eine Verbindung der Formel II 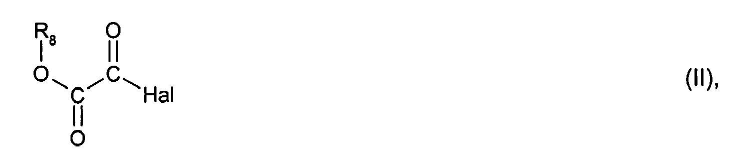 worin R₈ für C₁-C₈-Alkyl steht, und Hal für Cl, Br oder I steht, in Gegenwart eines tertiären Amins mit wenigstens einer äquivalenten Menge einer Verbindung der Formel III, 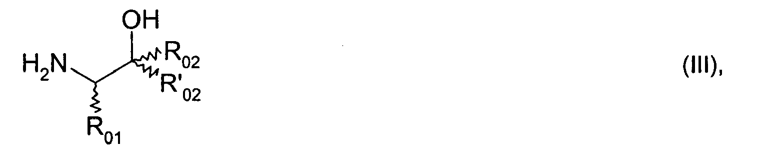 worin R₀₁ und R₀₂ die in Anspruch 1 angegeben Bedeutungen haben, zu einer Verbindung der Formel IV umsetzt, 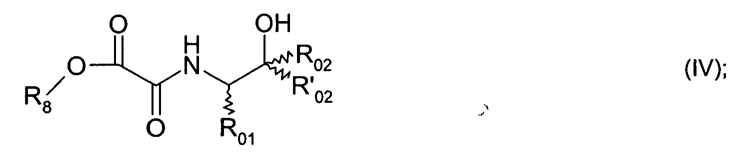
b) die Verbindung der Formel IV mit wenigstens äquivalent Mengen eines Halogenierungsmittels zu einer Verbindug der Formel V umsetzt, 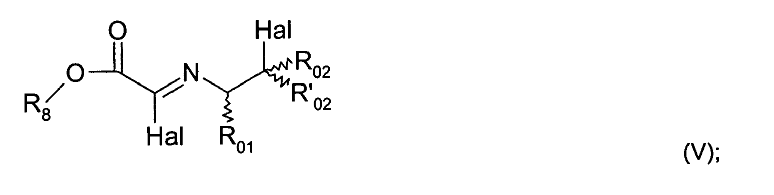
c) die Verbindung der Formel V mit einem primären Amin der Formel R₃-NH₂ (X) in Gegenwart eines tertiären Amins zu einer Verbindung der Formel VI cyclisiert, 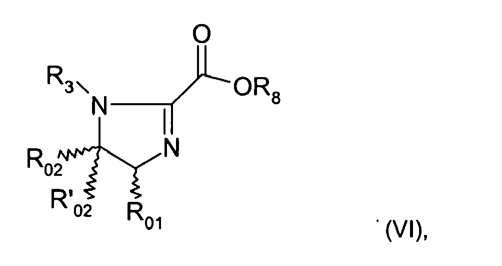
d) die Verbindung der Formel VI mit wenigstens 2 Äquivalenten einer metallorganischen Verbindung der Formel VII oder Vlla
R₄-X₂ (VII),
R₄-(X₂)₂ (VIIa),
worin R₄ die die in Anspruch 1 angegebene Bedeutung hat, X₂ ein Alkalimetall oder -Me₁X₃ darstellt, Me₁ für Mg oder Zn steht, und X₃ für Cl, Br oder I steht, zu einer Verbindung der Formel VIII 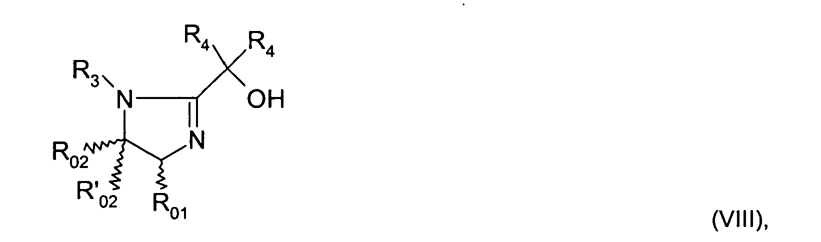 umsetzt; und
e) die Hydroxylgruppe in der Verbindung der Formel VIII metallisiert und anschliessend mit einem Halogenphosphin der Formel IX,
X₁-Y₁ (IX),
worin X₁ die die in Anspruch 1 angebene Bedeutung hat und Y₁ für Cl, Br oder I steht, zu einer Verbindung der Formel Ia oder Ib umsetzt.

13. Metallkomplexe von Metallen, ausgewählt aus der Gruppe der TM8-Metalle mit Verbindungen der Formeln I und Ia gemäss Anspruch 1 als Liganden.

14. Metallkomplexe gemäss Anspruch 13, **dadurch gekennzeichnet, dass** es sich bei den TM-Metallen um Cu, Ag, Au, Ni, Co, Rh, Ru, Pd, Ir und Pt handelt.

15. Metallkomplexe gemäss Anspruch 14, **dadurch gekennzeichnet, dass** es sich bei den TM-Metallen um Rhodium, Iridium, Ruthenium, Platin und Palladium handelt.

16. Metallkomplexe gemäss Anspruch 13, **dadurch gekennzeichnet, dass** die Metallkomplexe Formeln XI und XII entsprechen,
A₁MeLₙ (XI),
(A₁MeLₙ)^{(z+)}(E⁻)_{z} (XII),
worin A₁ für eine Verbindung der Formel 1 oder Ia steht,
L für gleiche oder verschiedene monodentate, anionische oder nicht-ionische Liganden steht, oder zwei L für gleiche oder verschiedene bidentate, anionische oder nicht-ionische Liganden steht;
n für 2, 3 oder 4 steht, wenn L einen monodentaten Liganden bedeutet, oder n für 1 oder 2 steht, wenn L einen bidentaten Liganden bedeutet;
z für 1, 2 oder 3 steht;
Me ein Metall ausgewählt aus der Gruppe Rh, Ir und Ru bedeutet; wobei das Metall die Oxidationsstufen 0, 1, 2, 3 oder 4 aufweist;
E⁻ das Anion einer Sauerstoffsäure oder Komplexsäure ist; und
die anionischen Liganden die Ladung der Oxidationsstufen 1, 2, 3 oder 4 des Metalls ausgleichen.

17. Metallkomplexe gemäss Anspruch 16, **dadurch gekennzeichnet, dass** E für -Cl⁻, -Br⁻, -I⁻, ClO₄⁻, CF₃SO₃⁻, CH₃SO₃⁻, HSO₄⁻, (CF₃SO₂)₂N⁻, (CF₃SO₂)₃C⁻, B(Phenyl)₄⁻, B[Bis(3,5-trifluormethyl)phenyl]₄⁻, B[Bis(3,5-dimethyl)phenyl]₄⁻, B(C₆F₅)₄⁻, B(4-Methylphenyl)₄⁻, Tetra-(C₁-C₅-perfluoralkyl)aluminat, BF₄⁻, PF₆⁻, SbCl₆-, AsF₆⁻ oder SbF₆⁻ steht.

18. Metallkomplexe gemäss Anspruch 13, **dadurch gekennzeichnet, dass** sie den Formeln XIII und XIV entsprechen,
[A₁Me₂YZ] (XIII),
[A₁Me₂Y]⁺E₁⁻ (XIV),
worin
A₁ für eine Verbindung der Formel I oder Ia steht;
Me₂ Rhodium oder Iridium bedeutet;
Y für zwei Olefine oder ein Dien steht;
Z Cl, Br oder I bedeutet; und
E₁⁻ das Anion einer Sauerstoffsäure oder Komplexsäure darstellt.

19. Metallkomplexe gemäss Anspruch 18, **dadurch gekennzeichnet, dass** Y ein C₂-C₁₂-Olefin ist, das Dien 5 bis 12 C-Atome enthält, und Z für Cl oder Br steht, und E₁ BF₄⁻, ClO₄⁻, CF₃SO₃⁻, CH₃SO₃⁻, HSO₄⁻, B(Phenyl)₄⁻, B [Bis(3,5-trifluormethyl)phenyl]₄⁻, PF₆⁻, SbCl₆⁻, AsF₆⁻ oder SbF₆⁻ bedeutet.

20. Verfahren zur Herstellung chiraler organischer Verbindungen durch asymmetrische Anlagerung von Wasserstoff, Borhydriden oder Silanen an eine Kohlenstoff- oder Kohlenstoff-Heteroatommehrfachbindung in prochiralen organischen Verbindungen, oder die asymmetrische Addition von C-Nukleophilen oder Aminen an Allylverbindungen in Gegenwart eines Katalysators, **dadurch gekennzeichnet, dass** man die Anlagerung in Gegenwart katalytischer Mengen wenigstens eines Metallkomplexes gemäss Anspruch 14 durchführt.

21. Verwendung der Metallkomplexe gemäss Anspruch 13 als homogene Katalysatoren zur Herstellung chiraler organischer Verbindungen durch asymmetrische Anlagerung von Wasserstoff, Borhydriden oder Silanen an eine Kohlenstoff- oder Kohlenstoff-Heteroatommehrfachbindung in prochiralen organischen Verbindungen, oder die asymmetrische Addition von C-Nukleophilen oder Aminen an Allylverbindungen.

## Revendications

1. Composés de formules I et Ia, 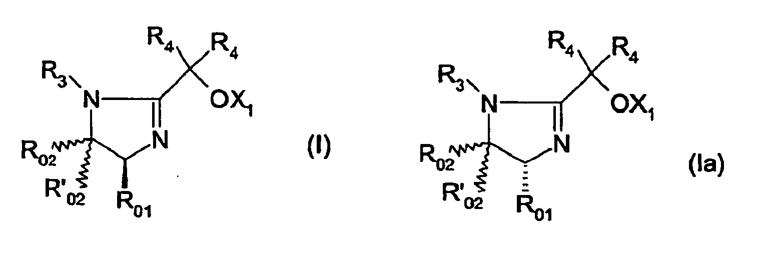 dans lesquelles
X₁ est un groupe phosphino secondaire ;
R₃ est un radical hydrocarboné ayant de 1 à 20 atomes de carbone, un radical hétérohydrocarboné qui est lié par l'intermédiaire d'un atome de carbone et possède de 2 à 20 atomes et au moins un hétéroatome choisi dans le groupe constitué par O, S, NH et NR, ou un radical -SO₂-R ;
R représente un radical alkyle en C₁ à C₁₈, phényle ou benzyle ;
les radicaux R₄ représentent chacun, indépendamment l'un de l'autre, un atome d'hydrogène ou un radical hydrocarboné ayant de 1 à 20 atomes de carbone, ou bien les deux radicaux R₄ conjointement avec l'atome de carbone auquel ils sont liés forment un cycle hydrocarboné de trois à huit éléments ;
R₀₁ représente un radical hydrocarboné ayant de 1 à 20 atomes de carbone ; et
R₀₂ et R'₀₂ représentent chacun un atome d'hydrogène ou, indépendamment l'un de l'autre, ont la signification de R₀₁, ou
R₀₁ et R₀₂ conjointement avec l'atome de carbone auquel ils sont liés forment un cycle hydrocarboné ou hétérohydrocarboné de trois à huit éléments.

2. Composés selon la revendication 1, **caractérisés en ce que** X₁ en tant que groupe phosphine contient deux radicaux hydrocarbonés identiques ou deux radicaux hydrocarbonés différents ayant de 1 à 22 atomes de carbone, ou bien les deux atomes hydrocarbonés conjointement avec l'atome P forment un cycle de 3 à 8 éléments.

3. Composés selon la revendication 2, **caractérisés en ce que** X₁ représente le groupe -PR₁R₂, où R₁ et R₂ représentent chacun, indépendamment l'un de l'autre, un radical hydrocarboné qui possède de 1 à 20 atomes de carbone et est non substitué ou substitué par un atome d'halogène, un radical alkyle en C₁ à C₆, halogénoalkyle en C₁ à C₆, alcoxy en C₁ à C₆, halogénoalcoxy en C₁ à C₆, (C₆H₅)₃Si, (alkyle en C₁ à C₁₂)₃Si ou -CO₂-(alkyle en C₁ à C₆) ; ou R₁ et R₂ forment ensemble un radical diméthylène, triméthylène, tétraméthylène ou pentaméthylène non substitué ou substitué par un groupe alkyle en C₁ à C₄ ou alcoxy en C₁ à C₄.

4. Composés selon la revendication 3, **caractérisés en ce que** R₁ et R₂ sont des radicaux identiques ou différents choisis dans le groupe constitué par un groupe alkyle en C₃ à C₆ ramifié, cyclopentyle ou cyclohexyle non substitué et cyclopentyle ou cyclohexyle portant de un à trois groupes alkyle en C₁ à C₄ ou alcoxy en C₁ à C₄ en tant que substituants, un groupe benzyle non substitué et benzyle portant de un à trois groupes alkyle en C₁ à C₄ ou alcoxy en C₁ à C₄ en tant que substituants et un groupe phényle non substitué et phényle portant de un à trois groupes alkyle en C₁ à C₄, alcoxy en C₁ à C₄, -NH₂, OH, F, Cl, un groupe fluoroalkyle en C₁ à C₄ ou fluoroalcoxy en C₁ à C₄ en tant que substituants.

5. Composés selon la revendication 3, **caractérisés en ce que** R₁ et R₂ représentent des radicaux identiques ou différents choisis dans le groupe constitué par un groupe phényle non substitué et phényle substitué par un à trois groupes alkyle en C₁ à C₄, alcoxy en C₁ à C₄ ou fluoroalkyle en C₁ à C₄.

6. Composés selon la revendication 1, **caractérisés en ce que** le radical hydrocarboné R₃ est un groupe alkyle en C₁ à C₁₈ ; cycloalkyle en C₃ à C₁₂ ou aryle en C₆ à C₁₆ ; et le radical hétérohydrocarboné R₃ est un groupe hétéroalkyle en C₂ à C₁₈ ; hétérocycloalkyle en C₃ à C₁₂ ou hétéroaryle en C₃ à C₁₆ contenant de 1 à 3 hétéroatomes choisis dans le groupe constitué par O, S et NR, et R représente un groupe alkyle en C₁ à C₄.

7. Composés selon la revendication 6, **caractérisés en ce que** le radical hydrocarboné aromatique R₃ représente un groupe aryle en C₆ à C₁₄.

8. Composés selon la revendication 7, **caractérisés en ce que** R₃ représente un groupe aryle en C₆ à C₁₀ qui est non substitué ou substitué par un atome d'halogène, CF₃, OCF₃, alkyle en C₁ à C₄ ou alcoxy en C₁ à C₄.

9. Composés selon la revendication 1, **caractérisés en ce que** R₄ représente un radical hydrocarboné choisi dans le groupe constitué par un groupe alkyle en C₁ à C₁₈, cycloalkyle en C₃ à C₁₂, aryle en C₆ à C₁₆ ou aralkyle en C₇ à C₁₆.

10. Composés selon la revendication 1, **caractérisés en ce que** R₀₁ représente un groupe alkyle α-ramifié ayant au moins 3 atomes de carbone, et R₀₂ et R'₀₂ représentent chacun un atome d'hydrogène.

11. Composés selon la revendication 1, **caractérisés en ce qu'**ils possèdent les formules Ib et Ic, 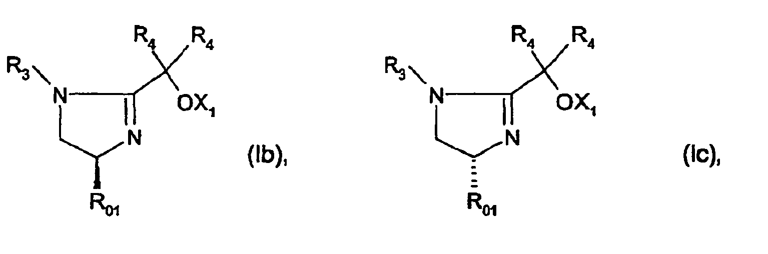 dans lesquelles
X₁ représente -PR₁R₂,
R₁ et R₂ sont des radicaux identiques ou différents et en particulier des radicaux identiques choisis dans le groupe constitué par un groupe alkyle en C₃ à C₆ α-ramifié, cycloalkyle en C₅ à C₇ non substitué et cycloalkyle en C₅ à C₇ portant de un à trois groupes alkyle en C₁ à C₄ ou alcoxy en C₁ à C₄ en tant que substituants et un groupe phényle non substitué et phényle portant de un à trois groupes alkyle en C₁ à C₄, alcoxy en C₁ à C₄ ou fluoroalkyle en C₁ à C₄ en tant que substituants et un radical diméthylène, triméthylène, tétraméthylène et hexaméthylène non substitué ou substitué par un groupe alkyle en C₁ à C₄ ou alcoxy en C₁ à C₄ ;
R₃ représente un groupe benzyle ou aryle en C₆ à C₁₂, et les groupes aryle et benzyle sont non substitués ou substitués par un atome d'halogène, un groupe alkyle en C₁ à C₄, halogénoalkyle en C₁ à C₄ ou alcoxy en C₁ à C₄ ;
R₄ représente un groupe alkyle en C₁ à C₆ ou benzyle, et
R₀₁ est un groupe alkyle en C₃ à C₈ α-ramifié.

12. Procédé de préparation de composés de formules I et Ia, 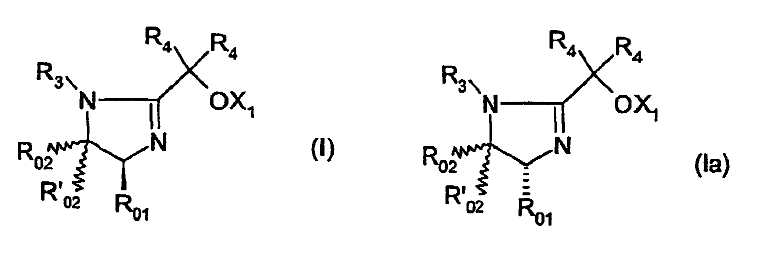 dans lesquelles R₀₁, R₀₂, R'₀₂, R₃, R₄ et X₁ sont tels que définis dans la revendication 1, et 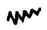 représente la forme R ou S, **caractérisée en ce que**
a) un composé de formule II 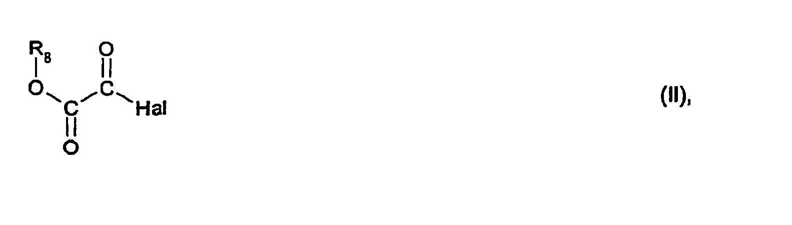 dans laquelle R₈ représente un groupe alkyle en C₁ à C₈ et Hal représente -Cl, -Br ou -I, est mis à réagir en présence d'une amine tertiaire avec au moins une quantité équivalente d'un composé de formule III, 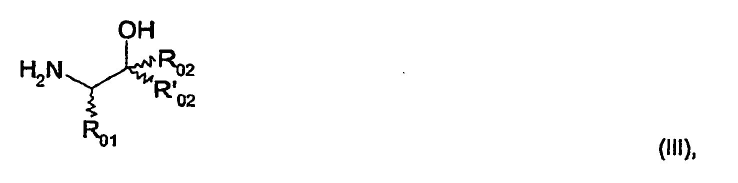 dans laquelle R₀₁ et R₀₂ sont tels que définis dans la revendication 1 pour former un composé de formule IV, 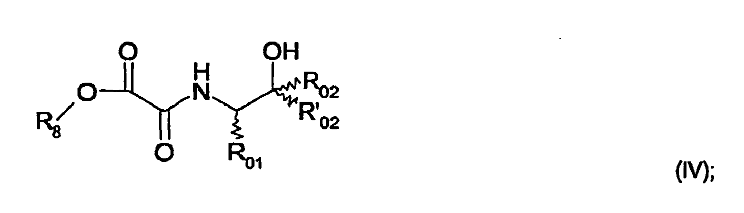
b) le composé de formule IV est mis à réagir avec au moins des quantités équivalentes d'un agent d'halogénation pour former un composé de formule V, 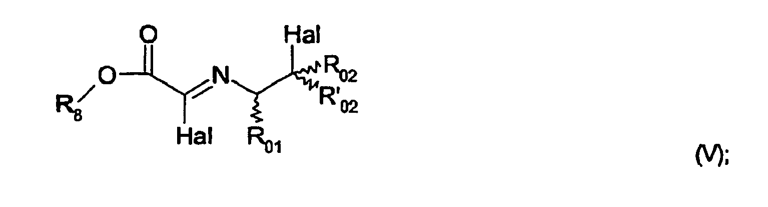
c) le composé de formule V est cyclisé avec une amine primaire de formule R₃-NH₂ (X) en présence d'une amine tertiaire pour former un composé de formule VI, 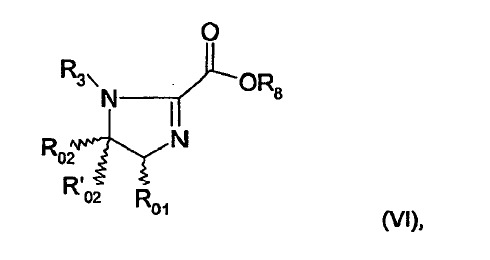
d) le composé de formule VI est mis à réagir avec au moins deux équivalents d'un composé organométallique de formule VII ou VIIa
R₄-X₂ (VII),
R₄-(X₂)₂ (VIIa),
dans laquelle R₄ est tel que défini dans la revendication 1, X₂ représente un métal alcalin ou -Me₁X₃, Me₁ représente Mg ou Zn, et X₃ représente Cl, Br ou I, pour former un composé de formule VIII : 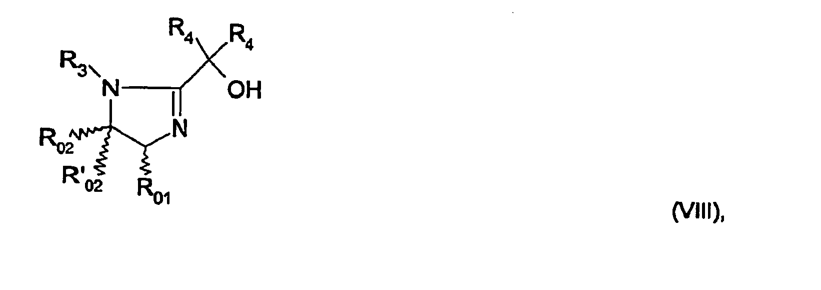 et
e) le groupe hydroxyle du composé de formule VIII est métallaté et est mis ultérieurement à réagir avec une halogénophosphine de formule IX,
X₁-Y₁ (IX),
dans laquelle X₁ est tel que défini dans la revendication 1 et Y₁ représente -Cl, -Br ou -I, pour obtenir un composé de formule Ia ou Ib.

13. Complexes de métaux choisis dans le groupe de métaux TM8 avec des composés de formules I et Ia selon la revendication 1 en tant que ligands.

14. Complexes métalliques selon la revendication 13, **caractérisés en ce que** les métaux TM sont Cu, Ag, Au, Ni, Co, Rh, Ru, Pd, Ir et Pt.

15. Complexes métalliques selon la revendication 14, **caractérisés en ce que** les métaux TM sont le rhodium, l'iridium, le ruthénium, le platine et le palladium.

16. Complexes métalliques selon la revendication 13, **caractérisés en ce que** les complexes métalliques possèdent les formules XI et XII :
A₁MeLₙ (XI),
(A₁MeLₙ)^{(z+)}(E⁻)_{z} (XII),
dans lesquelles A₁ représente un composé de formule I ou Ia,
L représente des ligands monodentés anioniques ou non ioniques, identiques ou différents, ou deux L représentent ensemble des ligands bidentés anioniques ou non ioniques identiques ou différents ;
n vaut 2, 3 ou 4 lorsque L est un ligand monodenté, ou n vaut 1 ou 2 lorsque L est un ligand bidenté ;
z vaut 1, 2 ou 3 ;
Me représente un métal choisi dans le groupe constitué par Rh, Ir et Ru, le métal ayant l'état d'oxydation 0, 1, 2, 3 ou 4 ;
E⁻, représente l'anion d'un acide oxo ou d'un acide complexe ; et
les ligands anioniques équilibrent la charge de l'état d'oxydation 1, 2, 3 ou 4 du métal.

17. Complexes métalliques selon la revendication 16, **caractérisés en ce que** E représente -Cl⁻, -Br⁻, -I⁻, ClO₄⁻, CF₃SO₃⁻, CH₃SO₃⁻, HSO₄⁻, (CF₃SO₂)₂N⁻, (CF₃SO₂)₃C⁻, B (phényl)₄⁻, B[bis(3,5-trifluorométhyl)phényl]₄⁻, B[bis(3,5-diméthyl)phényl]₄⁻, B(C₆F₅)₄⁻, B(4-méthylphényl)₄⁻, tétra-(perfluoroalkyle en C₁ à C₅)aluminate, BF₄⁻, PF₆⁻, SbCl₆⁻, AsF₆⁻ ou SbF₆⁻.

18. Complexes métalliques selon la revendication 13, **caractérisés en ce qu'**ils possèdent les formules XIII et XIV,
[A₁Me₂YZ] (XIII),
[A₁Me₂Y]⁺E₁⁻ (XIV),
dans lesquelles
A₁ représente un composé de formule I ou Ia ;
Me₁ représente le rhodium ou l'iridium ;
Y représente deux oléfines ou un diène ;
Z représente Cl, Br ou I ; et
E₁- représente l'anion d'un acide oxo ou d'un acide complexe.

19. Complexes métalliques selon la revendication 18, **caractérisés en ce que** Y représente une oléfine en C₂ à C₁₂, le diène contient de 5 à 12 atomes de carbone, Z représente Cl ou Br et E₁ représente BF₄⁻, ClO₄⁻, CF₃SO₃⁻, CH₃SO₃⁻, HSO₄⁻, B(phényl)₄⁻, B[bis(3,5-trifluorométhyl)phényl]₄⁻, PF₆⁻, SbCl₆⁻, AsF₆⁻ ou SbF₆⁻.

20. Procédé de préparation de composés organiques chiraux par l'addition asymétrique d'hydrogène, d'hydrures de bore ou de silanes sur une liaison multiple carbone-carbone ou carbone-hétéroatome dans des composés organiques prochiraux, ou l'addition asymétrique de nucléophiles C sur des composés allyle en présence d'un catalyseur, **caractérisé en ce que** la réaction d'addition est effectuée en présence de quantités catalytiques d'au moins un complexe métallique selon la revendication 14.

21. Utilisation des complexes métalliques selon la revendication 13, sous forme de catalyseurs homogènes pour préparer des composés organiques chiraux par l'addition asymétrique d'hydrogène, d'hydrures de bore ou des silanes sur une liaison multiple carbone-carbone ou carbone-hétéroatome en composés organiques prochiraux, ou l'addition asymétrique de nucléophiles C ou d'amines sur des composés allyle.
